(19) **Europäisches Patentamt / European Patent Office / Office européen des brevets**

(11) **EP 1 782 778 A1**

(12) # EUROPEAN PATENT APPLICATION
published in accordance with Art. 158(3) EPC

(43) Date of publication:
09.05.2007 Bulletin 2007/19

(51) Int Cl.:
*A61F 7/08* (2006.01)    *C09K 5/16* (2006.01)

(21) Application number: 05765772.8

(86) International application number:
PCT/JP2005/013008

(22) Date of filing: 14.07.2005

(87) International publication number:
WO 2006/006655 (19.01.2006 Gazette 2006/03)

(84) Designated Contracting States:
DE FR GB

(30) Priority: 14.07.2004 JP 2004207836

(71) Applicant: Mycoal Products Corporation
Tochigi-chi, Tochigi 328-0067 (JP)

(72) Inventor: DODO, Toshihiro,
MYCOAL PRODUCTS CORPORATION
Tochigi-shi,
Tochigi 3280067 (JP)

(74) Representative: Thun, Clemens et al
Mitscherlich & Partner
Sonnenstrasse 33
80331 München (DE)

(54) **HEATING PAD AND METHOD OF USING THE SAME**

(57) To provide a heat generating pad which is able to relax symptoms such as stiff shoulder, low-back pain, muscular fatigue, menstrual pain, a symptom of poor circulation and in particular, can be suitably used for relaxation of a symptom of menstrual pain and a method of use of the same.

A heat generating pad in which a heat generating composition molded body resulting from molding a moldable heat generating composition containing surplus water as a connecting substance is interposed between packaging materials and the periphery of the heat generating composition molded body is heat sealed, which is **characterized in that** the packaging materials are constituted of a substrate and a covering material; that the substrate is substantially planar and does not contain an accommodating pocket; that the heat sealing forms a sectioned part as formed in a striped form, and plural sectional exothermic parts as sectioned by the heat sealing are provided via the sectioned part; that the moldable heat generating composition has a water content of from 1 to 60 % by weight, contains, as essential components, a flocculant aid, a drybinding agent, a flocculant, adhesive binder, an iron powder, a carbon component, a reaction accelerator and water, does not contain, a thickener and an excipient, contains the surplus water so as to have a water mobility value of from 0.01 to 20, with the water in the moldable heat generating composition not functioning as a barrier layer, and is capable of causing an exothermic reaction upon contact with air; that the heat generating pad contains a region having a ratio of bending resistance in the orthogonal directions of 2 or more on the surface thereof orthogonal to the thickness direction of the heat generating pad; that at least a part of the heat generating pad has air permeability; and that the heat generating pad has a fixing measure on at least a part of the exposed surface thereof.

FIG.2(a)

EP 1 782 778 A1

# FIG.2(b)

**Description**

[Technical Field]

The present invention relates to a heat generating pad which is adhered to clothes or the body of a user or which is intended to be adhered directly to the skin of a user and held.
In addition, the invention relates to a heat generating pad which holds a heat generating composition capable of causing heat generation upon permeation of oxygen within a pad. In addition, more specifically, the invention relates to a heat generating pad which is able to relax symptoms such as stiff shoulder, low-back pain, abdominal pain, joint pain, and muscular fatigue and in particular, can be suitably used for relaxation of a symptom of menstrual pain, and method of use of the same.

[Background Art]

For the purpose of relaxing various symptoms such as menstrual pain, there have hitherto been used warm patch materials which upon sticking onto an affected part, expand a blood vessel to increase a blood flow, raise the temperature of an affected part and promote metabolism. For example, Patent Document 1 and Patent Document 2 disclose patch materials blended with a drug such as vitamins and an essence of capsicum. Such a patch material is composed of a support and a sticky ointment layer containing a drug. With respect to an ejector of a drug, only a function for penetrating the drug into an affected part is emphasized. Accordingly, for the purposes of making the presence thereof thin during sticking to the skin, improving a sticking feeling to the skin and making it hard to be peeled away from the skin, a material which is flexible and stretchable is selected as the support.

Furthermore, examples of a heating pad include electrically heating pads and heat generating pads utilizing an oxidation reaction of iron.

Heat generating compositions utilizing an oxidation reaction of a metal such as iron have been provided as a powder or granule, or a viscous material or creamy material. Heat generating pads utilizing such a heat generating composition are very excellent in view of costs, safety, exothermic temperature, and so on and are already put into practical use as, for example, a so-called chemical body warmer as filled in an air-permeable bag.

In order to obtain a more comfortable feeling for use, there have been proposed various heat generating compositions which design to have shape holding properties and to hold exothermic characteristics while using a thickener, a binding agent, etc. in quest of prevention of deviation of a heat generating composition and fitness to various kinds of shapes.
For example, Patent Document 3 proposes a process for producing a heat generating composition as granulated so as to have an average particle size of 0.5 mm or more and a process for producing a heat generating composition having an improved granular strength by blending from 10 to 20 parts by weight of an adhesive binder component in addition water and granulating.
Also, Patent Document 4 proposes a throwaway body warmer composed of a heat generating composition having shape holding characteristics by adding a powdered thickener such as corn starch and potato starch.
Also, Patent Document 5 proposes a solid heat generating composition as prepared by mixing a binding agent such as CMC in a powdered or granular heat generating composition and compression molding the mixture.
Also, Patent Document 6 proposes a heat generating pad as prepared by using a crosslinking agent, etc. and a water absorptive polymer and integrating them under pressure.
Also, Patent Document 7 proposes a heat generating composition in an ink form and/or a creamy form using a thickener so as to have viscosity, a heat generating pad and a process for producing the same.

Also, Patent Document 8 proposes a heat generating composition molded body using a binding agent, the surface of which is covered by an air-permeable film material such as CMC, thereby designing to hold the shape.

Also, Patent Document 9 and Patent Document 10 propose a heat generating composition as processed into a viscous material or a creamy material, in which the shape is changed from a conventional rectangle to a foot shape or an elliptical shape so as to adapt to the outline of a body to be warmed.

Also, there is disclosed a heat generating pad having a soft structure in which an exothermic part having a heat generating composition sealed between packaging materials at least one surface of which is permeable to air is constituted of plural small exothermic parts as divided by a seal part. Patent Document 11 and Patent Document 12 each discloses a heat generating pad in which a powdered heat generating composition is filled in sectioned divisions and which is made of plural exothermic parts as divided by a seal part. Also, Patent Document 13, Patent Document 14, Patent Document 15, Patent Document 16, Patent Document 17 and Patent Document 18 each proposes a heat generating composition using a flocculant and a dry binding agent and a heat generating pad in which a heat generating composition exothermic part is sectioned into plural divisions by using a substrate having an accommodating pocket.

Also, it is said that a symptom of menstrual pain is caused when a direct stimulus in the affected part or an indirect stimulus which has passed from the central nerve through the peripheral nerve due to a mental stimulus causes tension of a muscle or tendon, whereby vital energy and blood stay in the affected part. Accordingly, in order to relax a symptom of menstrual pain, it is said that it is effective to relieve the tension of a muscle or tendon which hinders the flow of vital energy and blood.

Also, in order to relax a symptom of poor circulation, it is said that it is effective to warm an area beneath the navel.

Though an electrically heating pad is excellent in adjusting capability of the temperature for compensating clothes of a user, the device becomes large in size and complicated so that it is problematic in portable use.

In conventional heat generating pads utilizing an oxidation reaction of iron, an exothermic part is large so that warming along the muscular or tendon directions was not obtainable.

Also, in a heat generating pad utilizing an oxidation reaction of iron and using plural cells for heat generation, the plural cells for heat generation contain a chemical substance for heat generation such as an iron powder between a cell forming layer and a cell covering layer, and the covering layer has an oxygen-permeable measure as a cell cover layer. As the oxygen-permeable measure, there is enumerated a method for providing an opening by so-called perforation by boring such as inserting a heated needle or a cold needle into the cell cover layer. Accordingly, the chemical substance for heat generation which is a heat generating composition uses a flocculant aid, a flocculant, a dry binding agent, an agglomeration aid, and the like and is compressed and shaped, whereby the chemical substance for heat generation is prevented from leakage from the opening by perforation. The exothermic performance of the chemical substance for heat generation becomes worse thereby, and therefore, cells for heat generation which withstand for practical use become inevitably large in size. By keeping apart the cells for heat generation, an effect for adapting to the outline of a human body reduces by half. Furthermore, in the case of using a chemical substance for heat generation which does not contain a flocculant aid, a flocculant, a dry binding agent, an agglomeration aid, and the like, it is necessary to previously prepare an accommodating pocket in an accommodating packaging material. In cells for heat generation and heat generating pads having cells for heat generation embedded therein, there was involved a problem that their production becomes complicated.

[Patent Document 1] JP-A-53-91114
[Patent Document 2] JP-A-3-184915
[Patent Document 3] JP-A-4-293989
[Patent Document 4] JP-A-6-343658
[Patent Document 5] JP-A-59-189183
[Patent Document 6] WO 00/13626
[Patent Document 7] JP-A-9-75388
[Patent Document 8] JP-A-60-101448
[Patent Document 9] JP-A-9-276317
[Patent Document 10] JP-A-11-299817
[Patent Document 11] JP-UM-A-1-110718
[Patent Document 12] JP-UM-A-6-26829
[Patent Document 13] JP-A-2000-288008
[Patent Document 14] JP-T-11-507593
[Patent Document 15] JP-T-11-508314
[Patent Document 16] JP-T-11-508786
[Patent Document 17] JP-T-11-512954
[Patent Document 18] JP-T-2002-514104

[Disclosure of the Invention]

[Problems that the Invention is to Solve]

In view of the foregoing, an object of the invention is to provide a heat generating pad which is able to relax symptoms such as stiff shoulder, low-back pain, muscular fatigue, menstrual pain, a symptom of poor circulation and in particular, can be suitably used for relaxation of a symptom of menstrual pain and a method of use of the same.

[Means for Solving the Problems]

Then, in order to solve the foregoing problems of the related art, the present inventor made extensive and intensive investigations and carried out various systematic experiments. As a result, he has reached the invention.
As set forth in claim 1, a heat generating pad of the invention is a heat generating pad in which a heat generating composition molded body resulting from molding a moldable heat generating composition containing surplus water as a connecting substance is interposed between packaging materials and the periphery of the heat generating composition molded body is heat sealed, which is characterized in that:

1) the packaging materials are constituted of a substrate and a covering material,
2) the substrate is substantially planar and does not contain an accommodating pocket,
3) the heat sealing forms a sectioned part as formed in a striped form, and plural sectional exothermic parts as sectioned by the heat sealing are provided via the sectioned part,
4) the moldable heat generating composition has a water content of from 1 to 60 % by weight, contains, as essential components, an iron powder, a carbon component, a reaction accelerator and water, does not contain a flocculant aid, a flocculant, an agglomeration aid, a dry binder, a dry binding agent, a dry binding material, a sticky raw material, a thickener and an excipient, contains the surplus water so as to have a water mobility value of from 0.01 to 20, with the water in the moldable heat generating composition not functioning as a barrier layer, and is capable of causing an exothermic reaction upon contact with air,
5) the heat generating pad contains a region having a ratio of bending resistance in the orthogonal directions of 2 or more on the surface thereof orthogonal to the thickness direction of the heat generating pad,
6) at least a part of the heat generating pad has air permeability, and
7) the heat generating pad has a fixing measure on at least a part of the exposed surface thereof.

Also, a heat generating pad as set forth in claim 2 is characterized in that in the heat generating pad as set forth in claim 1, a minimum bending resistance in one direction on the surface orthogonal to the thickness direction of the heat generating pad is not more than 100 mm.

Also, a heat generating pad as set forth in claim 3 is characterized in that in the heat generating pad as set forth in claim 1, a ratio of the capacity of the sectional exothermic parts to a ratio of the heat generating composition molded body is from 0.6 to 1.0.

Also, a heat generating pad as set forth in claim 4 is characterized in that in the heat generating pad as set forth in any one of claims 1 to 3, the heat generating composition contains a component resulting from a contact treatment of a mixture containing at least an iron powder, a carbon component, a reaction accelerator and water as essential components with an oxidizing gas.

Also, a heat generating pad as set forth in claim 5 is characterized in that in the heat generating pad as set forth in claim 1, the iron powder comprising particles, a surface of each of which is at least partially coverd with an iron oxide film, the iron oxide film has a thickness of 3 nm or more, and the iron powder at least contains from 20 to 100 % by weight of an active iron powder, which comprises particles having a region of an oxygen-free iron component in at least one region selected from a central part region of the iron powder particle and a region beneath the iron oxide film.

Also, a heat generating pad as set forth in claim 6 is characterized in that in the heat generating pad as set forth in claim 1, the iron powder particle is covered on at least a part of the surface thereof by a wustite film and contains from 20 to 100 % by weight of an active iron powder having an amount of wustite of from 2 to 50 % by weight in terms of an X-ray peak intensity ratio to iron.

Also, a heat generating pad as set forth in claim 7 is characterized in that in the heat generating pad as set forth in claim 1, the heat generating composition molded body is compressed.

Also, a heat generating pad as set forth in claim 8 is characterized in that in the heat generating pad as set forth in claim 1, the heat seal part is heat sealed after temporary adhesion by an adhesive layer, and an adhesive component which constitutes the adhesive layer and a component of a heat seal material which constitutes the heat seal layer are copresent in the heat seal part.

Also, a heat generating pad as set forth in claim 9 is characterized in that in the heat generating pad as set forth in claim 8, after heat sealing, a part of the heat generating composition molded body is moved to a temporary adhering part which is not heat sealed, thereby deadhering the temporary adhering part.

Also, a heat generating pad as set forth in claim 10 is characterized in that in the heat generating pad as set forth in claim 1, the substrate and the covering material have stretchability.

Also, a heat generating pad as set forth in claim 11 is characterized in that in the heat generating pad as set forth in claim 1, at least a part of the sectioned part has an irregular pattern.

Also, a heat generating pad as set forth in claim 12 is characterized in that in the heat generating pad as set forth in claim 1, the sectional exothermic parts and the sectioned part have a difference of altitude, the sectional exothermic parts and the sectioned part are covered with an air permeability adjusting material, an airway is formed under the air permeability adjusting material, and air is taken in from an end part of the heat generating pad.

Also, a heat generating pad as set forth in claim 13 is characterized in that in the heat generating pad as set forth in claim 12, the air permeability adjusting material is constituted of an air-impermeable raw material.

Also, a heat generating pad as set forth in claim 14 is characterized in that in the heat generating pad as set forth in claim 12, the fixing measure is provided outside the air permeability adjusting material.

Also, a heat generating pad as set forth in claim 15 is characterized in that in the heat generating pad as set forth in claim 1, the fixing measure is an adhesive layer.

Also, a heat generating pad as set forth in claim 16 is characterized in that in the heat generating pad as set forth in claim 15, the adhesive layer is provided on at least a part of the air-permeable surface.

Also, a heat generating pad as set forth in claim 17 is characterized in that in the heat generating pad as set forth in claim 15, a thermal buffer sheet is provided in the central part of the adhesive layer.

Also, a heat generating pad as set forth in claim 18 is characterized in that in the heat generating pad as set forth in claim 15, an adhesive which constitutes the adhesive layer is a non-aromatic hot melt based adhesive.

Also, a heat generating pad as set forth in claim 19 is characterized in that in the heat generating pad as set forth in claim 15, the adhesive layer is a hydrophilic adhesive layer, and the packaging material between the hydrophilic adhesive layer and the heat generating composition molded body has a moisture permeability of not more than 2 $g/m^2/24$ hr.

Also, a heat generating pad as set forth in claim 20 is characterized in that in the heat generating pad as set forth in claim 15, the adhesive layer contains a drug.

Also, a heat generating pad as set forth in claim 21 is characterized in that in the heat generating pad as set forth in claim 1, the moldable heat generating composition contains at least one member selected from additional components consisting of a water retaining agent, a water absorptive polymer, a pH adjusting agent, a hydrogen formation inhibitor, an aggregate, a fibrous material, a functional substance, a surfactant, an organosilicon compound, a pyroelectric substance, a moisturizer, a fertilizer component, a hydrophobic polymer compound, a heat generating aid, a metal other than iron, a metal oxide other than iron oxide, an acidic substance, and a mixture thereof.

Also, a heat generating pad as set forth in claim 22 is characterized in that in the heat generating pad as set forth in claim 1, the heat generating pad is accommodated in an air-impermeable accommodating bag.

As set forth in claim 23, a method of use of a heat generating pad of the invention is characterized by bringing the heat generating pad as set forth in claim 1 into contact with a necessary part of the body.

[Advantages of the Invention]

In the light of the above, the heat generating pad of the invention is not one as prepared by previously preparing an exothermic part and embedding it in a packaging material but an integral heat generating pad as produced directly from a moldable heat generating composition, a substrate and a covering material. By constituting an exothermic part of sectional exothermic parts in a striped form, namely in a form of stripe and a sectioned part along them, on the surface of the heat generating pad orthogonal to the thickness direction thereof, a ratio of a bending resistance in one direction to a small bending resistance in the orthogonal direction thereto is 2 or more. In addition, by regulating a minimum bending resistance in one direction on the surface orthogonal to the thickness direction of the heat generating pad at not more than 100 mm, fitness to an adherend remarkably increased.

Furthermore, by employing a constitution having sectional exothermic parts in a striped form (in a form of stripe), the following advantages are brought.

1) Since the exothermic part is composed of an exothermic part having sectional exothermic parts in a striped form (in a form of stripe), a minimum bending resistance in one direction on the surface orthogonal to the thickness direction of the heat generating pad is not more than 60 mm. Thus, the heat generating pad of the invention is excellent in adhesion to curved surfaces such as the body.

2) Since the heat generating pad of the invention has a region having a ratio of bending resistance of 2 or more in at least a part thereof on the surface orthogonal to the thickness direction thereof, it can be easily fixed along curved surfaces such as the body.

3) Since the bending resistance of the heat generating pad of the invention in one direction is low, it is possible to easily wind the heat generating pad in that direction and fold it. Also, it is possible to compactly accommodate and store the heat generating pad in an outer bag which is an air-impermeable accommodating bag for storage and lighten deterioration of the heat generating composition.

4) The heat generating pad of the invention can relax symptoms such as stiff shoulder, low-back pain, joint pain, muscular fatigue, and menstrual pain and in particular, can be effectively used for relaxation of a symptom of menstrual pain.

[Best Modes for Carrying Out the Invention]

The invention is concerned with a heat generating pad in which a heat generating composition molded body resulting from molding a moldable heat generating composition containing surplus water as a connecting substance is interposed between packaging materials and the periphery of the heat generating composition molded body is heat sealed, which is characterized in that:

1) the packaging materials are constituted of a substrate and a covering material,

2) the substrate is substantially planar and does not contain an accommodating pocket,

3) the heat sealing forms a sectioned part as formed in a striped form, and plural sectional exothermic parts as sectioned by the heat sealing are provided via the sectioned part,

4) the moldable heat generating composition has a water content of from 1 to 60 % by weight, contains, as essential components, an iron powder, a carbon component, a reaction accelerator and water, does not contain a flocculant aid, a flocculant, an agglomeration aid, a dry binder, a dry binding agent, a dry binding material, a sticky raw material, a thickener and an excipient, contains the surplus water so as to have a water mobility value of from 0.01 to 20, with the water in the moldable heat generating composition not functioning as a barrier layer, and is capable of causing an exothermic reaction upon contact with air,

5) the heat generating pad contains a region having a ratio of bending resistance in the orthogonal directions of 2 or more on the surface thereof orthogonal to the thickness direction of the heat generating pad,

6) at least a part of the heat generating pad has air permeability, and

7) the heat generating pad has a fixing measure on at least a part of the exposed surface thereof.

Here, examples of the shape of the heat generating composition molded body include a parallelepiped shape, a spindle shape, a truncated cone shape, a pillar shape, an elliptic cylindrical shape, and a semi-pillar shape. Furthermore, a concave may be present in a central part, etc. of the heat generating composition molded body. In the invention, a heat generating composition compressed body which is a compressed body of a heat generating composition molded body is also included in the heat generating composition molded body.

With respect to the heat generating pad of the invention, a heat generating pad in which sectional exothermic parts having a parallelepiped shape, in which an absolute value of a difference between bending resistances in the two directions orthogonal to each other becomes maximal, are provided in a striped form at intervals; a heat generating pad further provided an adhesive layer; or a heat generating pad in which adhesive layers are provided in a striped form at intervals is very flexible in one direction and rigid in the other direction. Thus, it exhibits an effect for relaxing symptoms such as stiff shoulder, low-back pain, and muscular fatigue and in particular, relaxing a symptom of menstrual pain. In addition, the heat generating pad of the invention can be wound in a size substantially equal to the width in a width direction thereof, becomes compact and is convenient for accommodation. Furthermore, in the case of a separator-provided heat generating pad, by using a separator having a low bending resistance, the heat generating pad can be wound.

Furthermore, in the case of providing a heat generating pad along the body, the body includes many two-dimensional curves, and in shoulders, legs, abdomen, waist, arms, and the like, one direction is substantially linear, and the other two directions are formed of a substantially curved surface. Accordingly, since the heat generating pad of the invention which is able to form a substantially linear surface in one direction and a curved surface in the other two directions is able to form a two-dimensional curved surface, it is able to well follow the body and is optimum for warming of the body and relaxation or treatment of various symptoms.

By regulating a bending resistance of the heat generating pad in the lengthwise direction high and a bending resistance thereof in the short direction substantially orthogonal thereto extremely low, at the time of installation, the lengthwise direction is stiff so that the heat generating pad can be installed in reliance thereupon, whereas the short direction is soft so that the heat generating pad can be easily fitted to a warmth-taking part. Also, an accommodating bag made of a non-stretchable packaging material has both flexibility and stiffness so that handling properties and fitness are very harmonious with each other.

As a method for relieving the tension of a muscle or tendon which hinders the flow of vital energy and blood, which is a method for relaxing symptoms such as stiff shoulder, low-back pain, and muscular fatigue and in particular, relaxing a symptom of menstrual pain, a method for continuously giving a physical tension in a striped form in parallel to a muscle or tendon which hinders the flow of vital energy and blood and in the opposite direction to the tension is extremely effective. However, in conventional warm patch materials, upon sticking onto an affected part, they relax various symptoms such as menstrual pain by expanding a blood vessel to increase a blood flow, raising the temperature of an affected part and promoting metabolism. Furthermore, with respect to an ejector of a drug, only a function for penetrating the drug into an affected part is emphasized. Accordingly, for the purposes of making the presence thereof thin during sticking to the skin, improving a sticking feeling to the skin and making it hard to be peeled away from the skin, a material which is flexible and stretchable is selected as the support. However, such conventional patch materials act merely as an ejector of the drug, but they were not ones which positively give such a physical tension to the affected part.

In the adhesive layer-provided heat generating pad of the invention, the adhesive layers and the sectional exothermic parts are provided in a striped form at intervals on a non-stretchable and rigid substrate.

The terms "provided in a striped form at intervals" as referred to in the invention mean that elongated and continuous sectional exothermic parts and/or adhesive layers are disposed at intervals and that stripes are disposed in, for example, a parallel stripe form, a radial form, or a folding fan form. In providing the stripes at intervals, it is preferable that the stripes have a width and a gap of from approximately 3 to 10 mm, respectively. Furthermore, the orientation of the stripes can also be adjusted corresponding to the distribution state of a muscle or tendon of the affected part onto which the heat generating pad is stuck.

In this way, in sticking the adhesive layer-provided heat generating pad to an affected part such that a muscle or tendon of the affected part is in parallel to the sectional exothermic parts and the adhesive layers, a reverse physical tension is continuously given to the tension of the muscle or tendon, whereby the tension of the muscle or tendon is relieved. Furthermore, since a fault strain is brought between the adjacent muscles or tendon, the physical tension for relieving the tension of the muscles or tendons is reinforced. As a result, residence of vital energy and blood is dissolved, and a symptom of menstrual pain is lightened. In addition, a stimulus of a so-called "acupuncture point" by a pressing feeling to the affected part by the stiff substrate or the thickness of the heat generating pad is also effective for relaxing the symptom of menstrual pain.

Furthermore, it is said that a symptom of menstrual pain is caused when a direct stimulus in the affected part or an indirect stimulus which has passed from the central nerve through the peripheral nerve due to a mental stimulus causes tension of a muscle or tendon, whereby vital energy and blood stay in the affected part.

Furthermore, in order to relax a symptom of poor circulation, it is said that it is effective to warm an area beneath the navel. Thus, warming along the abdominal region is effective.

With respect to the air permeability of the heat generating pad of the invention, it is only required that at least a part of one surface has air permeability. In the case where the both surfaces are permeable to air, the air permeability of the both surfaces may be different from each other.

Furthermore, a heat generating pad of one embodiment of the invention has a structure in which one surface (air-permeable sticky surface) of the heat generating pad has air permeability and has an adhesive layer, whereas the other surface (non-sticky surface) does not have an adhesive layer and warms the body in such a manner that the air-permeable sticky surface of the heat generating pad is stuck to the inside of an underwear, whereas the non-sticky surface is brought into contact with the skin.

The heat generating pad has a structure in which one surface (air-permeable sticky surface) of the heat generating pad has air permeability and has an adhesive layer, whereas the other surface (non-sticky surface) does not have an adhesive layer and is constituted such that the heat generating pad is worn between an underwear and the skin while sticking the air-permeable sticky surface to the inside of the underwear. In this way, the adhesive layer of the heat generating pad does not come into contact with the skin, neither coldness at the time of wearing nor a pain during peeling away is brought, neither itchiness nor an eruption on the skin surface is caused during wearing, and an uncomfortable feeling as caused due to sticking can be dissolved.

In addition, the air-permeable surface of the flexible heat generating pad is stuck to the inside of an underwear, namely the air-permeable surface is faced outward a human body and does not come into contact with the surface which hinders the air permeability in bringing it into intimate contact with the skin surface, etc., and uneven distribution of the heat generating composition does not occur during the use. Thus, it has become possible to keep desired exothermic characteristics without being affected by posture, movement, etc. during wearing. Besides, since heat of the heat generating body can be efficiently transferred to the body without sticking it directly to the skin, the amount of the heat generating composition in the heat generating pad can be reduced, and a heat generating pad which is light in weight and thin and gives a desired duration can be obtained.

Furthermore, according to the heat generating pad of the invention, by adjusting the size or gap of the convex sectional exothermic parts, an exothermic part which is flexible and exhibits a uniform temperature distribution and an exothermic part which exhibits a pattern-like temperature distribution are obtainable. By the pattern-like temperature distribution, it is possible to improve an acupuncture point effect in the warming part.

By using a raw material having a bending resistance of not more than 100 mm, preferably not more than 60 mm, more preferably from 10 to 60 mm, and further preferably from 10 to 50 mm for the substrate or covering material, a good touch at the time of contacting with the body or at the time of use is obtainable. Furthermore, since a good touch is obtained, the surface which comes into contact with the body is preferably made of a non-woven fabric, a textile or a knitted material, and especially preferably a non-woven fabric, a textile or a knitted material each having a napping structure.

Accordingly, examples of the raw material on the surface which comes into contact with the body include non-woven fabric/film, textile/film, and knitted material/film.

The surface having an adhesive layer as the fixing measure is preferably made of a film, a sheet, or a non-woven fabric, a textile or a knitted material each having low napping properties.

Furthermore, since the packaging material on the surface of the heat generating pad which comes into contact with the skin does not have an adhesive layer, it can be relatively arbitrarily set up. For example, by taking into consideration a tough such as a texture, heat transfer properties, water absorption, and the like, the packaging material can be made of an arbitrary raw material in an arbitrary shape, whereby an uncomfortable feeling during the use can be dissolved.

In addition, in the heat generating pad of the invention, a first outer packaging material or a second outer packaging material, each of which is an air-permeable packaging material, may be provided on the first surface or second surface of the heat generating pad. The first outer packaging material or second outer packaging material may be provided with the foregoing fixing measure. As the fixing measure, the foregoing adhesive layer is preferable. It is preferable that the adhesive layer is protected by a separator, etc.

Next, the invention will be described below with reference to Figs. 1 to 16.

Fig. 1 is a plan view of a heat generating pad 1 in which plural sectional exothermic parts 3 are provided in a striped form, and the sectional exothermic parts 3 are provided at intervals by a sectioned part 4 made of a heat seal part. Fig. 2 is a cross-sectional view along the line Z-Z of Fig. 1.

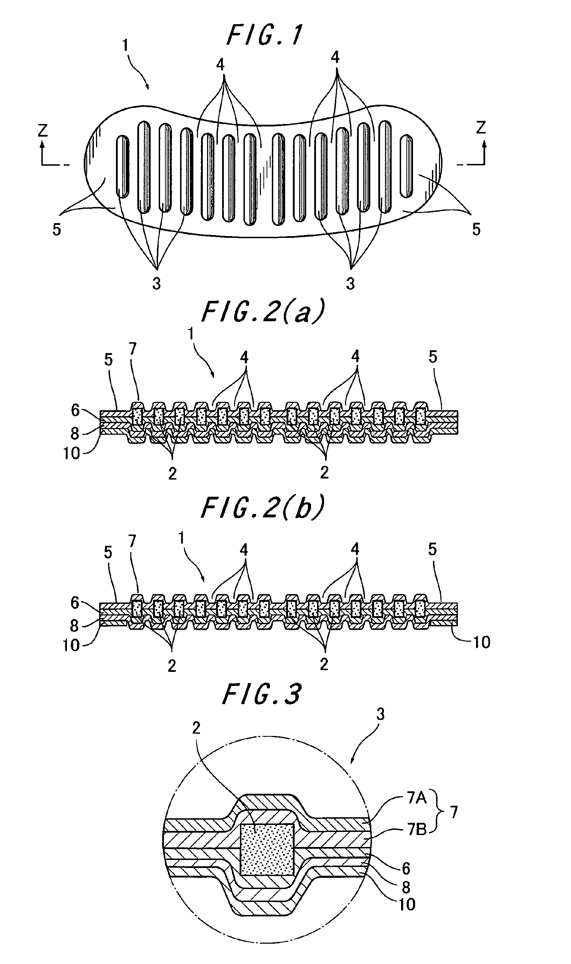

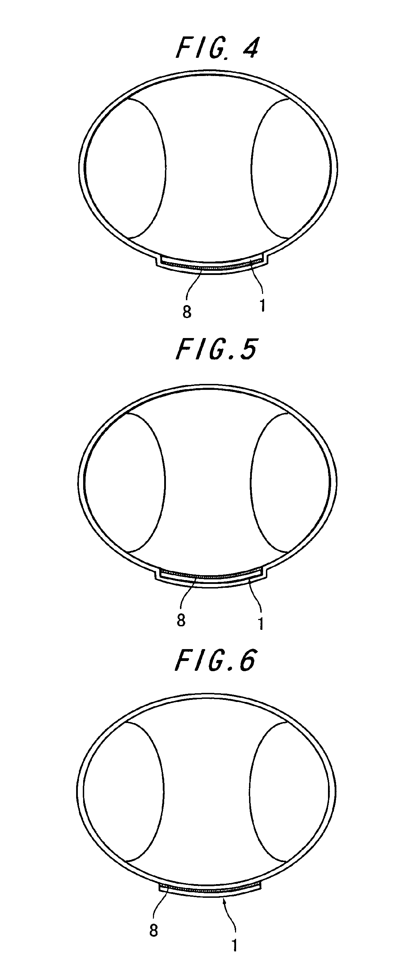

Fig. 3 is an enlarged view to show the sectional exothermic part 3 of a part of Fig. 2 and the surroundings thereof. Fig. 6 shows the state of use in which a heat generating pad is stuck to the outside of panties and the heat generating pad transfers heat through the panties. A heat generating pad of a type for sticking to clothes, in which an acrylic solid type adhesive layer is provided on a substrate which is an air-impermeable surface, is used. The heat generating pad is preferably placed in the direction of an upper end of a waist band of the panties. In this case, the heat generating pad works as a heat generating pad for relaxation of menstrual pain. Furthermore, it can be used for relaxation of low-back pain. In this case, the heat generating pad may be stuck such that it comes into contact with the vicinity of the waist.

Fig. 4 is a plan view of women's panties as seen from the waist part and shows a heat generating pad as stuck to the inside of the panties such that when the panties are worn, the heat generating pad transfers heat to the abdominal of a user. The illustrated material is a heat generating pad of a type for sticking to the inside of clothes, in which an SIS based adhesive is used in the adhesive layer and the air-permeable surface of the heat generating pad has air permeability. In this case, the heat generating pad works as a heat generating pad for relaxation of menstrual pain. Furthermore, it can be used for relaxation of low-back pain. In this case, the heat generating pad may be stuck such that it comes into contact with the vicinity of the waist.

Fig. 5 is a plan view of the abdominal of a woman as seen from the waist part and shows a heat generating pad as stuck to the abdominal of a user when panties are worn. In the illustrated material, the adhesive layer uses an SIS based adhesive and is provided on a substrate which is an air-impermeable surface of the heat generating pad. In this case, the heat generating pad works as a heat generating pad for relaxation of menstrual pain. Furthermore, it can be used for relaxation of low-back pain. In this case, the heat generating pad may be stuck such that it comes into contact with the vicinity of the waist.

Fig. 7 shows an example in which the adhesive layer of Fig. 1 is changed from the solid form to an air-permeable netlike form (honeycomb form) and is provided in the side of the air-permeable surface. That is, a hot melt based adhesive 8A which is a pressure sensitive heat fusible adhesive is provided in a netlike form (honeycomb form) by a melt blow method. Even by providing a solid adhesive layer in the sectioned part in the side of the air-permeable surface, the same function is obtainable. Its use method is, for example, shown in Fig. 5.

Fig. 8 is a cross-sectional view along the line Y-Y of Fig. 7.

Fig. 9 shows the state that the heat generating pad 1 of Fig. 1 is folded by half in such a manner that the separator is positioned outward and accommodated in an outer bag 11 which is an air-impermeable accommodating bag in the folded state.

Fig. 10 shows the state that sectional exothermic parts 3 are provided in a striped form in a two-stage way and one sectional exothermic part for interrupting a row of the two-stage exothermic parts is provided in each end part.

Fig. 11 (a) is a heat generating pad 1 in which sectional exothermic parts as provided in a striped form are provided along the outer circumference. The material as shown in Fig. 9 is an air permeability adjusting material.

Fig. 11 (b) is a cross-sectional view along the line X-X of Fig. 11 (a) and is a heat generating pad 1 such that an air permeability adjusting material 9 covers the tops of sectional exothermic parts 3.

Fig. 12 is a view to show the state that an air permeability adjusting material 9 is fixed to the tops of exothermic parts 3 and substantially the central part of a sectioned part 4 and that spacial parts 9A are formed.

Fig. 13 shows a paper lantern-like heat generating pad 1 in which a separator-provided adhesive layer 8B which is a fixing measure is provided in each end part of the heat generating pad 1.

Fig. 14 (a) is a cross-sectional view along the line W-W of Fig. 13. Fig. 14 (b) shows a heat generating pad of the same type as in Fig. 13 in which, however, an adhesive layer is provided on one surface thereof and a thermal buffer sheet 9 is provided in the central part of the adhesive layer.

Fig. 15 shows a modified example of a paper lantern-like heat generating pad 1, in which the vicinity of the central part of the heat generating pad 1 is constricted. Such a paper lantern-like heat generating pad is used for a shoulder in such a manner that it is hung on a shoulder and fixed to the skin of the shoulder via adhesive layers in both end parts thereof. On this occasion, the heat generating pad 1 is fitted along the shoulder, does not cause deviation or falling off and is excellent in feeling for use.

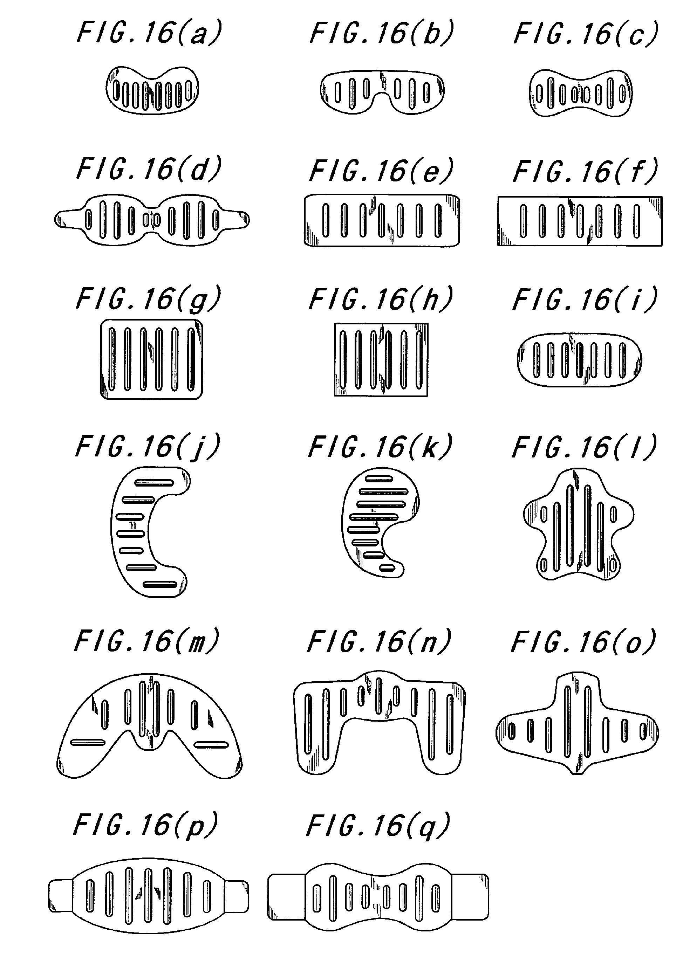

Figs. 16(a) to 16(q) show examples of the shape of the heat generating pad of the invention. (a) shows a broad bean-like shape; (b) shows an eye mask-like shape; (c) shows a cocoon-like shape; (d) shows a gourd-like shape; (e) shows a rectangular shape with rounded corners; (f) shows a rectangular shape; (g) shows a square shape with rounded corners; (h) shows a square shape; (i) shows an egg-like shape; (j) shows a boomerang-like shape; (k) shows a comma-shaped bead-like shape; (1) shows a star-like shape; (m) shows a wing-like shape; (n) shows a wing-like shape; (o) shows a nose-like shape; (p) shows a paper lantern-like shape; and (q) shows a paper lantern-like shape, respectively. (m) and (n) are suited for a neck or the surroundings of a shoulder. Furthermore, while the directions of the long axes along the long sides of the rectangles of the sectional exothermic parts are parallel to each other, they may be arbitrarily set up. Also, a gathering of sectional exothermic parts in different directions may be employed. Modified shapes as modified on the basis of these basic skeletons can also be used. In the case of fixing the heat generating pad along the curved surfaces of a human body, the majority of the curved surfaces of a human body is curved in one direction, and the exothermic part constituted of sectional exothermic parts in a striped form has both easiness of handling and fitness to the curved surfaces of a human body with a good balance because the bending direction is extremely bent in one direction and the orthogonal direction thereto is extremely hardly bent. It is preferable that a portion which comes into contact with the skin is constituted of a soft packaging material such as napped (fluffy) non-woven fabrics. The shape of the heat generating pad as described in the present description also includes modifications which are made on the basis of the shapes described therein.

In the heat generating pad of the invention, the moldable heat generating composition is activated by oxygen as obtained from circumferential oxygen, thereby causing heat generation. In order to feed oxygen into the sectional exothermic parts, the covering material has air permeability.

The plural sectional exothermic parts are separated from each other, and each of the sectional exothermic parts functions independently upon the remainder. Since the heat generating composition molded body is tightly packed in each sectional exothermic part, by separating the sectional exothermic parts from each other, it is possible to easily adapt the heat generating pad to the outline of the body as compared with a single exothermic part. The heat generating composition molded body may be compressed as a compressed body in each of the sectional exothermic parts. Therefore, the sectional exothermic parts are not easily bent, whereas the sectioned part present between the sectional exothermic parts can be bent because the heat generating composition does not exist. Thus, adaptability to the body can be revealed.

Desirably, the respective sectional exothermic parts have a similar amount of the heat generating composition molded body and similar permeability to oxygen. Separately, the amount, shape and permeability to oxygen of the heat generating composition may be different between the respective sectional exothermic parts so far as the temperature of the sectional exothermic parts as generated consequently is similar.

In the light of the above, the sectional exothermic part of the invention is not of a structure in which an exothermic part as prepared from a certain packaging material and a heat generating composition is wrapped by a substrate and a covering material as other packaging materials but has an integral structure in which a heat generating composition molded body resulting from molding a moldable heat generating composition is wrapped by a substrate and a covering material and sealed.

The cross-sectional shape of the sectioned part which is the seal surface may be formed irregularly, thereby providing a pattern.

The cross-sectional shape of the seal surface may be a planar and plain surface. However, for the purposes of not only making fashionableness rich and imparting visible pleasant but also making slipperiness small in forwarding the heat generating pad, it is preferred to form irregularities to provide a pattern.

It is not always required that this pattern is provided over the whole of the seal surface. A pattern may be provided only in the side of the sectional exothermic part, with the remainder being not provided with a pattern. Inversely, no pattern may be provided in the side of the sectional exothermic part, with the remainder being provided with a pattern.

The pattern on the seal surface is not particularly limited so far as the cross-sectional shape is irregular. Examples thereof include an orthogonal lattice shape, a parallel vertical linear shape, a parallel horizontal linear shape, a zigzag shape, an oblique lattice shape, a broken oblique linear shape, an oblique checkerwork shape, and a scattered point shape. For example, no pattern may be provided in the side of the sectional exothermic part, with the central part being patterned. Inversely, the side of the sectional exothermic part is patterned, with the remainder as a central part being not provided with a pattern. The pattern can be arbitrarily chosen. Furthermore, a pattern in the surroundings as the circumferential part of the heat generating pad is the same.

The heat generating pad of the invention can be used outside the inner layer of clothes or inside the inner layer of clothes, or can be used by sticking to the body. The relation between the heat generating pad and the body of a user can be used for different purposes depending upon the desire. Furthermore, the heat generating pad is designed such that the fixing measure produces a fixed temperature even on any surface of the heat generating pad by providing the exothermic part with irregularities by the sectional exothermic parts and the sectioned part. The heat generating pad may be brought into direct contact with the body of a user upon wearing in clothes of the user. The heat generating composition is designed so as to achieve oxidation at a specific rate for the purpose of producing a specific temperature.

By using the heat generating pad of the invention, the skin temperature is kept at from 32 to 50 °C, preferably from 32 to 45 °C, and more preferably from 32 to 39 °C for from 30 seconds to 24 hours, and in the case of relaxing the pain, a person who uses the heat generating pad may properly select it.

In this way, in sticking the heat generating pad of the invention to an affected part such that a muscle or tendon of the affected part is in parallel to the sectional exothermic parts, a reverse physical tension is continuously given to the tension of the muscle or tendon, whereby the tension of the muscle or tendon is relieved. Furthermore, since a fault strain is brought between the adjacent muscles or tendons and the physical tension is reinforced, the tension of the muscles or tendons is relieved. As a result, residence of vital energy and blood is dissolved, and a symptom of menstrual pain is lightened. In addition, a stimulus of a so-called "acupuncture point" by a pressing feeling to the affected part by the stiffness of the sectional exothermic parts or the thickness of the sectional exothermic parts is also effective for relaxing the symptom of menstrual pain. In addition, the heat generating pad has follow-up properties of fitting feeling to the body due to flexibility of the sectioned part and is excellent in feeling for use.

The heat generating pad of the invention can be used for relaxation of symptoms other than the symptom of menstrual pain and can be used for relaxation of a pain of a muscle or a bone structure and/or pains of the body as mentioned previously. For the purpose of achieving a desired remedy effect without causing malfunctioning, it is possible to adequately select a maximum skin temperature and the length of a time for keeping the skin temperature at a maximum skin temperature depending upon a human being who requires such remedy. Preferably, according to this method, by keeping the skin temperature which is continued against the body of a person who has an acute, repeated and/or chronic pain of the body including a pain of a muscle or a bone structure and/or pains of the body as mentioned previously at from about 32 °C to about 43 °C, preferably from about 32 °C to about 42 °C, more preferably from about 32 °C to about 41 °C, further preferably from about 32 °C to about 39 °C, and still further preferably from about 32 °C to about 37 °C for a period of time of about one hour or more, preferably about 4 hours or more, more preferably about 8 hours or more, further preferably about 16 hours or more, and still further preferably about 24 hours, an acute, repeated and/or chronic pain of the body including a pain of a bone structure or a muscle of the human being who has such a pain and/or pains of the body as mentioned previously such as abdominal pain and/or menstrual pain are lightened to considerable extent.

A raw material of the substrate or covering material is not limited so far as it functions as an accommodating bag of the heat generating composition. Usually, raw materials which are used in chemical body warmers or heat generating bodies can be used. Examples of the raw material include air-impermeable raw materials, air-permeable raw materials, water absorptive raw materials, non-water absorptive raw materials, non-extensible raw materials, extensible raw materials, stretchable raw materials, non-stretchable raw materials, foamed raw materials, non-foamed raw materials, non-heat sealable raw materials, and heat sealable raw materials. The raw material can be properly used depending upon a desired utility in a desired form such as films, sheets, non-woven fabrics, woven fabrics, and composites thereof. In general, the substrate is made of an air-impermeable film or sheet, and the covering material is made of an air-permeable film or sheet or non-woven fabric, and vice versa. The both may be air-permeable. As the underlay material, an air-permeable underlay material and an air-impermeable underlay material may be used for different purposes.

The packaging material of the accommodating bag may be of a single-layered structure or multilayered structure, and its structure is not limited. Furthermore, though the packaging material is composed of at least a substrate and a covering material, a packaging material for laminating the heat generating composition molded body is the substrate, and a packaging material for covering on the heat generating composition molded body is the covering material regardless of whether the packaging material is air-permeable or air-impermeable. An embodiment of a multilayered structure in which an air-impermeable packaging material is the substrate and an air-permeable packaging material is the covering material will be hereunder described as one example. That is, in this embodiment, the substrate is made of layer A/layer B, layer A/layer B/layer C, or layer A/layer B/layer C/layer D; and the covering material is made of layer F/layer G, layer E/layer F/layer G, or layer F/layer H/layer G. Examples of the layer A include thermoplastic resin films (for example, polyethylene), heat seal layers (for example, polyethylene and EVA), and water absorptive papers; examples of the layer B include non-woven fabrics of a thermoplastic resin (for example, nylons), non-water absorptive papers, water absorptive papers, thermoplastic resin films (for example, polyethylene films, polypropylene films, polyester films, and polyamide (for example, nylons) films), wicks (for example, non-water absorptive papers and water absorptive papers); examples of the layer C include adhesive layers, non-water absorptive papers, water absorptive papers, thermoplastic resin films (for example, polyethylene), non-slip layers, and non-woven fabrics of a thermoplastic resin (for example, polyesters and nylons); examples of the layer D include separators, thermoplastic resin films (for example, polyethylene), and non-woven fabrics; examples of the layer E include heat seal layers; examples of the layer F include porous films or perforated films made of a thermoplastic resin (for example, polyethylene), films made of a thermoplastic resin (for example, polyethylene), non-water absorptive papers, and water absorptive papers; examples of the layer G include non-woven fabrics of a thermoplastic resin (for example, polyesters and nylons) ; and examples of the layer H include non-water absorptive papers and water absorptive papers. Examples of the substrate or covering material include heat seal layer made of polyethylene obtained by using a metallocene catalyst/polypropylene film, polyethylene-made heat seal layer/polypropylene film, EVA-made heat seal layer/polypropylene film, EVA-made heat seal layer/polypropylene film/adhesive layer/separator, EVA-made heat seal layer/polyethylene film/nylon non-woven fabric, non-woven fabric/porous film, heat seal layer made of polyethylene obtained by using a metallocene catalyst/polyethylene film/nylon non-woven fabric, heat seal layer made of polyethylene obtained by using a metallocene catalyst/polypropylene film/polypropylene non-woven fabric, non-woven fabric/(paper and/or perforated (provided by a needle or laser) film)/porous film, non-woven fabric/ (paper and/or porous film) /perforated (provided by a needle or laser) film, and non-woven fabric/(paper and/or porous film)/non-woven fabric. A method for laminating the respective layers is not limited. The respective layers may be directly laminated; the respective layers may be laminated via an air-permeable adhesive layer or a laminating agent layer; and the respective layers may be laminated by hot melt extrusion or the like. Furthermore, in the invention, it is to be noted that polyethylene produced by using a metallocene catalyst is also included in the polyethylene.

For example, in the case of laminating the foregoing raw material such as non-woven fabrics and porous films via an air-permeable sticky layer, examples of a method for forming the air-permeable sticky layer include a method in which a sticky substance is fibrillated by an appropriate system such as a curtain spray system, a melt blow system or a slot spray system for blowing and spreading a sticky substance via hot air under heat melting and spread and accumulated on an appropriate supporting substrate made of a porous film, an air-permeable substrate, a separator, etc., thereby forming a porous sticky layer.

A thickness of each of the substrate, the covering material, the underlay material, and the raw material constituting the same varies depending upon the utility and is not limited. The thickness is usually from 5 to 5,000 $\mu$m, preferably from 10 to 500 $\mu$m, and more preferably from 20 to 250 $\mu$m.

The air-impermeable raw material is not limited so far as it is air-impermeable. Examples thereof include films, sheets or coatings made of a polymer (for example, polyethylene, polypropylene, nylons, polyacrylates, polyesters, polyvinyl alcohols, and ethylene-vinyl acetate copolymers) and laminates thereof with a metal (including a semiconductor) compound (for example, silicon oxide) or composite raw materials using the same.

Of the foregoing air-impermeable raw materials, examples of a film having high air impermeability include films provided with a single layer or multiple layers of a thin film having a metal including a semiconductor or a compound thereof provided on an air-impermeable raw material film. Examples of the metal including a semiconductor include silicon, aluminum, and alloys or mixtures containing such a metal. Examples of the metal (including a semiconductor) compound include oxides, nitrides and oxynitrides of the foregoing metals or alloys or mixtures. Examples of the layer include silicon oxide layers, aluminum oxide layers, and silicon oxynitride layers; layers obtained by laminating an arbitrary layer of these layers on a polyester-made film; and layers obtained by further laminating a stretched polyolefin film (for example, a biaxially stretched polypropylene film) thereon.

The air-permeable raw material is not limited so far as it is air-permeable. Examples thereof include air-permeable films (for example, porous films and perforated films); materials having air permeability by themselves (for example, papers and non-woven fabrics); materials prepared by laminating at least one of papers and air-permeable films and non-woven fabrics so as to have air permeability; materials prepared by providing an air-impermeable packaging material comprising a non-woven fabric having a polyethylene film laminated thereon with fine pores by using a needle, etc. so as to have air permeability; non-woven fabric whose air permeability is controlled by laminating a fiber and heat bonding under pressure; porous films; and materials prepared by sticking a non-woven fabric onto a porous film. The "perforated film" as referred to herein is a film prepared by providing an air-impermeable film (for example, polyethylene films) with fine pores by using a needle so as to have air permeability.

The air permeability is not limited so far as the heat generation can be kept. In the case of use in usual heat generation, the air permeability is usually from 50 to 10,000 $g/m^2/24$ hr, preferably from 70 to 5,000 $g/m^2/24$ hr, more preferably from 100 to 2, 000 $g/m^2/24$ hr, and further preferably from 100 to 700 $g/m^2/24$ hr in terms of moisture permeability by the Lyssy method.

When the moisture permeability is less 50 $g/m^2/24$ hr, the heat value is small and a sufficient thermal effect is not obtained, and therefore, such is not preferable. On the other hand, when it exceeds 10,000 $g/m^2/24$ hr, the exothermic temperature is high so that a problem in safety may possibly be generated, and therefore, such is not preferable. However, there is no limitation even when the moisture permeability exceeds 10, 000 $g/m^2/24$ hr depending upon the utility, or even in the use at a moisture permeability closed to the open system, according to circumstances.

The stretchable packaging material is not particularly limited so far as it is stretchable. That is, it is only required that the stretchable packaging material is stretchable as a whole. The stretchable packaging material may be formed of a single material or a composite material of stretchable substrates or a combination of a stretchable substrate and a non-stretchable substrate.

Examples of the stretchable packaging material include single materials (for example, natural rubbers, regenerated rubbers, synthetic rubbers, elastomers, and stretchable shape memory polymers) and mixtures thereof, mixed materials or blended materials of such a stretchable raw material and a non-stretchable raw material or fabrics constituted of a combination of these materials, films, yarns, strands, ribbons, tapes, and stretchable films with a scrim structure.

The porous film is not limited and can be properly selected among porous films obtained by stretching a film made of a polyolefin based resin (for example, polyethylene, linear low density polyethylene, and polypropylene) or a fluorine based resin (for example, polytetrafluoroethylene) and a filler.

The non-woven fabric is not limited. Single non-woven fabrics of a single fiber or composite fiber made of a material such as rayon, nylons (polyamides), polyesters, polyacrylates, polypropylene, vinylon, polyethylene, polyurethane, cupra, cotton, cellulose, and pulp, or laminates of blended or accumulated fiber layers of such fibers are useful. Furthermore, from the standpoint of production process, dry non-woven fabrics, wet non-woven fabrics, spunbonds, spunlaces, and the like can be used. Non-woven fabrics made of a composite fiber having a core-sheath structure are also useful. A non-woven fabric in the side which is brought into contact with the skin is preferably a napping (fluffy) non-woven fabric. Also, stretchable non-woven fabrics and non-stretchable non-woven fabrics are useful.

The water absorptive raw material is not particularly limited so far as it is a water absorptive film or sheet.

The water absorptive raw material is not particularly limited so far as it has water absorption properties consequently regardless of whether or not the raw material has water absorption properties by itself.

Specific examples thereof include water absorptive foamed films or sheets having water absorption properties (for example, foamed bodies of water absorptive foamed polyurethane, etc.) or papers, non-woven fabrics or woven fabrics formed of a fiber having water absorption properties, non-woven fabrics or woven fabrics containing a fiber having water absorption properties, and water absorptive materials such as water absorptive porous films or sheets. Besides, there are enumerated materials in which regardless of the presence or absence of water absorption properties, a water absorbing agent is contained, impregnated, kneaded, transferred or carried on a foamed film or sheet, a non-woven fabric, a woven fabric or porous film or sheet, thereby imparting or increasing water absorption properties; and materials in which regardless of the presence or absence of water absorption properties, a water absorptive raw material such as water absorptive foamed films or sheets, papers, non-woven fabrics, woven fabrics, and porous films or sheets as cut in a planar shape according to the invention is attached to one side or both sides of the material according to the invention, thereby imparting water absorption properties.

In particular, in the heat generating body of the invention, for the purpose of forming the plane which is brought into contact with the skin into a comfortable plane by imparting water absorption properties against sweat, etc., in order that in the case of sweating, the sweat is absorbed, it is preferable that a packaging material in the plane which is brought into contact with the skin is constituted of a packaging material using a non-woven fabric or a woven fabric containing, as the major component, a water absorptive fiber having a water retention of 20 % or more. Examples of the water absorptive fiber having a water retention of 20 % or more include cottons, silks, hemps, wools, polyacrylonitrile based synthetic fibers, polyamide based synthetic fibers, polyvinyl alcohol based synthetic fibers, acetate fibers, triacetate fibers, and regenerated fibers. In addition, non-woven fabrics having a highly water absorptive polymer held in a non-woven fabric can be used as the non-woven fabric having excellent water absorption properties. Incidentally, non-woven fabrics or woven fabrics containing such a fiber as the major component are relatively good with respect to the feeling against the skin.

In addition, highly water absorptive packaging materials having high absorption properties of sweat can be used as the packaging material. Examples thereof include non-woven fabrics containing a fiber whose surface is coated with a highly water absorptive resin, non-woven fabrics containing a hollow fiber having a number of fine pores on the surface thereof, and non-woven fabrics containing a fiber having a capillary action by forming a number of pouches or plural layers in the cross-sectional shape.

Besides, non-woven fabrics or films having a water absorptive inorganic compound held on a non-sticky surface of a packaging material can be used. Examples thereof include non-woven fabrics resulting from holding a powder (for example, diatomaceous earth, zeolite, and silica gel) on a non-woven fabric and films resulting from holding a relatively large amount of a powder (for example, silica and alumina) on a synthetic resin (for example, polyethylene).

In addition, highly water absorptive packaging materials having high absorption properties of sweat can be used as the packaging material. Examples thereof include non-woven fabrics containing a fiber whose surface is coated with a highly water absorptive resin, non-woven fabrics containing a hollow fiber having a number of fine pores on the surface thereof, and non-woven fabrics containing a fiber having a capillary action by forming a number of pouches or plural layers in the cross-sectional shape.

Besides, non-woven fabrics or films having a water absorptive inorganic compound held on the surface of a packaging material to be adhered can be used. Examples thereof include non-woven fabrics resulting from holding a powder (for example, diatomaceous earth, zeolite, and silica gel) on a non-woven fabric and films resulting from holding a relatively large amount of a powder (for example, silica and alumina) on a synthetic resin (for example, polyethylene).

Furthermore, multilayered materials in which the skin side is made of a raw material having poor water absorption capability (for example, polystyrene, polypropylene, polyesters, and nylons) and the exothermic part side is made of a water absorptive raw material, a water absorptive polymer-provided packing material, etc. may be employed. Examples of the form thereof include non-woven fabrics, meshes, woven fabrics, and knitted fabrics.

As the thermal buffer sheet, the foregoing substrates, covering materials, non-woven fabrics and water absorptive raw materials can be used.

In the invention, as a heat seal material constituting a heat seal layer, a single raw material may be used, or a composite raw material having a heat seal layer may be used. The heat seal material is not limited so far as at least a part thereof can be welded upon heating. Examples thereof include hot melt based resins such as polyolefins (for example, polyethylene and polypropylene) or olefin copolymer resins, ethylene based hot melt resins (for example, ethylene-vinyl acetate copolymer resins and ethylene-acrylic acid ester copolymer resins (for example, ethylene-isobutyl acrylate copolymer resins)), polyamide based hot melt resins, butyral based hot melt resins, polyester based hot melt resins, polyamide based hot melt resins, polyester based hot melt resins, polymethyl methacrylate based hot melt resins, polyvinyl ether based hot melt resins, polyurethane based hot melt resins, polycarbonate based hot melt resins,such as polyvinyl acetate, and vinyl chloride-vinyl acetate copolymers; and films or sheets thereof. Furthermore, in these hot melt based resins or films or sheets thereof, ones having various additives (for example, an antioxidant) compounded therein can be used. In particular, low density polyethylene and polyethylene obtained by using a metallocene catalyst are useful.

In the case of interposing a heat generating composition molded body between a substrate and a covering material, the "temporary adhesion" as referred to in the invention means weak pressure-sensitive bonding or adhesion for the purpose of holding the accommodated heat generating composition molded body until at least the substrate and the covering material are adhered to each other via a sticky layer made of an adhesive and heat sealed.

Furthermore, the "deadhesion" as referred to herein means that in the temporary adhering seal part after heat seal, the heat generating composition in a non-heat sealed region is transferred to the foregoing region, thereby releasing the temporary adhesion.

The temporary adhering seal part is formed via a sticky layer. An adhesive constituting the sticky layer is not limited so far as it is a layer formed of a polymer composition which is tacky at the normal temperature and can be heat sealed after the temporary adhesion.

Furthermore, although the adhesive of the foregoing adhesive layer can be used as the adhesive constituting the sticky layer to be used for the temporary adhesion, a non-hydrophilic adhesive is preferable. As the adhesive constituting the sticky layer, one which is well compatible with the heat seal material constituting the heat seal is preferable, and a melting point of a base polymer of the adhesive is preferably not higher than a melting point of the heat seal material. In particular, hot melt based adhesives are preferable. Furthermore, in the case where the heat seal material is made of an olefin based raw material, preferred examples of the adhesive include olefin based adhesives.

Incidentally, a method for providing a sticky layer for the temporary adhesion is not limited. The sticky layer may be entirely provided or partially or intermittently provided. Examples of its shape include various shapes such as a network-like shape, a stripe-like shape, a dot-like shape, and strip-like shape.

The fixing means is not limited so far as it has capability for fixing a thermal packaging body for joint surroundings or a material having an exothermic part to a prescribed part.

As the fixing means, an adhesive layer, a hook and eye, a hook and button, a hook and loop fastener such as Velcro, a magnet, a band, a string, and combination thereof can be arbitrarily used.

Incidentally, in the case of a band, fixing means for adjustment may be further constructed by a combination of a hook and loop fastener and an adhesive layer.

Here, the "hook and loop fastener" as referred to herein has a fastening function by a combination of a loop as a female fastener with a male fastener capable of fastening the female fastener thereto, which is known as trade names such as Magic Tape (a registered trademark), Magic Fastener (a registered trademark), Velcro Fastener, and Hook and Loop Tape. Examples of the material having a loop function include non-woven fabrics and woven fabrics of napped or hole-containing yarns. Such a material having a loop function (female fastener function) may be covered on the surface of a paddling forming the band, or the band may be constructed of such a material itself. Although the hook member which is the male fastener member is not particularly limited, examples thereof include hook members formed of a polyolefin based resin (for example, polyethylene and polypropylene), a polyamide, a polyester, etc. Although the shape of the hook is not particularly limited, a hook having a cross-sectional shape such as an I type, an inverted L type, an inverted J type, and a so-called mushroom type is preferable because it is easily hooked by the loop and does not give an extreme stimulus to the skin. Incidentally, the hook may be adhered to the entire area of a fastening tape, and only the hook may be used as a fastening tape while omitting a tape substrate.

The adhesive layer may contain at least one member selected from additional components consisting of a water retaining agent, a water absorptive polymer, a pH adjusting agent, a surfactant, an organosilicon compound, a hydrophobic polymer compound, a pyroelectric substance, an antioxidant, an aggregate, a fibrous material, a moisturizer, a functional substance, and a mixture thereof.

The adhesive of the invention is classified into a non-hydrophilic adhesive, a mixed adhesive, and a hydrophilic adhesive (for example, a gel).

The adhesive constituting the adhesive layer is not limited so far as it has an adhesive strength necessary for adhering to the skin or clothes. Adhesives of every form such as a solvent based adhesive, an aqueous adhesive, an emulsion type adhesive, a hot melt type adhesive, a reactive adhesive, a pressure-sensitive adhesive, a non-hydrophilic adhesive, and a hydrophilic adhesive are employable.

The adhesive layer includes one layer of a non-hydrophilic adhesive constituted of the non-hydrophilic adhesive and non-hydrophilic adhesive layers constituted of the non-hydrophilic adhesive.

It is to be noted that a material whose water absorption properties are improving by containing a water absorptive polymer or a water retaining agent in the non-hydrophilic adhesive layer is dealt as the non-hydrophilic adhesive layer.

A hot melt based adhesive may be provided between the hydrophilic adhesive layer and a substrate or a covering material. Furthermore, in the case where the hydrophilic adhesive is provided in a thermal packaging body for joint surroundings, there is no limitation. After seal treating a thermal packaging body for joint surroundings, a hydrophilic adhesive layer may be provided in the thermal packaging body for joint surroundings.

Furthermore, the adhesive layer may or may not have air permeability and may be properly selected depending upon the utility. With respect to the air permeability, the adhesive layer may be air-permeable as a whole. Examples thereof include an adhesive layer having air permeability as a whole of a region in which an adhesive is partially present and a portion where no adhesive is present is partially present.

In laminating an adhesive on an air-permeable substrate and/or a covering material in a stratiform state as it is, examples of a method for keeping its air permeability include a method in which an adhesive layer is partially laminated by printing or transferring an adhesive, thereby forming a non-laminated part as an air-permeable part; a method in which an adhesive is transferred in one direction while drawing a circle in a filament-like form or properly moved in the two-dimensional directions by transferring in a zigzag manner, whereby a space of the filament-like adhesive keeps air permeability or moisture permeability or the adhesive is foamed; and a method for forming a layer by a melt blow system.

Examples of the adhesive which constitutes the non-hydrophilic adhesive layer include acrylic adhesives, polyvinyl acetate based adhesives (for example, vinyl acetate resin based emulsions and ethylene-vinyl acetate resin based holt melt adhesives), polyvinyl alcohol based adhesives, polyvinyl acetal based adhesives, vinyl chloride based adhesives, polyamide based adhesives, polyethylene based adhesives, cellulose based adhesives, chloroprene (neoprene) based adhesives, nitrile rubber based adhesives, polysulfide based adhesives, butyl rubber based adhesives, silicone rubber based adhesives, styrene based adhesives (for example, styrene based hot melt adhesives), rubber based adhesives, and silicone based adhesives. Of these, rubber based adhesives, acrylic adhesives, and adhesives containing a hot melt based polymer substance for the reasons that they are high in the adhesive strength, are cheap, are good in long-term stability, and are small in reduction of the adhesive strength even by providing heat.

In addition to the base polymer, if desired, the adhesive may be compounded with other components such as tackifiers (for example, petroleum resins represented by rosins, chroman-indene resins, hydrogenated petroleum resins, maleic anhydride-modified rosins, rosin derivatives, and C-5 based petroleum resins), phenol based tackifiers (especially, tackifiers having an aniline point of not higher than 50 °C; for example, terpene phenol based resins, rosin phenol based resins, and alkylphenol based resins), softeners (for example, coconut oil, castor oil, olive oil, camellia oil, and liquid paraffin), softeners, anti-aging agents, fillers, aggregates, adhesion adjusting agents, adhesion modifiers, coloring agents, anti-foaming agents, thickeners, and modifiers, thereby improving performance such as an improvement in adhesion to nylon-made clothes and mixed yarn clothes.

Examples of the hot melt based adhesive include known hot melt based adhesives imparted with adhesion. Specific examples thereof include styrene based adhesives made of, as a base polymer, an A-B-A type block copolymer (for example, SIS, SBS, SEBS, and SIPS), vinyl chloride based adhesives made of, as a base polymer, a vinyl chloride resin, polyester based adhesives made of, as a base polymer, a polyester, polyamide based adhesives made of, as a base polymer, a polyamide, acrylic adhesives made of, as a base polymer, an acrylic resin, polyolefin based adhesives made of, as a base polymer, a polyolefin (for example, polyethylene, super low density polyethylene, polypropylene, ethylene-$\alpha$-olefin copolymers, and ethylene-vinyl acetate copolymers), 1,2-polybutadiene based adhesives made of, as a base polymer, 1,2-polybutadiene, and polyurethane based adhesives made of, as a base polymer, polyurethane; adhesives made of a modified body of the foregoing adhesive whose adhesion is improved or whose stability is changed; and mixtures of two or more kinds of these adhesives. Adhesive layers constituted of a foamed adhesive and adhesive layers constituted of a crosslinked adhesive can also be employed.

The non-aromatic hot melt based adhesive is not limited so far as it is made of, as a base polymer, a hot melt based adhesive not containing an aromatic ring. Examples thereof include olefin based hot melt based adhesives and acrylic hot melt based adhesives. As the non-aromatic polymer which is the base polymer not containing an aromatic ring, there are enumerated polymers or copolymers of an olefin or a diene. Examples thereof include olefin polymers. The olefin polymer includes polymers or copolymers of ethylene or an $\alpha$-olefin. Also, polymers resulting from adding a diene (for example, butadiene and isoprene) as other monomer thereto may be employed.

The $\alpha$-olefin is not limited so far as it is a monomer having a double bond in the terminal thereof. Examples thereof include propylene, butene, heptane, hexene, and octene.

The "aromatic hot melt based adhesive" as referred to herein is a hot melt based adhesive whose base polymer contains an aromatic ring. Examples thereof include styrene based hot melt based adhesives represented by A-B-A type block copolymers.

In the foregoing A-B-A type block copolymers, the A block is a non-elastic polymer block made of a monovinyl substituted aromatic compound A such as styrene and methylstyrene; and the B block is an elastic polymer block made of a conjugated diene such as butadiene and isoprene. Specific examples thereof include a styrene-butadiene-styrene block copolymer (SBS), a styrene-isoprene-styrene block copolymer (SIS), and hydrogenated types thereof (for example, SEBS and SIPS), and mixtures thereof.

As a countermeasure for preventing a lowering of adhesive strength caused due to an increase of water of the non-hydrophilic adhesive layer, an adhesive layer obtained by further compounding a water absorptive polymer in the non-hydrophilic adhesive can be used.

The hydrophilic adhesive which constitutes the hydrophilic adhesive layer is not particularly limited so far as it contains a hydrophilic polymer or a water-soluble polymer as the major component, has adhesion and is hydrophilic as an adhesive.

Examples of the constitutional components of the hydrophilic adhesive include hydrophilic polymers (for example, polyacrylic acid), water-soluble polymers (for example, poly(sodium acrylate) and polyvinylpyrrolidone), crosslinking agents (for example, dry aluminum hydroxide and meta-silicic acid aluminic acid metal salts), softeners (for example, glycerin and propylene glycol), higher hydrocarbons (for example, soft liquid paraffin and polybutene), primary alcohol fatty acid esters (for example, isopropyl myristate), silicon-containing compounds (for example, silicone oil), fatty acid glycerin esters (for example monoglycerides), oily components (for example, vegetable oils such as olive oil), antiseptics (for example, methyl p-hydroxybenzoate and propyl p-hydroxybenzoate), solubilizing agents (for example, N-methyl-2-pyrrolidone), thickeners (for example, carboxymethyl cellulose), surfactants (for example, polyoxyethylene hardened castor oil and sorbitan fatty acid esters), hydroxycarboxylic acid (for example, tartaric acid), excipients (for example, light silicic anhydride, water absorptive polymers, and kaolin), moisturizers (for example, D-sorbitol), stabilizers (for example, sodium edetate, p-hydroxybenzoic acid esters, and tartaric acid), crosslinking type water absorptive polymers, boron compounds (for example, boric acid), and water. They may be used as an arbitrary combination.

A temporary adhering seal part is formed via a sticky layer. An adhesive which constitutes the sticky layer is a layer formed of a polymer composition which is tacky at the normal temperature and is not limited so far as it can be heat sealed after temporary adhesion.

Furthermore, the foregoing adhesives of the sticky layer can be used as the adhesive which constitutes the sticky layer as used for temporary adhesion. Of these, non-hydrophilic adhesives are preferable. With respect to the adhesive constituting the adhesive layer, it is preferable that the adhesive is well compatible with a heat seal material constituting a heat seal and that a melting point of the base polymer of the adhesive is not higher than a melting point of the heat seal material. Hot melt based adhesives are especially preferable for hot melt based bonding agents. Furthermore, in the case where the heat seal material is an olefin based raw material, preferred examples thereof include olefin based adhesives.

A bonding layer for fixing the air permeability adjusting material is constituted of a bonding agent or an adhesive which is usually used. In particular, an adhesive is useful, and the foregoing adhesives for constituting the adhesive layer can be used.

Furthermore, a method for providing a bonding layer is not limited so far as the air permeability adjusting material can be fixed. The bonding layer may be entirely provided or partially or intermittently provided. Examples of its shape include various shapes such as a network-like shape, a stripe-like shape, a dot-like shape, and strip-like shape.

Furthermore, in the case where an adhesive layer is employed as the hydrophilic adhesive layer, if there is a difference in a water retaining force between the hydrophilic adhesive layer and the heat generating composition molded body, transfer of water occurs via a packaging material present therebetween such as a substrate, thereby causing in-conveniences against the both. In particular, the transfer of water occurs during the storage. In order to prevent this, it is preferable that the packaging material present therebetween at least has a moisture permeability of not more than 2 $g/m^2/day$ in terms of a moisture permeability according to the Lyssy method. By using this, in the case where the heat generating body is accommodated in an outer bag as an air-impermeable accommodating bag and stored, the transfer of water can be prevented.

In the case where a hydrophilic adhesive layer is used as the adhesive layer, the moisture permeability of a moisture-proof packaging material provided between the heat generating composition molded body and the hydrophilic adhesive layer is not limited so far as the transfer of water can be prevented within the range where the exothermic performance is not affected. The moisture permeability according to the Lyssy method is usually not more than 2 $g/m^2/day$, preferably not more than 1.0 $g/m^2/day$, more preferably not more than 0.5 $g/m^2/day$, and further preferably from 0.01 to 0.5 $g/m^2/day$. These values are a value under a condition under an atmospheric pressure at 40 °C and 90 % RH. Incidentally, the moisture-proof packaging material can be used as a substrate or a covering material and may be laminated singly on a substrate, a covering material, or the like.

The moisture-proof packaging material is not limited so far as the transfer of water between the heat generating composition molded body and the hydrophilic adhesive layer can be prevented. Examples thereof include metal vapor deposited films, vapor deposited films of a metal oxide, metal foil-laminated films, EVOH (ethylene/vinyl alcohol copolymer or ethylene/vinyl acetate copolymer saponified product) based films, biaxially stretched polyvinyl alcohol films, polyvinylidene chloride coated films, polyvinylidene chloride coated films obtained by coating polyvinylidene chloride on a substrate film (for example, polypropylene), metal foils such as an aluminum foil, air-impermeable packaging materials obtained by vapor depositing or sputtering a metal (for example, aluminum) on a polyester film substrate, and packaging laminates using a transparent barrier film of a structure in which silicon oxide or aluminum oxide is provided on a flexible plastic substrate. The air-impermeable packaging materials which are used in the outer bag, etc. can also be used.

Furthermore, packaging materials such as moisture-proof packaging materials as described in JP-A-2002-200108, the disclosures of which can be incorporated herein by reference, can be used.

In the case of using a water-containing hydrophilic adhesive (for example, a gel) in the adhesive layer, in order to adjust the moisture equilibrium between the heat generating composition and the adhesive layer, the content of a reaction accelerator (for example, sodium chloride) or a substance having a water holding power (for example, a water absorptive polymer) in the heat generating composition may be adjusted within the range of from 10 to 40 % by weight, preferably from 15 to 40 % by weight, and more preferably from 15 to 30 % by weight based on the heat generating composition. Furthermore, as the adhesive having good moisture permeability and low stimulation to the skin, water-containing adhesives (for example, hydrophilic adhesives and gels) as described in JP-A-10-265373 and JP-A-9-87173, adhesives which can be subjected to hot melt coating as described in JP-A-6-145050 and JP-A-6-199660, and rubber based adhesives as described JP-A-10-279466 and JP-A-10-182408, the disclosures of which are totally incorporated herein by reference, are useful.

The functional substance which is contained in the adhesive layer is not limited so far as it is a substance having any function. There can be enumerated at least one member selected from aromatic compounds, vegetable extracts, crude drugs, perfumes, slimming agents, analgesics, blood circulation promoters, swelling improvers, antibacterial agents, sterilizers, mold inhibitors, odor eaters, deodorants, percutaneously absorptive drugs, fat-splitting components, minus ion generators, far infrared ray radiants, magnetic bodies, fomentations, cosmetics, bamboo vinegar, and wood vinegar. Specific examples thereof include aromatic compounds (for example, menthol and benzaldehyde), vegetable extracts (for example, mugwort extract), crude drugs (for example, moxa), perfumes (for example, lavender and rosemary), slimming agents (for example, aminophylline and tea extract), analgesic drugs (for example, indomethacin and dl-camphor), blood circulation promoters (for example, acidic mucopolysaccharide and chamomile), swelling improvers (for example, horse chestnut extract and flavone derivatives), fomentations (for example, aqueous boric acid, physiological saline, and aqueous alcohols), fat-splitting components (for example, jujube extract, caffeine, and tonalin), cosmetics (for example, aloe extracts, vitamin preparations, hormone preparations, anti-histamines, and amino acids), antibacterial agents and sterilizers (for example, carbolic acid derivatives, boric acid, iodine preparations, invert soaps, salicylic acid based substances, sulfur, and antibiotics), and mold inhibitors.

The percutaneously absorptive drug is not particularly limited so far as it has percutaneous absorption. Examples thereof include corticosteroids, anti-inflammatory drugs, hypertension drugs, anesthetics, hypnotic sedatives, tranquillizers, antibacterial substances, antifungal substances, skin stimulants, inflammation inhibitors, anti-epileptics, analgesics, antipyretics, anesthetics, mold inhibitors, antimicrobial antibiotics, vitamins, antiviral agents, swelling improvers, diuretics, antihypertensives, coronary vasodilators, anti-tussive expectorants, slimming agents, anti-histamines, antiarrhythmic agents, cardiotonics, adrenocortical hormones, blood circulation promoters, local anesthetics, fat-splitting components, and mixtures thereof. However, it should not be construed that the invention is limited thereto. These drugs are used singly or in admixture of two or more kinds thereof as the need arises.

The content of such a functional substance is not particularly limited so far as it falls within the range where the effect of a medicine can be expected. However, from the viewpoints of adhesive strength as well as pharmacological effect and economy, the content of the functional substance is preferably from 0.01 to 25 parts by weight, and more preferably from 0.5 to 15 parts by weight based on 100 parts by weight of the adhesive.

Furthermore, a method for providing the adhesive layer is not limited so far as a thermal packaging body for joint surroundings can be fixed. The adhesive layer may be entirely provided or partially or intermittently provided. Examples of its shape include various shapes such as a network-like shape, a stripe-like shape, a dot-like shape, and strip-like shape.

The bonding layer for fixing the air permeability adjusting material to a raw material in a region which is brought into direct contact with the heat generating composition is not limited so far as it can achieve fixing. Examples of a material constituting the bonding layer include an adhesive, a heat seal material, and a bonding agent.

The fixing region between the air permeability adjusting material and the exothermic part is not limited so far as the both can be fixed and air can go in and out from at least the periphery of the sectional exothermic part. However, the following can be enumerated.

1) The fixing region is fixed in the both ends of the exothermic part or heat generating body.

2) A space is provided entirely in a substantially central part of the exothermic part, and other exothermic part region is defined as the fixing region.

3) A substantially top part of each sectional exothermic part and a substantially central part of each sectioned part are defined as the fixing region.

Here, as the air permeability adjusting material, any material can be used so far as it is provided with a space which communicates with the outside in the surroundings of the sectional exothermic part. Examples of an air permeability adjusting material having a bonding layer and utilizing a plastic film include PE/adhesive, PP/adhesive, polyester/adhesive, PE/non-woven fabric/air-permeable adhesive, PE/non-woven fabric/PE/adhesive, PE/PET/M/PE/non-woven fabric/air-permeable adhesive, PE/heat seal material, PE/non-woven fabric/heat seal material, PE/non-woven fabric/PE/heat seal material, and PE/polyester/M/PE/non-woven fabric/heat seal material. Here, M represents a metal (for example, aluminum and silver), a semiconductor (for example, silicon oxide, silicon oxynitride, silicon nitride, and aluminum oxide), or a metal oxide, oxynitride or nitride. Furthermore, a portion for placing fixing means such as an adhesive layer and a heat sealing agent layer is not limited, and whether it is provided partially or entirely may be properly determined depending upon the intended purpose.

The bonding layer for fixing the air permeability adjusting material is not limited so far as the air permeability adjusting material can be fixed to the heat generating body and is constituted of a usually used bonding agent or adhesive. In particular, an adhesive is useful, and the adhesive constituting the foregoing adhesive layer can be used.

Furthermore, a method for providing the bonding layer is not limited so far as the air permeability adjusting material can be fixed. The bonding layer may be entirely provided or partially or intermittently provided. Examples of its shape include various shapes such as a network-like shape, a stripe-like shape, a dot-like shape, and strip-like shape. Its thickness is not particularly limited but is in the range of from 5 to 1, 000 $\mu$m, preferably from 10 to 500 $\mu$m, and more preferably from 15 to 250 $\mu$m. When the thickness of the bonding layer is less than 5 $\mu$m, a desired adhesive strength may not be possibly obtained. On the other hand, when it exceeds 1,000 $\mu$m, not only it becomes bulky and becomes worse in feeling for use, but also it becomes worse in economy, and therefore, such is not preferable.

The bonding substance which constitutes the bonding layer is not limited so far as it can fix the air permeability adjusting material to the heat generating pad. Examples thereof include heat seal materials and adhesives.

The raw material which constitutes the packaging material and the foregoing heat seal material and adhesive can be used for the air adjusting material, the raw material which constitutes the bonding layer or sticky layer, the heat seal material, and the adhesive.

The "air-permeable adjusting material" as referred to in the invention comprises a sectional exothermic part and a sectioned part and covers an exothermic part having a difference of altitude via an adhesive layer, etc., thereby adjusting the air permeability into the sectional exothermic part. That is, in the air permeability adjusting material, by covering the exothermic part by the air-permeable adjusting material while utilizing a difference of altitude between the sectional exothermic part and the sectioned part, a partitioned space is formed in at least a part of the periphery of the sectional exothermic part, thereby adjusting the air permeability between the outside and the sectional exothermic part and also imparting a heat insulating effect.

The air permeability of the air permeability adjusting material is not limited so far as it is able to adjust air retention or air permeability in at least a part of the periphery of the sectional exothermic part. However, it is preferable that the air permeability of the air permeability adjusting material is lower than that on the air-permeable surface of the sectional exothermic part as a covering part for covering the heat generating composition molded body.

Furthermore, a region where the air permeability is higher than that in the covering part for covering the heat generating composition molded body may be provided in a local region of the air-permeable adjusting material, thereby keeping the air permeability of other region lower than that on the air-permeable surface of the sectional exothermic part. In this way, it is possible to control an air passage for air, etc.

As the raw material which constitutes the air permeability adjusting material, the substrate and covering material of a chemical body warmer or a heat generating pad and the raw material which is used in a packaging material to be used in an air-impermeable accommodating bag for sealing and accommodating the heat generating pad can be used. Above all, adhesives which are used in a chemical body warmer or a heat generating pad are preferable.

The heat generating body excluding the air-permeable adjusting material is not limited with respect to the heat generating composition, the accommodating bag and the raw material constituting the same so far as it is a heat generating body comprising a sectional exothermic part for accommodating the heat generating composition and a sectioned part as a seal part and having a difference of altitude. However, a heat generating body having a heat generating composition molded body accommodated in an air-permeable accommodating bag, which is produced from a moldable heat generating composition containing surplus water as a connecting substance by a molding system, is preferable. The detailed description will be given hereunder.

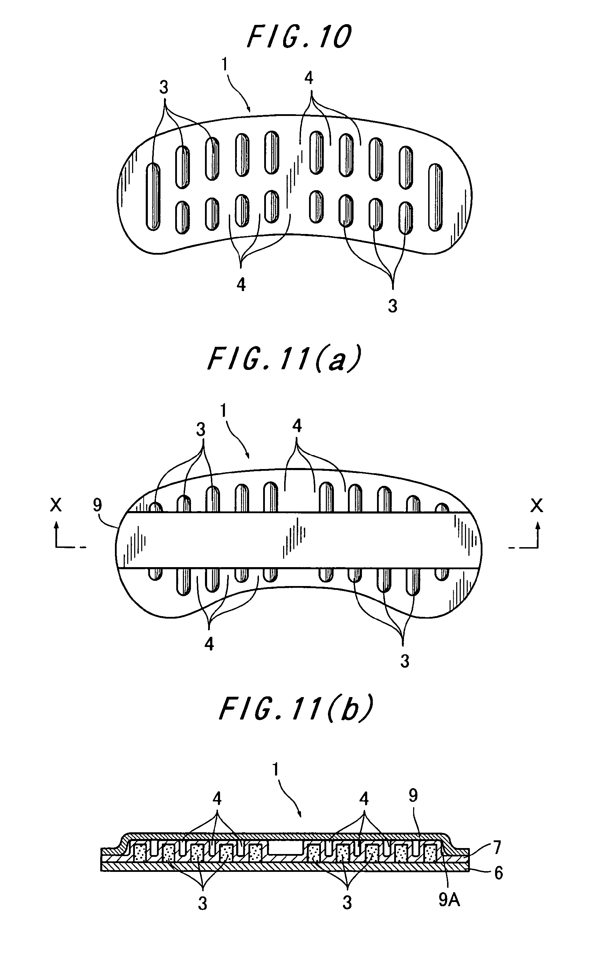

Here, the heat generating pad using an air permeability adjusting material will be described. Fig. 11(a) is a plan view of an embodiment of the heat generating pad in which a region including the whole of plural sectional exothermic parts and the both end parts in one direction of the heat generating pad as well as the surroundings of the sectional exothermic part is covered by the air permeability adjusting material; the sectioned part works as a concave; the sectional exothermic part works as a convex; the sectional exothermic part works as a support of the air permeability adjusting material; a spacial air-permeable layer is made of a spacial part which is constituted of the air permeability adjusting material and the sectioned part; and an air hole 16 constituted of the both end parts of the sectioned part, the sectional exothermic part and the air permeability adjusting material works as an air intake. Fig. 11 (b) is a cross-sectional view along the line X-X of Fig. 11(a).

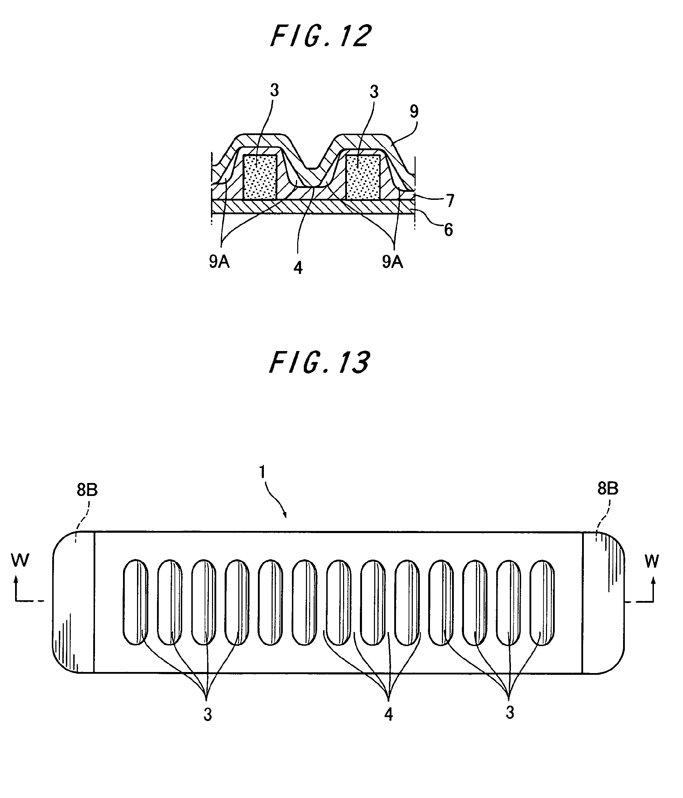

Fig. 12 shows an example in which a part of the air permeability adjusting material is fixed in substantially the central part of the sectioned part via sticky layer, etc. and the special part in Fig. 12(b) is divided into two parts by the air permeability adjusting material, whereby the respective sectional exothermic parts have an independent spacial part.

The sticky layer can be constituted of the foregoing adhesive. The thickness of the sticky layer is not limited so far as temporary adhesion can be carried out. It is preferably from 0.001 to 10 $g/m^2$, more preferably from 0.001 to 8 $g/m^2$, further preferably from 0.001 to 6 $g/m^2$, still further preferably from 0.001 to 5 $g/m^2$, and even further preferably from 0.001 to 4.999 $g/m^2$. Furthermore, in the case where after temporary adhesion and heat sealing, a non-heat sealed part is subjected to deadhesion, the thickness is preferably from 0.001 to 1 $g/m^2$, more preferably from 0.001 to 0.8 $g/m^2$, further preferably from 0.01 to 0.8 $g/m^2$, still further preferably from 0.1 to 0.8 $g/m^2$, and even further preferably from 0.01 to 0.5 $g/m^2$. When the thickness of the adhesive layer is less than 0.001 $g/m^2$, there may be the case where a required adhesive strength is not obtained. On the other hand, when it exceeds 10 $g/m^2$, the strength of a heat seal becomes weak and therefore, such is not preferable.

In the heat generating pad of the invention, the bending resistance in two directions substantially orthogonal to each other is made different. The heat generating pad is flexible in one direction, whereas its flexibility in the direction substantially orthogonal thereto is limited. Thus, the heat generating pad of the invention is not only excellent in adhesion to the body but also very excellent in usefulness.

For example, in a heat generating pad which is prepared by providing twelve sectional exothermic parts having a length of the long side of 124 mm and a length of the width as the short side of 10 mm substantially in parallel at equal intervals of 4 mm, the circumferential surroundings of the heat generating pad are heat sealed in a width of 8 mm and cutting while leaving the heat seal in the circumferential surroundings of the heat generating pad, an absolute value of a difference between bending resistances in the two directions substantially orthogonal to each other becomes maximal so that the heat generating pad is very excellent in usefulness.

The outer bag is not limited so far as it is impermeable to air, and it may be made of a laminate. Examples thereof include nylon, polyester and polypropylene films which are subjected to a moisture-proof treatment with OPP, CPP, polyvinylidene chloride, metal oxides (including semiconductors) such as aluminum oxide and silicon oxide, etc., aluminum foils, and aluminum-deposited plastic films. As one example thereof, there is enumerated a heat generating pad in which the produced heat generating pad is sealed and fixed between two air-impermeable films or sheets.

The heat generating composition is not limited so far as it is a heat generating composition which has a water content of from 1 to 60 % by weight, contains, as essential components, a flocculant aid, a dry binding agent, a flocculant, an adhesive binder, an iron powder, a carbon component, a reaction accelerator and water, does not contain a thickener and an excipient, contains surplus water so as to have a water mobility value of from 0.01 to 20, has moldability due to the surplus water, with the water in the heat generating composition not functioning as a barrier layer, and is capable of causing an exothermic reaction upon contact with air.

Incidentally, in the invention, what water does not function as a barrier layer and causes an exothermic reaction upon contact with air means that water in a heat generating composition does not function as a barrier layer which is an air intercepting layer and immediately after the production of a heat generating composition, comes into contact with air, thereby immediately causing an exothermic reaction.

In addition, if desired, at least one member selected from additional components consisting of a water retaining agent, a water absorptive polymer, a pH adjusting agent, a hydrogen formation inhibitor, an aggregate, a fibrous material, a functional substance, a surfactant, an organosilicon compound, a pyroelectric substance, a moisturizer, a fertilizer component, a hydrophobic polymer compound, a heat generating aid, a metal other than iron, a metal oxide other than iron oxide, an acidic substance, and a mixture thereof may be further added to the heat generating composition.

Furthermore, in the heat generating composition of the invention or the like, although there is no particular limitation for the compounding ratio thereof, it is preferred to select the compounding ratio such that the amount of the reaction accelerator is from 1.0 to 50 parts by weight, the amount of water is from 1.0 to 60 parts by weight, the amount of the carbon component is from 1.0 to 50 parts by weight, the amount of the water retaining agent is from 0.01 to 10 parts by weight, the water absorptive polymer is from 0.01 to 20 parts by weight, the amount of the pH adjusting agent is from 0.01 to 5 parts by weight, and the amount of the hydrogen formation inhibitor is from 0.01 to 12 parts by weight, respectively based on 100 parts by weight of the iron powder; and that the heat generating composition has a water mobility value of from 0.01 to 20.

In addition, the following components may be added in compounding ratios as described below to the iron powder to the heat generating composition. That is, the amount of the metal other than iron is from 1.0 to 50 parts by weight, the amount of the metal oxide other than iron oxide is from 1.0 to 50 parts by weight, the amount of the surfactant is from 0.01 to 5 parts by weight, the amount of each of the hydrophobic polymer compound, the aggregate, the fibrous material, the functional substance, the organosilicon compound and the pyroelectric substance is from 0.01 to 10 parts by weight, the amount of each of the moisturizer, the fertilizer component and the heat generating aid is from 0.01 to 10 parts by weight, and the amount of the acidic substance is from 0.01 to 1 part by weight based on 100 parts by weight of the iron powder. Incidentally, a magnetic material may further be compounded, and its compounding ratio may be properly determined depending upon the desire.

Incidentally, these compounding ratios can also be applied in a reaction mixture and a heat generating mixture. Furthermore, a water mobility value of the reaction mixture is usually less than 0.01.

As the water, one from a proper source may be employed.

Its purity and kind and the like are not particularly limited.

In the case of the heat generating composition, the content of water is preferably from 1 to 70 % by weight, more preferably from 1 to 60 % by weight, further preferably from 7 to 60 % by weight, still further preferably from 10 to 50 % by weight, and even further preferably from 20 to 50 % by weight of the heat generating composition.

Furthermore, in the case of the reaction mixture or heat generating mixture prior to the contact treatment with an oxidizing gas, the content of water is preferably from 0.5 to 20 % by weight, more preferably from 1 to 20 % by weight, further preferably from 3 to 20 % by weight, and still further preferably from 4 to 15 % by weight of the reaction mixture or heat generating mixture.

The carbon component is not particularly limited so far as it contains carbon as a component. Examples thereof include carbon black, graphite, active carbon, carbon nanotubes, carbon nanohorns, and flullerenes. Carbon which has become conductive by doping or the like is also employable. There are enumerated active carbons as prepared from coconut shell, wood, charcoal, coal, bone carbon, etc. and carbons as prepared from other raw materials such as animal products, natural gases, fats, oils, and resins. In particular, active carbons having an adsorption retaining ability are preferable.

Furthermore, it is not always required that the carbon component is present alone. In the case where an iron powder containing the carbon component and/or covered by the carbon component is used in the heat generating composition, it is to be noted that the heat generating composition contains the carbon component even though the carbon component is not present alone.

The reaction accelerator is not particularly limited so far as it is able to promote the reaction of the heat generating substance. Examples thereof include metal halides, nitrates, acetates, carbonates, and metal sulfates. Examples of metal halides include sodium chloride, potassium chloride, magnetic chloride, calcium chloride, ferrous chloride, ferric chloride, sodium bromide, potassium bromide, ferrous bromide, ferric bromide, sodium iodide, and potassium iodide. Examples of nitrates include sodium nitrate and potassium nitrate. Examples of acetates include sodium acetate. Examples of carbonates include ferrous carbonate. Examples of metal sulfates include potassium sulfate, sodium sulfate, and ferrous sulfate.

The water retaining agent is not limited so far as it is able to retain water. Examples thereof include porous materials derived from plants having high capillary function and hydrophilicity such as wood meal, pulp powder, active carbon, saw dust, cotton cloth having a number of cotton fluffs, short fiber of cotton, paper dust, and vegetable materials, water-containing magnesium silicate based clay minerals such as active clay and zeolite, pearlite, vermiculite, silica based porous substances, coralline stone, and volcanic ash based substances (for example, terraballoon, shirasu balloon, and taisetsu balloon). In order to increase a water retaining ability and enhance a shape holding ability of such a water retaining agent, the water retaining agent may be subjected to a processing treatment such as baking and/or pulverization.

The water absorptive polymer is not particularly limited so far as it is a resin having a crosslinking structure and having a water absorption magnification of ion-exchanged water of 3 times or more of the dead weight. Furthermore, a water absorptive polymer the surface of which is crosslinked may be employed. Conventionally known water absorptive polymers and commercial products may also be employed.

Examples of the water absorptive polymer include poly(meth)acrylic acid crosslinked materials, poly(meth)-acrylic acid salt crosslinked materials, sulfonic group-containing poly(meth)acrylic ester crosslinked materials, polyoxyalkylene group-containing poly(meth)acrylic ester crosslinked materials, poly(meth)acrylamide crosslinked materials, crosslinked materials of a copolymer of a (meth)acrylic acid salt and a (meth)acrylamide, crosslinked materials of a copolymer of a hydroxyalkyl (meth)acrylate and a (meth)acrylic acid salt, polydioxolane crosslinked materials, crosslinked polyethylene oxide, crosslinked polyvinylpyrrolidone, sulfonated polystyrene crosslinked materials, crosslinked polyvinylpyridine, saponification products of a starch-poly(meth)acrylonitrile graft copolymer, starch-poly(meth)acrylic acid (salt) graft crosslinked copolymers, reaction products of polyvinyl alcohol and maleic anhydride (salt), crosslinked polyvinyl alcohol sulfonic acid salts, polyvinyl alcohol-acrylic acid graft copolymers, and polyisobutylene maleic acid (salt) crosslinked polymers. These water absorptive polymers may be used alone or in combination with two or more kinds thereof.

Of these water absorptive polymers, water absorptive polymers having biodegradation properties are not limited so far as they are a biodegradable water absorptive polymer. Examples thereof include polyethylene oxide crosslinked materials, polyvinyl alcohol crosslinked materials, carboxymethyl cellulose crosslinked materials, alginic acid crosslinked materials, starch crosslinked materials, polyamino acid crosslinked materials, and polylactic acid crosslinked materials.

The pH adjusting agent is not limited so far it is able to adjust the pH. Examples thereof include alkali metal weak acid salts and hydroxides and alkaline earth metal weak acid salts and hydroxides such as $Na_2CO_3$, $NaHCO_3$, $Na_3PO_4$,

$Na_2HPO_4$, $Na_5P_3O_{10}$, NaOH, KOH, $Ca(OH)_2$, $Mg(OH)_2$, and $Ca_3(PO_4)_2$.

The hydrogen formation inhibitor is not limited so far as it is able to inhibit the formation of hydrogen. Examples thereof include one member or two or more members selected from the group consisting of sulfur compounds, oxidizing agents, alkaline substances, sulfur, antimony, selenium, phosphorus, and tellurium. Incidentally, examples of sulfur compounds include compounds with an alkali metal or an alkaline earth metal, metal sulfides such as calcium sulfide, metal sulfites such as sodium sulfite, and metal thiosulfates such as sodium thiosulfate.

The aggregate is not limited so far as it is useful as a filler and/or is useful for making the heat generating composition porous. Examples thereof include fossilized coral (for example, coral fossil and weathered coral fossil), bamboo charcoal, *bincho* charcoal, silica-alumina powders, silica-magnesia powders, kaolin, crystalline cellulose, colloidal silica, pumice, silica gel, silica powders, mica powders, clays, talc, synthetic resin powders or pellets, foamed synthetic resins such as foamed polyesters or polyurethanes, diatomaceous earth, alumina, and cellulose powder. Incidentally, it is to be noted that kaolin and crystalline cellulose are not contained in the heat generating composition of the invention.

The fibrous material is an inorganic fibrous material and/or an organic fibrous material. Examples thereof include rock wool, glass fibers, carbon fibers, metal fibers, pulps, papers, non-woven fabrics, woven fabrics, natural fibers such as cotton and hemp, regenerated fibers such as rayon, semi-synthetic fibers such as acetates, synthetic fibers, and pulverized products thereof.

The functional substance is not limited so far as it is a substance having any function. Examples thereof include at least one member selected from minus ion emitting substances and far infrared ray radiating substances. The minus ion emitting substance is not limited so far as it emits a minus ion as a result either directly or indirectly, and examples thereof include ferroelectric substances such as tourmaline, fossilized coral, granite, and calcium strontium propionate, and ores containing a radioactive substance such as radium and radon. The far infrared ray radiating substance is not limited so far as it radiates far infrared rays. Examples thereof include ceramics, alumina, zeolite, zirconium, and silica.

The surfactant includes anionic surfactants, cationic surfactants, nonionic surfactants, and ampholytic surfactants. Especially, nonionic surfactants are preferable, and examples thereof include polyoxyethylene alkyl ethers, alkylphenol·ethylene oxide adducts, and higher alcohol phosphoric acid esters.

The organosilicon compound is not limited so far as it is a compound having at least an Si-O-R bond and/or an Si-N-R bond and/or an Si-R bond. The organosilicon compound is in the form of a monomer, a lowly condensed product, a polymer, etc. Examples thereof include organosilane compounds such as methyltriethoxysilane; and dimethylsilicone oil, polyorganosiloxane, or silicone resin compositions containing the same.

The pyroelectric substance is not limited so far as it has pyroelectricity. Examples thereof include tourmaline, hemimorphic ores, and pyroelectric ores. Tourmaline or achroite which is a kind of tourmaline is especially preferable. Examples of the tourmaline include dravite, schorl, and elbaite.

The moisturizer is not limited so far as it is able to hold moisture. Examples thereof include hyaluronic acid, collagen, glycerin, and urea.

The fertilizer component is not limited so far as it is a component containing at least one of three elements of nitrogen, phosphorus and potassium. Examples thereof include a bone powder, urea, ammonium sulfate, calcium perphosphate, potassium chloride, and calcium sulfate.

The hydrophobic polymer compound is not limited so far as it is a polymer compound having a contact angle with water of 40° or more, preferably 50° or more, and more preferably 60° or more in order to improve the draining in the composition. The shape of the hydrophobic polymer compound is not limited, and examples thereof include powdery, particulate, granular, and tablet shapes. Examples of the hydrophobic polymer compound include polyolefins such as polyethylene and polypropylene, polyesters, and polyamides.

Examples of the heat generating aid include metal powders, metal salts, and metal oxides such as Cu, Mn, $CuCl_2$, $FeCl_2$, manganese dioxide, cupric oxide, triiron tetroxide, and mixtures thereof.

As the metal oxide other than iron oxide, any material can be employed so far as it does not hinder the oxidation of iron by an oxidizing gas, and examples thereof include manganese dioxide and cupric oxide.

The acidic substance may be any of an inorganic acid, an organic acid, or an acidic salt. Examples thereof include hydrochloric acid, sulfuric acid, nitric acid, acetic acid, oxalic acid, citric acid, malic acid, maleic acid, chloroacetic acid, iron chloride, iron sulfate, iron oxalate, iron citrate, aluminum chloride, ammonium chloride, and hypochlorous acid.

As the "iron powder" as referred to herein, usual iron powders, iron alloy powders and active iron powders such as iron powders comprising particles, a surface of each of which is at least partially covered with an oxygen-containing film, and iron alloy powders comprising particles, a surface of each of which is at least partially covered with an oxygen-containing film, are preferable. Incidentally, the "iron oxide film" as referred to herein is a film made of oxygen-containing iron such as iron oxide, hydroxide or oxyhydroxide. Furthermore, the "active iron powder" as referred to herein is a powder in which an iron oxide film is formed at least locally on the surface of an iron powder, from which an oxidation reaction promoting effect is obtained by a local cell as formed between an iron matrix and an iron oxide film or a pit inside and outside the iron oxide film.

The iron powder is not limited, and examples thereof include cast iron powders, atomized iron powders, electrolyzed iron powders, reduced iron powders, sponge iron powders, and iron alloy powders thereof. In addition, the iron powder may contain carbon or oxygen, and an iron powder containing 50 % or more of iron and other metals may be employed. The kind of the metal which is contained as an alloy, etc. is not particularly limited so far as the iron component works as a component of the heat generating composition. Examples of such a metal include metals such as aluminum, manganese, copper, nickel, silicon, cobalt, palladium, and molybdenum, and semiconductors. The metal of the invention includes a semiconductor. Such a metal or alloy may be contained only in the surface or the interior, or may be contained in both the surface and the interior.

In the iron powder of the invention, the content of the metal other than iron is usually from 0.01 to 50 % by weight, and preferably from 0.1 to 10 % by weight based on the whole of the iron powder.

Examples of the iron powder having an oxygen-containing film on at least a part of the surface of the iron include:

(A) an active iron powder in which the surface of an iron component is at least partially oxidized, which is obtained by contact treating the essential components of the heat generating composition or the essential components to which acidic substances or other necessary components are added with an oxidizing gas, thereby partially oxidizing the iron component;
(B) an active iron powder in which the content of wustite is from 2 to 50 % by weight in terms of an X-ray peak intensity ratio to iron;
(C) an iron powder having an iron oxide film having a thickness of 3 nm or more on the surface thereof; and
(D) a mixture of an active iron powder and an iron powder other than an active iron powder.

With respect to (A), although the mechanism is not elucidated in detail, it is assumed that upon contact between the oxidizing gas and the components, not only an iron oxide film, namely, an oxygen-containing film is formed on the surface of the iron powder due to the oxidation of the components, especially the oxidation of the iron powder, but also the surface of active carbon is oxidized and/or the oxidized iron component is adhered, whereby hydrophilicity is imparted or improved, and coupling between the components or structurization takes place through the mediation of water.

That is, it is assumed that some kind of a change in the function occurs such that an iron oxide film is formed on the surface of the iron powder, the shape of the iron powder particle becomes irregular, a strain is generated due to the oxidation, or a water-containing pit is formed, whereby the iron powder is activated and exothermic rising properties are improved.

Furthermore, the case where magnetite ($Fe_3O_4$) is present in the iron oxide film is preferable because the conductivity is excellent, and the case where hematite ($Fe_2O_3$) is present in the iron oxide film is also preferable because the iron oxide film becomes porous. Moreover, it is assumed that the carbon component is oxidized on the surface thereof and becomes a carbon component which is rich in oxides on the surface thereof, whereby the hydrophilicity increases and the activity increases.

The thickness of the iron oxide film which is an oxygen-containing film covering the surface of the iron powder, as measured by the Auger electron spectroscopy, is usually 3 nm or more, preferably from 3 nm to 100 $\mu$m, more preferably from 30 nm to 100 $\mu$m, further preferably from 30 nm to 50 $\mu$m, still further preferably from 30 nm to 1 $\mu$m, even further preferably from 30 nm to 500 nm, and even still further preferably from 50 nm to 300 nm.

When the thickness of the oxygen-containing film of iron is 3 nm or more, the thickness of the oxygen-containing film of iron is able to exhibit a promoting effect of the oxidation reaction, and upon contact with an oxidizing gas such as air, is able to immediately initiate the oxidation reaction. When the thickness of the oxygen-containing film of iron is 100 $\mu$m or more, though the heat generation time may possibly be shortened, such is applicable depending upon the utility.

Furthermore, according to the active iron powder, by using a reaction mixture containing, as essential components, an iron powder, a reaction accelerator and water and having a water content of from 0.5 to 20 % by weight and a water mobility value showing a surplus water content of less than 0.01, the reaction rate at the time of the contact treatment with an oxidizing gas can be raised, thereby achieving a time required for regulating a temperature rise of the reaction mixture at 1 °C or more within 10 minutes. By shortening a time required for arrival at a prescribed temperature or higher, proper activation can be achieved, and unnecessary oxidation on the iron powder can be prevented.

Furthermore, the heat generating composition prepared by adding a carbon component, etc. to a heat generating mixture as produced by contact treating the reaction mixture with an oxidizing gas or adjusting the water content so as to have a water mobility value of from 0.01 to 50 is properly tacky, has excellent moldability and is able to be applied with a molding method such as a force-through die molding method and a cast molding method, whereby heat generating bodies of various shapes can be produced. In particular, a heat generating composition having a water mobility value of from 0.01 to 20 is excellent because it initiates an exothermic reaction immediately after contacting with air, has excellent exothermic rising properties and has excellent moldability.

The contact treatment method of the reaction mixture with an oxidizing gas is not particularly limited so far as it is able to contact treat a reaction mixture containing, as essential components, an iron powder, a reaction accelerator and water and having a water content of from 0.5 to 20 % by weight and a water mobility value of less than 0.01 with an oxidizing gas and regulate a temperature rise of the reaction mixture at 1 °C or more.
Specific examples thereof include:

(1) a process for producing a heat generating mixture containing an iron powder having an iron oxide film on the surface thereof by subjecting a reaction mixture of an iron powder, a reaction accelerator and water in an oxidizing gas atmosphere to a self-exothermic reaction, thereby partially oxidizing the iron powder;
(2) a process for producing a heat generating mixture by subjecting a reaction mixture of an iron powder, a reaction accelerator, an acidic substance and water in an oxidizing gas atmosphere to a self-exothermic reaction;
(3) a process for producing a heat generating mixture by subjecting a reaction mixture of an iron powder, a reaction accelerator, a carbon component and water in an oxidizing gas atmosphere to a self-exothermic reaction;
(4) a process for producing a heat generating mixture by subjecting a reaction mixture of an iron powder, a reaction accelerator, an acidic substance, a carbon component and water in an oxidizing gas atmosphere to a self-exothermic reaction;
(5) a process for producing a heat generating mixture containing a partially oxidized iron powder by carrying out the method as set forth above in any one of (1) to (4), wherein the reaction mixture or heat generating mixture as set forth above in any one of (1) to (4) contains a component other than the foregoing components;
(6) a process for producing a heat generating mixture by carrying out the method as set forth above in any one of (1) to (5) under circumstances heated so as to have temperature of at least 10 °C higher than the circumferential temperature;
(7) a process for producing a heat generating mixture by carrying out the method as set forth above in any one of (1) to (6) by blowing an oxidizing gas;
(8) a process for producing a heat generating mixture by carrying out the method as set forth above in (7) by blowing the oxidizing gas heated so as to have a temperature of at least 10 °C higher than the circumferential temperature;
(9) a process for producing a heat generating composition by carrying out the method as set forth above in any one of (1) to (8) by contact treating with an oxidizing gas until the temperature exceeds a maximum temperature which is a maximum point of temperature rise by the exothermic reaction;
(10) a process for producing a heat generating mixture by carrying out the method as set forth above in any one of (1) to (8) by contact treating with an oxidizing gas until the temperature exceeds a maximum temperature by the exothermic reaction and drops by at least 10 to 20 °C from the maximum temperature;
(11) a process for producing a heat generating composition by carrying out the method as set forth above in any one of (1) to (8) by contact treating with an oxidizing gas until the temperature exceeds a maximum temperature which is a maximum point of temperature rise by the exothermic reaction and after intercepting the oxidizing gas, holding it until the temperature of at least the reaction mixture drops by at least 10 to 20 °C from the maximum temperature; and
(12) a process for producing a heat generating mixture by heating the reaction mixture or heat generating mixture as set forth above in any one of (1) to (5) under oxidizing gas circumstances while regulating a temperature rise at 1 °C or more.

In addition, a heat generating mixture as prepared by adding other components to the heat generating mixture and further treating with an oxidizing gas may be employed.
Incidentally, the circumstances of the reaction mixture at the time of contact treatment with an oxidizing gas are not limited so far as the reaction mixture is brought into contact with an oxidizing gas under circumstances of 0 °C or higher and a temperature rise of the reaction mixture is regulated at 1 °C or more within 10 minutes. In the case where the contact treatment is carried out in an open system, the circumstances may be either the state that the reaction mixture is present in a lid-free vessel or the state that an oxidizing gas such as air comes into a vessel through an air-permeable sheet-like material such as non-woven fabrics.
Furthermore, the contact treatment with an oxidizing gas may be carried out with or without stirring in a fluidized or non-fluidized state and may be carried out in a batch or continuous system.
Examples of the final heat generating composition include:

1) a heat generating composition containing, as a heat generating composition raw material, a heat generating mixture produced in the process as set forth above in any one of (1) to (12);
2) a heat generating composition obtained by adding other components to the heat generating composition as set forth above in 1); and
3) a heat generating composition obtained by adjusting the water content of the heat generating composition as set forth above in 1) or 2).

The order of the timing of adding other components than the essential components and the timing of adjusting the water content is not limited.

Here, the water content in the reaction mixture and also the heat generating mixture prior to the treatment with an oxidizing gas is usually from 0.5 to 20 % by weight, preferably from 1 to 15 % by weight, more preferably from 2 to 10 % by weight, further preferably from 3 to 10 % by weight, and still further preferably from 6 to 10 % by weight.

The temperature of the reaction mixture after the contact with an oxidizing gas is not limited so far as the temperature rise is regulated at 1 °C or more. The temperature of the reaction mixture after the contact with an oxidizing gas is preferably from 1 to 80 °C, more preferably from 1 to 70 °C, further preferably from 1 to 60 °C, and still further preferably from 1 to 40 °C.

The circumferential temperature at the time of contact between the reaction mixture and the oxidizing gas is not limited so far as the temperature of the reaction mixture is raised to a prescribed temperature or higher. The circumferential temperature at the time of contact between the reaction mixture and the oxidizing gas is preferably 0 °C or higher, more preferably from 0 to 250 °C, further preferably from 10 to 200 °C, still further preferably from 20 to 150 °C, even further preferably from 25 to 100 °C, and even still further preferably from 25 to 50 °C.

The time of contact between the reaction mixture and the oxidizing gas is not limited so far as the time required for regulating a temperature rise at 1 °C or more is within 10 minutes. The time of contact between the reaction mixture and the oxidizing gas is preferably from one second to 10 minutes, more preferably from one second to 7 minutes, further preferably from one second to 5 minutes, still further preferably from 2 seconds to 5 minutes, even further preferably from 2 seconds to 3 minutes, and even still further preferably from 2 seconds to one minute.

The temperature of the oxidizing gas is not limited so far as the foregoing circumferential temperature is kept.

As the "oxidizing gas" as referred to herein, any gas can be used as the oxidizing gas so far as it is oxidizing. Examples thereof include an oxygen gas, air, and mixed gases of an inert gas (for example, a nitrogen gas, an argon gas, and a helium gas) and an oxygen gas. Although the mixed gas is not limited so far as it contains oxygen, mixed gases containing 10 % or more of an oxygen gas are preferable, and of these, air is especially preferable. If desired, a catalyst such as platinum, palladium, iridium, and compounds thereof can also be used.

The oxidation reaction can be carried out under stirring in an oxidizing gas atmosphere optionally under a pressure and/or upon irradiation of ultrasonic waves.

The optimal condition of the oxidation reaction may be properly experimentally determined.

An amount of the oxidizing gas to be used is not limited but may be adjusted depending upon the kind of the oxidizing gas, the kind and particle size of the iron powder, the water content, the treatment temperature, the treatment method, and the like.

In the case of an open system, there is no limitation so far as a necessary amount of oxygen can be taken in. In order to prevent fly of the reaction mixture or contamination of dusts, etc., the system may be surrounded by an air-permeable raw material such as non-woven fabrics and woven fabrics. So far as the system is in an air-permeable state, it is to be noted that the system is an open system.

In the case where air is used in the system of blowing an oxidizing gas, for example, the amount of air is preferably from 0.01 to 1,000 L/min, more preferably from 0.01 to 100 L/min, and further preferably from 0.1 to 50 L/min per 200 g of the iron powder under one atmosphere. In the case of other oxidizing gas, the amount of the oxidizing gas may be converted on the basis of the case of air.

If desired, a peroxide may be added. Examples of the peroxide include hydrogen peroxide and ozone.

Here, so far as the iron powder is partially oxidized, the state of the reaction mixture or heat generating mixture at the time of the contact treatment with an oxidizing gas may be any of a standing state, a transfer state, or a fluidizing state by stirring, etc. and may be properly selected. Furthermore, the circumstances at the time of mixing the respective components of the reaction mixture, the heat generating mixture or the heat generating composition and at the time of the contact treatment with a mixed oxidizing gas at the time of adjusting the water content are not limited, and examples thereof include those in an oxidizing gas atmosphere and those in blowing of an oxidizing gas.

A method for measuring a temperature rise of the heat generating composition is as follows.

1) A heat generating composition is allowed to stand in a state that it is sealed in an air-impermeable outer bag for one hour under a condition that the circumferential temperature is 20 ± 1 °C.

2) A magnet is provided in the vicinity of a central part of the back side of a polyvinyl chloride-made supporting plate (3 mm in thickness × 600 mm in length × 600 mm in width) of a footed supporting table so as to cover a cavity shape of a molding die.

3) A temperature sensor is placed on the central part of the supporting plate.

4) A polyethylene film (25 μm in thickness × 250 mm in length × 200 mm in width) as provided with an adhesive layer having a thickness of about 80 μm is stuck onto the supporting plate via a sticky layer such that the center of the polyethylene film is positioned at the sensor.

5) The heat generating composition is taken out from the outer bag.

6) A template (250 mm in length × 200 mm in width) having a cavity (80 mm in length × 50 mm in width × 3 mm in height) is placed above the central part of the polyethylene film; a sample is placed in the vicinity of the cavity; a force-in die plate is moved along the template; the sample is charged into the cavity while stuffing; and the sample is leveled while stuffing along the template plane (force-in die molding), thereby filling the sample in the die. Next, the magnet beneath the supporting plate is removed, and the temperature measurement is started.

With respect to the measurement of the exothermic temperature, the temperature is measured for 10 minutes at a measurement timing of 2 seconds using a data collector, and exothermic rising properties are judged in terms of the temperature after elapsing 3 minutes.

The heat generation test of the heat generating body follows the JIS temperature characteristic test.

In the iron powder or active iron powder in the oxidizing gas-treated heat generating composition, at least a part of the surface thereof is covered by an oxygen-containing film of iron. The degree of covering on the surface of the oxygen-containing film of iron is not limited so far as at least a part of the surface thereof is covered, and the surface may be entirely covered. In the case of the heat generating composition of the invention, since an ion of the reaction accelerator such as a chlorine ion is contained in the heat generating composition, there is no corrosion effect of the oxide film due to anti-corrosion effect by the ion of the reaction accelerator such as a chlorine ion. Thus, the oxidation reaction which is a sort of corrosion is not hindered. In particular, in the case where an oxygen-containing film of iron is prepared while the ion of the reaction accelerator such as a chlorine ion exists together, the subject effect is large. In the case where a metal other than iron is present on the surface, it is only required that at least other part of the metal portion other than iron is covered by the oxygen-containing film of iron.

In the iron powder of the invention, not only a region where (1) entire (uniform) corrosion, (2) pitting or crevice corrosion, (3) stress corrosion cracking, or the like is generated, but also irregularities or crevices are formed. For that reason, it is assumed that the iron powder of the invention has hydrophilicity and oxidation catalytic properties (FeO, etc.) in its own portion. In producing the heat generating composition, it is important that the iron powder has an oxygen-containing film in its own portion without relying upon mixing. In particular, in the iron component as prepared by contact treating the iron component and the reaction accelerator and water as essential components with an oxidizing gas, it is thought that a reaction active part composed mainly of an oxide, a hydroxide, a chlorine ion, a hydrogen ion, etc. is formed, whereby exothermic reactivity and hydrophilicity are improved and exothermic rising properties and moldability are remarkably improved.

With respect to (B), the amount of FeO (wustite) which is contained in the iron component containing a prescribed amount of wustite is usually from 2 to 50 % by weight, preferably from 2 to 40 % by weight, more preferably from 2 to 30 % by weight, further preferably from 5 to 30 % by weight, and still further preferably from 6 to 30 % by weight in terms of an X-ray peak intensity ratio of iron. When the amount of FeO (wustite) exceeds 50 % by weight, though the exothermic rising properties are good, the duration of heat generation becomes short. On the other hand, when it is less than 2 % by weight, the exothermic rising properties become dull.

The thickness of the oxygen-containing film of a prescribed amount or the oxygen-containing film of iron powder containing wustite and the amount of wustite are applied to the heat generating composition or the heat generating composition molded body at the time of lamination.

An iron powder containing a carbon component and/or covered by a carbon component is also preferable. Although a proportion of the carbon component is not limited so far as a ratio of the iron component to the carbon component is 50 % by weight or more, an iron powder in which the surface thereof is partially covered by from 0.3 to 3.0 % by weight of a conductive carbonaceous substance is useful. Examples of the conductive carbonaceous substance include carbon black, active carbon, carbon nanotubes, carbon nanohorns, and flullerenes. Ones which have become conductive by doping are also employable. Examples of the iron powder include reduced iron powders, atomized iron powders, and sponge iron powders. In particular, the case where the conductive carbonaceous substance is active carbon and the iron powder is a reduced iron powder is useful as a heat generating body.

Furthermore, in order to efficiently carry out covering by a conductive carbonaceous substance, an oil such as a spindle oil may be added in an amount of from 0.01 to 0.05 % by weight to such an extent that the fluidity of the iron powder is not hindered.

In the case of measuring the water mobility value of the heat generating composition in the heat generating body and the thickness and amount of wustite of the iron oxide film of iron powder in the mixture or the heat generating composition in the heat generating body, the heat generating composition or mixture may be measured according to the following items.

1) Water mobility value:

The heat generating composition is taken out from the heat generating body and measured according to the foregoing method of measuring a water mobility value.

2) Thickness and amount of wustite of iron oxide film of iron powder:

A measuring sample as prepared by dispersing the heat generating composition, the heat generating composition molded body, the heat generating composition compression molded body or the mixture in nitrogen-purged ion-exchanged water in a nitrogen atmosphere, separating the iron powder using a magnet and drying the iron powder in a nitrogen atmosphere is used.

The heat generating composition of the invention contains, as essential components, an iron powder, a carbon component, a reaction accelerator and water, and its production process is one which can be put into practical use on an industrial scale. A reaction mixture containing, as essential components, an iron powder, a reaction accelerator and water and having a water content of from 1 to 20 % by weight and a water mobility value showing a surplus water content of less than 0.01 is brought into contact with an oxidizing gas under circumstances at 0 °C or higher, a temperature rise of the reaction mixture is regulated at 1 °C or more within 10 minutes to produce a heat generating mixture, and the subject heat generating mixture is used as a raw material to form a heat generating composition. Alternatively, a heat generating composition may be formed by subsequently further adjusting the water content, or by further adding a carbon component, etc. and adjusting the water content.

In the invention, it has become possible to realize the contact treatment with an oxidizing gas within a short period of time by regulating the water content of the reaction mixture at a fixed amount or less, especially regulating the surplus water content of the reaction mixture at a fixed amount or less and carrying out an oxidizing contact treatment. By specifying the surplus water content and performing the treatment within a short period of time, adverse influences such as poor initial exothermic rising of the heat generating composition and shortening of the heat generation-retaining time can be avoided. Thus, it has become possible to establish an industrial mass-production process. Furthermore, although stirring or the like may not be achieved during the contact treatment with an oxidizing gas, when stirring or the like is achieved, the contact treatment with an oxidizing gas can be surely carried out.

Here, so far as the iron powder is partially oxidized, the state of the reaction mixture or heat generating mixture at the time of the contact treatment with an oxidizing gas may be any of a standing state, a transfer state, or a fluidizing state by stirring, etc. and may be properly selected. Furthermore, the circumstances at the time of mixing the respective components of the reaction mixture, the heat generating mixture or the heat generating composition and at the time of mixing at the time of adjusting the water content are not limited, and examples thereof include those in an oxidizing gas atmosphere and those in blowing of an oxidizing gas.

The "adjustment of the water content" as referred to herein means that after contact treating the heat generating mixture with an oxidizing gas, water or an aqueous solution of a reaction accelerator is added. Although the amount of addition of water or an aqueous solution of a reaction accelerator is not limited, examples thereof include the addition of a weight corresponding to a reduced weight by the contact treatment and the addition of a weight such that a desired water mobility value is obtained.

Whether or nor the adjustment of the water content is introduced may be properly determined depending upon the utility.

The heat generating composition of the invention contains, as essential components, an iron powder, a carbon component, a reaction accelerator and water and is started from a mixture obtained by contact treating a reaction mixture containing, as essential components, an iron powder, a reaction accelerator and water with an oxidizing gas. The heat generating composition of the invention is usually one obtained by adjusting the water content of a heat generating mixture and is a heat generating composition which is satisfactory in the exothermic rising, has a suitable amount of surplus water and has excellent moldability. Furthermore, it is possible to produce a heat generating body which can become promptly warm at the time of use.

Accordingly, at least the iron powder further including the carbon component has a history of oxidation by the contact treatment with an oxidizing gas, and it is thought that this is deeply related to excellent exothermic rising properties, exothermic endurance and excellent moldability.

When the iron powder which is contact treated with an oxidizing gas according to the invention is used, the amount of addition of the carbon component (for example, active carbon) in the heat generating composition can be reduced by, for example, 20 % or more. By reducing the amount of addition of the carbon component, the costs are lowered.

According to the production process of the heat generating mixture of the invention, it is possible to obtain a heat generating composition having excellent exothermic rising properties, excellent hydrophilicity, and excellent moldability. In particular, a heat generating composition having remarkably excellent moldability and exothermic characteristics together can be obtained while specifying the water availability value at from 0.01 to 50, in particular 0.01 to 20.

The heat generating composition as produced by the production process of the invention is remarkably improved with respect to exothermic rising properties. Thus, the amount of addition of the carbon component (such as active carbon) in the heat generating composition can be reduced by, for example, 20 % or more so that it can contribute to a reduction in costs.

Furthermore, since the hydrophilicity is remarkably improved, the moldability with a mold is remarkably improved. Thus, since after molding, collapsed pieces of the heat generating composition are not scattered on the surroundings of the heat generating composition molded body, sealing can be appropriately achieved so that a heat generating body free from sealing cut can be produced. In this way, heat generating composition molded bodies of various shapes can be produced, and heat generating bodies of various shapes are formed.

Furthermore, in view of improving the exothermic rising properties of the heat generating composition, the following are preferable.

1) A heat generating composition obtained by a contact treatment (self heat generation) of a mixture of the essential components of the heat generating composition, or a mixture of the foregoing mixture and an acidic substance or other necessary components with an oxidizing gas, a heat generating composition obtained by additionally adjusting the water content of the foregoing heat generating composition, or a heat generating composition obtained by adding and mixing other components in the foregoing heat generating composition.

2) Any one of the following active iron powders having an oxygen-containing film (for example, oxides) on at least a part of the surface thereof is used as the iron powder: (a) an iron powder having an oxygen-containing film of iron having a thickness, as measured by the Auger electron spectroscopy, of 3 nm or more on the surface thereof and (b) an iron powder having a content of wustite of from 2 to 50 % by weight in terms of an X-ray peak intensity ratio to iron.

3) A mixture of an active iron powder having an oxygen-containing film (for example, oxides) on at least a part of the surface thereof and an iron powder not having an oxygen-containing film is used as the iron powder. In this case, a mixture containing 60 % by weight or more of an active iron powder and less than 40 % by weight of an iron powder other than the active iron is preferable.

In the case of storing the heat generating composition which is treated with an oxidizing gas or the heat generating composition containing an active iron powder, or a material utilizing the same over a long period of time, it is preferred to combine a hydrogen formation inhibitor therewith. This is because in this way, a heat generating body having excellent exothermic characteristics, which is inhibited in the formation of hydrogen, is free from swelling of the outer bag at the time of storage, etc. and has satisfactory exothermic rising properties, is obtained.

Furthermore, the heat generating composition having a water mobility value falling outside the foregoing range of from 0.01 to 20 can contain a water-soluble polymer, an agglomeration aid, a dry binder, a sticky raw material, a thickener, an excipient, a flocculant, and a soluble sticky raw material so far as the rising characteristics are not affected.

Furthermore, since a marketed heat generating body in which a heat generating composition is accommodated in an accommodating bag is provided on the assumption that it is accommodated in an outer bag which is an air-impermeable accommodating bag and is storable over a long period of time, it is preferred to use a heat generating composition containing a hydrogen formation inhibitor. Since the heat generating composition which has passed through the contact treatment with an oxidizing gas is an active composition, it is important that the heat generating composition contains a hydrogen formation inhibitor. Also, this efficacy is further strengthened by using a pH adjusting agent together.

Furthermore, so far as the reaction characteristics and exothermic characteristics are not affected, the heat generating composition having a water mobility value of less than 0.01 may contain a flocculant aid, a flocculant, an agglomeration aid, a dry binder, a dry binding agent, a dry binding material, a sticky raw material, a thickener, an excipient, or a water-soluble polymer in an amount ranging from 0.01 to 3 parts by weight respectively.

The "flocculant aid" as referred to herein is a flocculant aid as described in Japanese Patent No. 3,161,605 (JP-T-11-508314) such as gelatin, natural gum, and corn syrup.

The "flocculant" as referred to herein is a flocculant as described in JP-T-2002-514104 such as corn syrup and maltitol syrup.

The "agglomeration aid" as referred to herein is an agglomeration aid as described in JP-T-2001-507593 such as corn syrup.

The "dry binder" as referred to herein is a dry binder as described in JP-T-2002-514104 such as microcrystalline cellulose, maltodextrin, and mixtures thereof.

The "dry binding agent" as referred to herein is a dry binding agent as described in JP-T-2001-507593 such as maltodextrin and sprayed lactose.

The "dry binding material" as referred to herein is a dry binding material as described in JP-T-11-508314 such as microcrystalline cellulose, maltodextrin, and mixtures thereof.

The "sticky raw material" or the "binder" as referred to herein is a sticky raw material or binder as described in JP-A-4-293989 such as water glass, polyvinyl alcohol (PVA), and carboxymethyl cellulose (CMC).

The "thickener" as referred to herein is a thickener as described in JP-A-6-343658 such as corn starch and potato starch.

The "excipient" as referred to herein is an excipient as described in JP-A-7-194641 such as $\alpha$-starch and sodium alginate.

As the "water-soluble polymer" as referred to herein, the water-soluble polymer in the adhesive layer can be used.

The particle size of the water-insoluble solid component constituting the moldable heat generating composition of the invention is not limited so far as the heat generating composition has moldability. In the case where any one of length, width and height as the size of the heat generating composition molded body as molded from the heat generating composition is small, the moldability is improved by making the particle size small.

In addition, it is preferable in view of molding that the particle size of the solid component constituting the moldable heat generating composition is small. A maximum particle size of the water-insoluble solid component exclusive of the reaction accelerator and water in the components constituting the moldable heat generating composition is preferably not more than 2.5 mm, more preferably not more than 930 $\mu$m, further preferably not more than 500 $\mu$m, still further preferably not more than 300 $\mu$m, even further preferably not more than 250 $\mu$m, and even still further preferably not more than 200 $\mu$m. Moreover, 80 % or more of the particle size of the solid component is usually not more than 500 $\mu$m, preferably not more than 300 $\mu$m, more preferably not more than 250 $\mu$m, further preferably not more than 200 $\mu$m, still further preferably not more than 150 $\mu$m, and even further preferably not more than 100 $\mu$m.

Incidentally, with respect to the particle size of the water-insoluble solid component, separation is conducted using a sieve, and the particle size of the component which has passed through the sieve is calculated from an opening of the sieve. That is, sieves of 8, 12, 20, 32, 42, 60, 80, 100, 115, 150, 200, 250 and 280 meshes and a receiving dish are combined in this order from up to down. About 50 g of water-insoluble solid component particles are placed on the uppermost 8-mesh sieve and shaken for one minute using an automatic shaker. Weights of the water-insoluble solid component particles on each of the sieves and the receiving dish are weighed. The total amount thereof is defined as 100 %, and the particle size distribution is determined from weight fractions. When the sum of all receiving dishes under the sieve of a specific mesh size becomes 100 % which is the total sum of the particle size distribution, the size ($\mu$m) calculated from the opening of the specific mesh is defined as the particle size of the water-insoluble solid component. Incidentally, each of the mesh sieves may be combined with other mesh sieves. Here, the particles which have passed through a 16-mesh sieve are defined to have a particle size of not more than 1 mm; the particles which have passed through a 20-mesh sieve are defined to have a particle size of not more than 850 $\mu$m; the particles which have passed through a 48-mesh sieve are defined to have a particle size of not more than 300 $\mu$m; the particles which have passed through a 60-mesh sieve are defined to have a particle size of not more than 250 $\mu$m; the particles which have passed through a 65-mesh sieve are defined to have a particle size of not more than 200 $\mu$m; the particles which have passed through an 80-mesh sieve are defined to have a particle size of not more than 180 $\mu$m; the particles which have passed through a 100-mesh sieve are defined to have a particle size of not more than 150 $\mu$m; the particles which have passed through a 115-mesh sieve are defined to have a particle size of not more than 120 $\mu$m; the particles which have passed through a 150-mesh sieve are defined to have a particle size of not more than 100 $\mu$m; and the particles which have passed through a 250-mesh sieve are defined to have a particle size of not more 63 $\mu$m, respectively. The same is applicable to mesh sizes of less than these mesh sizes.

Furthermore, the heat generating composition can be classified into a powder, a granulate heat generating composition (having a water mobility value of less than 0.01), a moldable heat generating composition (having a water mobility value of from 0.01 to 20), and a sherbet-like heat generating composition (having a water mobility value exceeding 20 but not more than 50) depending upon the state of adjustment of the water content or surplus water. The heat generating composition as classified depending upon the water mobility value is as described previously.

The "moldability" as referred to in the invention exhibits that a laminate of the heat generating composition having a cavity or concave die shape can be formed by force-through molding using a trimming die having a cavity or cast molding using a concave die and after molding including mold release, the molding shape of the heat generating composition molded body is held. When the moldability is revealed, since the shape is held until the heat generating composition molded article is at least covered by a covering material and a seal part is formed between the substrate and the covering material, sealing can be achieved in the periphery of the shape with a desired shape. Also, since so-called "spots" which are a collapsed piece of the heat generating composition are not scattered in the seal part, sealing can be achieved without causing cutting in seal. The presence of the spots causes insufficient sealing.

Next, with respect to the moldability, a measurement device, a measurement method and a judgment method will be described below.

1) Measurement device:

With respect to the measurement device, a stainless steel-made molding die (a plate having a size of 2 mm in thickness $\times$ 200 mm in length $\times$ 200 mm in width and having a cavity as treated by R5 in four corners of 60 mm in length $\times$ 40 mm in width in a central part thereof) and a fixable leveling plate are disposed above a travelable endless belt, and magnets, (two magnets having a size of 12.5 mm in thickness $\times$ 24 mm in length $\times$ 24 mm in width are disposed in parallel) are disposed under the endless belt. The magnets should cover a region of the leveling plate and the vicinity thereof and a region larger than a region covered by a cut side (40 mm) vertical to the advancing direction of the cavity of the molding die.

2) Measurement method:

With respect to the measurement method, a stainless steel plate having a size of 1 mm in thickness $\times$ 200 mm in length $\times$ 200 mm in width is placed on the endless belt of the measurement device, a polyethylene film having a size of 70 $\mu$m in thickness $\times$ 200 mm in length $\times$ 200 mm in width is placed thereon, and a stainless steel-made molding die is further placed thereon. Thereafter, a leveling plate is fixed in a position of the cavity of the molding die of 50 mm far from the end portion in the advancing direction of the endless belt, 50 g of a heat generating composition is then placed in the vicinity of the leveling plate between the leveling plate and the cavity, and the heat generating composition is filled in the cavity of the molding die while leveling it by moving the endless belt at 1.8 m/min.

After the molding die has completely passed through the leveling plate, the traveling of the endless belt is stopped. Next, the molding die is removed, and a heat generating composition molded body as laminated on the polyethylene film is observed.

3) Judgment method:

With respect to the judgment method, in the surroundings of the heat generating composition molded body, in the case where any collapsed piece of the heat generating composition molded body exceeding a maximum length of 800 $\mu$m is not present and the number of collapsed pieces of the heat generating composition molded body having a maximum length of from 300 to 800 $\mu$m is not more than 5, it is to be noted that the heat generating composition has moldability. The moldability is an essential property for a heat generating composition to be used in the molding system. If the heat generating composition does not have moldability, it is impossible to produce a heat generating body by the molding system.

The heat generating composition of the invention has resistance to compression. The "resistance to compression" as referred to herein means that a heat generating composition compressed body obtained by compressing a heat generating composition molded body as accommodated in a molding die within the die to such an extent that the thickness is 70 % of the die thickness holds 80 % or more of exothermic rising properties of the exothermic rising properties of the heat generating composition molded body before compression (a difference in temperature between one minute and 3 minutes after starting a heat generation test of the heat generating composition).

Here, the measurement method of exothermic rinsing properties for the resistance to compression will be described below.

1. Heat generating composition molded body:

1) A magnet is provided in the vicinity of a central part of the back side of a polyvinyl chloride-made supporting plate (3 mm in thickness $\times$ 600 mm in length $\times$ 600 mm in width) of a footed supporting table so as to cover a cavity shape of a molding die.

2) A temperature sensor is placed on the central part the surface of the supporting plate.

3) A polyethylene film (25 $\mu$m in thickness $\times$ 250 mm in length $\times$ 200 mm in width) as provided with an adhesive layer having a thickness of about 80 $\mu$m is stuck onto the supporting plate via a sticky layer such that the center of the polyethylene film is positioned at the sensor.

4) On an underlay plate (280 mm in length $\times$ 150 mm in width $\times$ 50 $\mu$m to 2 mm in thickness), a polyethylene film (230 mm in length $\times$ 155 mm in width $\times$ 25 $\mu$m to 100 $\mu$m in thickness) is placed such that one end of the polyethylene film is projected by about 20 mm outside the underlay plate and that one end thereof in the length direction is substantially coincident with one end of the underlay plate.

5) A template (230 mm in length $\times$ 120 mm in width $\times$ 3 mm in thickness) having a cavity (80 mm in length $\times$ 50 mm in width $\times$ 3 mm in height) is placed on the polyethylen film placed on the underlay plate; a template is placed on the polyethylene film such that one end thereof in the length direction is fitted to one end where the underlay plate and the polyethylene film are coincident with each other and that in the width direction, one end part of the width of the template is placed at a position of the central part by about 20 mm far from an opposing end to the side where the polyethylene film is projected outward from the underlay plate. Next, the resulting assembly is placed on the supporting plate together with the underlay plate.

6) A sample is placed in the vicinity of the cavity; a force-in die plate is moved along the molding die; the sample is charged into the cavity while stuffing; and the sample is leveled while stuffing along the template plane (force-in die molding), thereby filling the sample in the die.

7) Next, the magnet beneath the supporting plate is removed; the end portion of the projected polyethylene film is pressed; the underlay plate is removed; and the temperature measurement is started.

2. Heat generating composition compressed body:

1) to 6) are the same as in the case of the heat generating composition molded body.

8) A die having a convex having a thickness of 0.9 mm which can substantially tightly come into the cavity in relation of the cavity with an unevenness is fitted to the cavity and compressed by a roll press or plate press to prepare a heat generating composition compressed body having a thickness of 2.1 mm (compressed to 70 % of the die thickness) within the die.

9) The resulting assembly is placed on the supporting plate together with the underlay plate; the magnet beneath the supporting plate is removed; the end portion of the projected polyethylene film is pressed; the underlay plate is removed; and the temperature measurement is started.

With respect to the measurement of the exothermic temperature, the temperature is measured for 5 minutes at a measurement timing of 2 seconds using a data collector, and resistance to compression is judged in terms of a difference in temperature between after elapsing one minute and after elapsing 3 minutes.

The thickness after compression is preferably from 50 to 99.5 %, more preferably from 60 to 99.5 %, and further preferably from 60 to 95 % of the die thickness.

Incidentally, in the invention, it is to be noted that the heat generating composition molded body includes a heat generating composition compressed body.

Further, the shape of the heat generating body is not limited but can be selected from the group consisting of a rectangular shape, a circular shape, an elliptical shape, a polygonal shape, a broad bean-like shape, an eye mask-like shape, a paper lantern-like shape, a cocoon-like shape, a gourd-like shape, a rectangular shape with rounded corners, a square shape with rounded corners, an egg-like shape, a boomerang-like shape, a comma-shaped bead-like shape, a wing-like shape, a nose-like shape, a star-like shape, and a foot-like shape.

Furthermore, the heat generating body or accommodating bag can be provided with at least one member of characters, designs, symbols, numerals, patterns, photographs, pictures, and colors in at least a part thereof.

Each of the substrate, the covering material and the adhesive layer constituting the heat generating body may be transparent, opaque, colored, or colorless. Furthermore, a layer constituting at least one layer of the layers constituting the respective materials and layers may be colored to a color different from those of other layers.

The heat generating body may be accommodated in an outer bag which is an air-impermeable accommodating bag, stored and transported. The outer bag is not limited so far as it is air-impermeable and may be made of a laminate. Examples thereof include a heat generating body prepared by interposing a produced heat generating body between two sheets of an air-impermeable film or sheet, punching the two sheets of film or sheet into a size larger than that of the heat generating body at the same time with or after this interposition, and sealing the two sheets of film or sheet in the surroundings exceeding the size of the heat generating body at the same time with or after this punching.

According to the process for producing a heat generating pad of the invention, plural heat generating composition molded bodies resulting from molding a moldable heat generating composition containing surplus water as a connecting substance by a force-through molding method or a cast molding method are laminated at intervals on a substrate which is substantially planar and does not have an accommodating pocket, a covering material is further covered thereon, and the surroundings of the heat generating composition molded bodies are sealed, thereby providing sectional exothermic parts in a striped form. Two or more of the sectional exothermic parts in a striped form are provided, the respective sectional exothermic parts in a striped form are disposed at intervals via the sectioned part as a heat sealing part, and an exothermic part is formed of a gathering of the sectional exothermic parts in a striped form. Furthermore, the circumferential surroundings of the respective sectional exothermic parts in a striped form adjacent to the surroundings of the heat generating pad are also heat sealed. Next, a heat generating pad is produced through a cutting step, etc. With respect to the sealing step and the cutting step and so on, conventional methods and devices may be properly selected and used.

The term "substantially planar" as referred to in the invention means a planar surface not having an accommodating concave such as an accommodating pocket, an accommodating section, and an accommodating zone as provided in advance for the purpose of accommodating the heat generating composition. Accordingly, irregularities which do not intentionally accommodate the heat generating composition may be present.

The "pocket" as referred to in the invention is an accommodating pocket which is provided in advance for the purpose of accommodating the heat generating composition and is a pocket as described in JP-T-2001-507593. Since irregularities which are not used for intentionally accommodating the heat generating composition molded body are not the pocket, even when such irregularities are present on a substrate, it is to be noted that such a substrate is defined as a substantially planar substrate.

The "accommodating section" as referred to herein is an accommodating section for accommodation as provided in advance on the packaging material for the purpose of accommodating the heat generating composition and is an accommodating section as described in Japanese Patent No. 3,161,605 and JP-T-11-508314. Since irregularities which are not used for intentionally accommodating the heat generating composition molded body are not the accommodating section, even when such irregularities are present on a substrate, it is to be noted that such a substrate is defined as a substantially planar substrate.

The "accommodating zone" as referred to herein is an accommodating zone for accommodation as provided in advance on the packaging material for the purpose of accommodating the heat generating composition and is an accommodating zone as described in Japanese Patent No. 3, 161, 605 and JP-T-11-508314. Since irregularities which are not used for intentionally accommodating the heat generating composition molded body are not the accommodating zone, even when such irregularities are present on a substrate, it is to be noted that such a substrate is defined as a substantially planar substrate.

Furthermore, the moldable heat generating composition or the heat generating composition molded body may be subjected to in-die compression or out-die compression. The "in-die compression" as referred to herein means that the heat generating composition is compressed by flexible rubber rolls or a compression die having a similar figure to the accommodation part or the like while the heat generating composition is present in the accommodation part within the die; and the "out-die compression" as referred to herein means that after the heat generating composition leaves from the die to become a heat generating composition molded body, the heat generating composition molded body is compressed by rolls or the like. Though this compression is usually carried out after covering the heat generating composition molded body by an underlay material and/or a covering material, this may not be carried out.

Incidentally, in the case where the heat generating composition molded body is compressed, with respect to a rate of compression, it is preferable that the heat generating composition molded body has a thickness of from 50 to 99.5 % on the basis of the thickness before the compression. The rate of compression is preferably from 50 to 95 %, more preferably from 55 to 95 %, further preferably from 60 to 95 %, and still further preferably from 65 to 90 %. Furthermore, with respect to the thickness before the compression, a thickness of a die at the time of die molding can be employed.

Furthermore, in the seal step, the seal is not limited so far as seal is possible. Usually, heat seal or compression seal or a mixture thereof is employed. The surface of the seal part may be of a plain shape or a patterned shape whose cross-sectional shape is irregular, and a mixture of a plain shape and a patterned shape whose cross-sectional shape is irregular. The mixture of pattern as referred to herein means a mixture of a plain shape in the inside of the seal part and a patterned shape in the outside of the seal part, or a mixture of a patterned shape in the inside of the seal part and a plain shape, a partially plain shape or a partially patterned shape in the outside of the seal part. Furthermore, the back side may be plain, with the front side being patterned, and vice versa. Furthermore, a part or the whole of the pattern may be a double pattern. Accordingly, following this, a plain or patterned seal roll is used as a seal roll. Furthermore, a pair of seal rolls may be used. Multiplex seal may be carried out by placing plural seal rolls of two or more. Examples of the multiplex seal include duplex seal, triplet seal, quadruplet seal, and quintuplet seal. The width of seal may be the same or different and may be properly determined. In the case of high-speed seal, a higher number of multiplex seal is preferable. In the case of using a seal roll or a compression seal roll to which the temperature is applied, the temperature of a pair of rolls may be the same, or the temperature of one roll may be different from that of the other roll.

The "force-through die molding" as referred to herein means a continuous formation method in which by using a molding machine for using a molding die and laminating a heat generating composition molded body having a shape of the molding die on a longitudinal substrate and a rotary sealer capable of covering the laminate by a longitudinal covering material and sealing (for example, heat seal, compression seal, and heat compression seal) a desired sectioned part and the substrate together with the surroundings of the covering material, the surroundings of the heat generating composition molded body and a necessary place of the sectioned part are heat sealed, thereby achieving a sealing treatment.

Furthermore, a magnet may be used for molding the moldable heat generating composition of the invention. By using a magnet, it becomes possible to easily achieve accommodation of the heat generating composition in a mold and separation of the molded body from the mold, thereby making it easier to mold a heat generating composition molded body.

The "cast molding method" as referred to herein means a molding method for laminating a heat generating composition molded body on a longitudinal substance by filling in a casting mold having a concave and transferring into a substrate. In the continuous case, there is enumerated a continuous formation method in which by using a molding machine for laminating a heat generating molding molded body on a longitudinal substrate by filling in a concave and transferring into a substrate by a drum-type rotary body and a rotary sealer capable of covering the laminate by a longitudinal covering material and sealing (for example, heat seal, compression seal, and heat compression seal) a desired sectioned part and the substrate together with the surroundings of the covering material, the surroundings of the heat generating composition molded body and a necessary place of the sectioned part are heat sealed, thereby achieving a sealing treatment.

Incidentally, the heat generating pad may be produced by providing an air-permeable sticky layer at least between the heat generating composition molded body and the covering material or providing an underlay material such as non-woven fabrics between the heat generating composition molded body and the covering material. In the case of providing an air-permeable sticky layer at least between the heat generating composition molded body and the covering material, there is no limitation so far as an air-permeable sticky layer is present at least between the heat generating composition molded body and the covering material. For example, the air-permeable sticky layer may be provided on the surface of the covering material opposing to the heat generating composition molded body; and the air-permeable sticky layer may be provided on the heat generating composition molded body or a laminate of the heat generating composition molded body and the substrate and temporarily adhered under pressure or the like between the covering material and the heat generating composition molded body and/or the substrate.

Furthermore, after temporarily adhering the covering material and the substrate and/or the heat generating composition molded body by the sticky layer among the heat generating composition molded body, the substrate and the covering material, the surroundings of the heat generating composition molded body and the surroundings of the heat generating pad may be heat sealed. In this way, the heat generating composition molded body become stable; real sealing by heat sealing becomes easy; seal deviation or the like does not occur; high-speed sealing becomes possible; and it becomes possible to realize a high-speed production process of a heat generating pad.

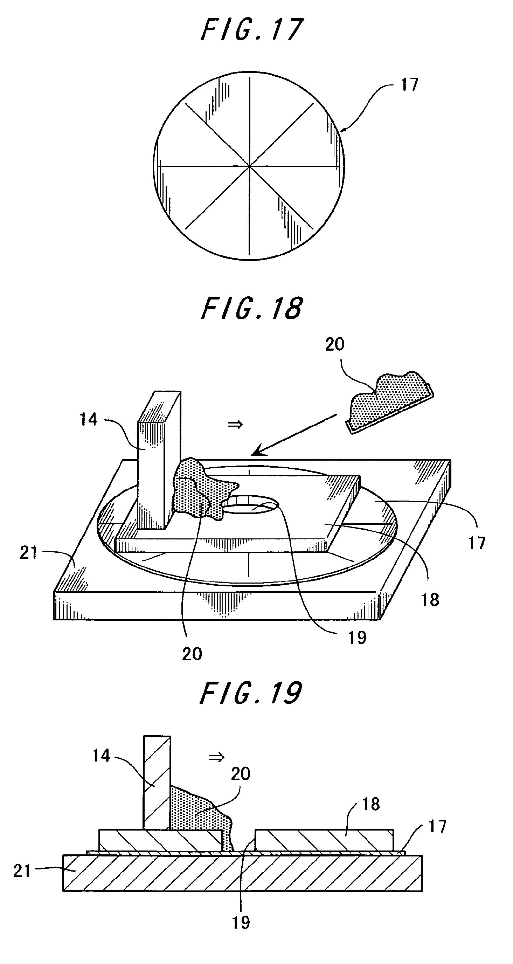

The "water mobility value" as referred to herein is a value showing an amount of surplus water which can transfer to the outside of the heat generating composition in water present in the heat generating composition. This water mobility value will be described below with reference to Figs. 17 to 21.

As shown in Fig. 17, a filter paper 17 of No. 2 (second class of JIS P3801) in which eight lines are drawn radiating from the central point with an interval of 45° is placed on a stainless steel plate 21 as shown in Figs. 18 and 19; a template 18 having a size of 150 mm in length $\times$ 100 mm in width and having a hollow cylindrical hole 19 having a size of 20 mm in inner diameter $\times$ 8 mm in height is placed in the center of the filter paper 18; a sample 20 is placed in the vicinity of the hollow cylindrical hole 19; and a stuffer plate 14 is moved on and along the template 18 and inserted into the hollow cylindrical hole 19 while stuffing the sample 20, thereby leveling the sample (force-in die molding).

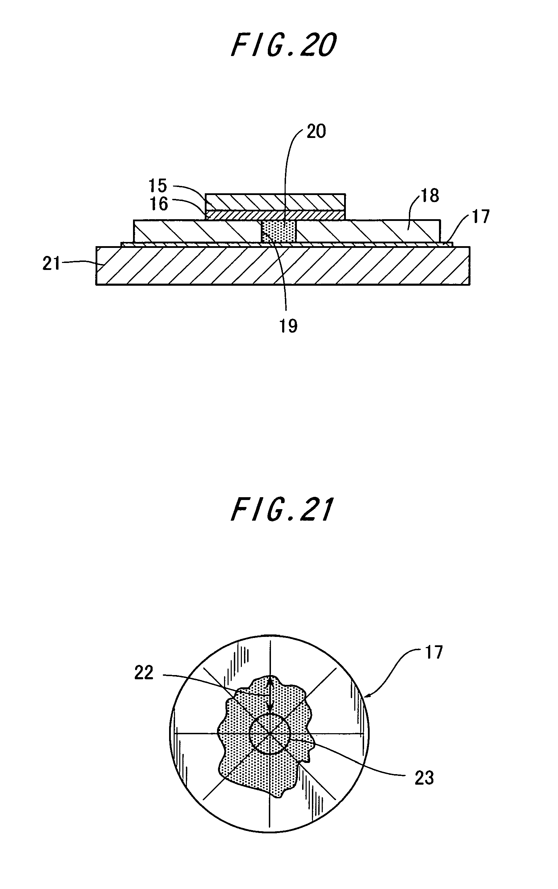

Next, as shown in Fig. 20, a non-water absorptive 70 $\mu$m-thick polyethylene film 16 is placed so as to cover the hole 19, and a flat plate 15 made of stainless steel having a size of 5 mm in thickness $\times$ 150 mm in length $\times$ 150 mm in width is further placed thereon and held for 5 minutes such that an exothermic reaction is not caused.

Thereafter, a shown in Fig. 21, the filter paper 17 is taken out, and an oozed-out locus of the water or aqueous solution is read as a distance 22 (unit: mm) from a periphery 23 as an edge of the hollow cylindrical hole to an oozed-out tip along the radiating lines. Similarly, a distance 22 from each of the lines is read, and eight values in total are obtained. Each of the eight values (a, b, c, d, e, f, g and h) which are read out is defined as a measured water content value. An arithmetic average value of the eight measured water content values is defined as a water content value (mm) of the sample.

Furthermore, the water content for the purpose of measuring a real water content value is defined as a compounded water content of the heat generating composition corresponding to the weight of the heat generating composition having a size of 20 mm in inner diameter $\times$ 8 mm in height or the like, similar measurement is conducted only with water corresponding to that water content, and a value as calculated in the same manner is defined as a real water content value (mm) . A value obtained by dividing the water content value by the real water content value and then multiplying with 100 is a water mobility value.

That is, the water mobility value is represented by the following expression.

```
(Water mobility value) = {[Water content value (mm)]/

[(Real water content value (mm))] × 100
```

With respect to the same sample, five points are measured, and the five water mobility values are averaged, thereby defining an average value thereof as a water mobility value of the sample.

Furthermore, in the case of measuring the water mobility value of the heat generating composition in the heat generating body, with respect to the water content for measuring a real water content, a percentage of water content of the heat generating composition is calculated through measurement of the water content of the heat generating composition by an infrared moisture meter, a water content necessary for the measurement is calculated on the basis of the percentage of water content, and a real water content value is measured and calculated from the foregoing water content. In the invention, a heat generating body can be formed only by laminating a heat generating composition molded body obtained by molding a heat generating composition having surplus water with a water mobility value of from 0.01 to 20 on a substrate, covering a covering material thereon, and sealing at least the periphery of the heat generating composition molded body. After accommodating it in a packaging material such as a substrate and a covering material, it is not necessary to add water. Accordingly, since the process is remarkably simplified, the invention is superior in view of the costs.

In the invention, the water mobility value (0 to 100) is preferably from 0.01 to 20, more preferably from 0.01 to 18, further preferably from 0.01 to 15, still further preferably from 0.01 to 13, even further preferably from 1 to 13, and even still further preferably from 3 to 13.

In a heat generating body using a heat generating composition molded body obtained by molding a moldable heat generating composition containing surplus water as a connecting substance according to the invention, the heat generating composition contains an appropriate amount of surplus water expressed by a water mobility value of from 0.01 to 20 as the connecting substance without using a flocculant aid, a dry binding agent, a flocculating agent, etc.

It is assumed that when the amount of surplus water in the heat generating composition is appropriate, the surplus water causes hydration against hydrophilic groups in the components of the composition due to a bipolar mutual action or hydrogen bond, etc. and that it is present even in the surroundings of hydrophobic groups while having high structural properties. Thus, it is assumed that the heat generating composition becomes in a state of a mud ball, thereby revealing moldability. This is connecting water as a connecting substance in some meaning. Besides, there is water in a state called as free water which can freely move, and it is thought that when the surplus water increases, the structure is softened, whereby the free water increases. Furthermore, controlling factors which an iron powder causes an oxidation reaction are an amount of existing water and a feed amount of oxygen to the surface of the iron powder. It is said that in a degree of water adsorbing film (less than 100 angstroms), the water is not sufficient and that the oxidation rate is small. When the adsorbing film becomes about 1 $\mu$m, the water content becomes sufficient. Furthermore, since the thickness of the water film is thin, feed of oxygen onto the surface of the iron powder becomes easy, whereby the oxidation rate becomes large. It is assumed that when the film becomes thicker to an extent that the adsorbing film exceeds 1 $\mu$m, the feed amount of oxygen is reduced. The present inventors have obtained knowledge that the water mobility value expressing the optimal water content at which moldability and oxidation rate in fixed levels or more are revealed is from 0.01 to 20, leading to accomplishment of the invention.

That is, by using an appropriate amount of surplus water, the respective component particles are coupled with each other by a surface tension of water, moldability is generated in the heat generating composition, and the water does not substantially function as a barrier layer. Thus, the heat generating composition comes into contact with air to generate heat. In addition, by using a heat generating composition using an active iron powder or an active heat generating composition using an active iron powder, the heat generating composition becomes a heat generating composition having remarkably excellent exothermic rising properties and high moldability. Furthermore, heat generation occurs without causing transfer of the water in the heat generating composition molded body as produced by a molding and laminating system into a packaging material or water absorptive sheet. In addition, by providing plural sectional exothermic parts of the heat generating composition molded body as sectioned by seal parts, it is possible to provide a heat generating body which has flexibility itself, is excellent in installation in places where flexibility is required, such as various places of a human body and curved bodies, and is excellent in feeling for use.

Furthermore, in the substrate, the covering material and the heat generating composition molded body, by temporarily adhering at least the covering material and the heat generating composition molded body to each other via a sticky layer and then heat sealing the periphery of the heat generating composition molded body and the surroundings of the heat generating body, certainty of heat seal is improved so that it becomes possible to design to make the production speed of a heat generating body high and make the heat seal width small.

The "moldability" as referred to in the invention exhibits that a molded body of the heat generating composition having a cavity or concave die shape is formed by force-through molding using a trimming die having a cavity or cast molding using a concave die, whereby after molding including mold release, the molding shape of the heat generating composition molded body is held.

When the moldability is revealed, since the shape is held until the heat generating composition molded article is at least covered by a covering material and a seal part is formed between the substrate and the covering material, sealing can be achieved in the periphery of the shape with a desired shape. Also, since so-called "spots" which are a collapsed piece of the heat generating composition are not scattered in the seal part, the sealing can be achieved without causing cutting in seal. The presence of the spots causes insufficient sealing.

1) Measurement device:

With respect to the measurement device, a stainless steel-made molding die (a plate having a size of 2 mm in thickness $\times$ 200 mm in length $\times$ 200 mm in width and having a cavity as treated by R5 in four corners of 60 mm in length x 40 mm in width in a central part thereof) and a fixable leveling plate are disposed above a travelable endless belt, and magnets (two magnets having a size of 12.5 mm in thickness x 24 mm in length $\times$ 24 mm in width are disposed in parallel) are disposed under the endless belt.

The magnets should cover a region of the leveling plate and the vicinity thereof and a region larger than a region covered by a cut side (40 mm) vertical to the advancing direction of the cavity of the molding die.

2) Measurement method:

With respect to the measurement method, a stainless steel plate having a size of 1 mm in thickness × 200 mm in length x 200 mm in width is placed on the endless belt of the measurement device, a polyethylene film having a size of 70 $\mu$m in thickness × 200 mm in length × 200 mm in width is placed thereon, and a stainless steel-made molding die is further placed thereon.

Thereafter, a leveling plate is fixed in a position of the cavity of the molding die of 50 mm far from the end portion in the advancing direction of the endless belt, 50 g of a heat generating composition is then placed in the vicinity of the leveling plate between the leveling plate and the cavity, and the heat generating composition is filled in the cavity of the molding die while leveling it by moving the endless belt at 1.8 m/min. After the molding die has completely passed through the leveling plate, the traveling of the endless belt is stopped. Next, the molding die is removed, and a heat generating composition molded body as laminated on the polyethylene film is observed.

3) Judgment method:

With respect to the judgment method, in the surroundings of the heat generating composition molded body, in the case where any collapsed piece of the heat generating composition molded body exceeding a maximum length of 800 $\mu$m is not present and the number of collapsed pieces of the heat generating composition molded body having a maximum length of from 300 to 800 $\mu$m is not more than 5, it is to be noted that the heat generating composition has moldability.

The moldability is an essential property for a heat generating composition to be used in the molding system. If the heat generating composition does not have moldability, it is impossible to produce a heat generating body by the molding system.

The "bending resistance" as referred to in the invention exhibits rigidity (tension or nerve) or flexibility and follows the A method according to JIS L1096 (45° cantilever method), except for using a heat generating body itself as a sample. That is, a heat generating body is placed on a horizontal table having a smooth surface and having a slope at an angle of 45° in one end thereof such that one side thereof coincides with a scale base line. Next, the heat generating body is slowly slid toward the slope by an appropriate method, and when a central point of the one end of the heat generating body comes into contact with the slope A, the position of the other end is read by a scale. The bending resistance is exhibited by a length (mm) for which the heat generating body moves. Respective five sheets of heat generating body are measured, and the bending resistance (calculated down to the integral place) is expressed by an average value of lengths measured in the length direction and the width direction, or in one direction and the orthogonal direction thereto. However, in the measurement, in the case of measuring an adhesive layer-provided heat generating body such that the adhesive side is faced at the horizontal table side, while the adhesive side provided with a separator is faced at the horizontal table side. In any way, a measured value in the side at which a minimum bending resistance is measured is employed.

Furthermore, in the measurement, the following must be taken into consideration.

(1) A heat generating composition-incorporated exothermic part of the heat generating body is to retain on the horizontal table to an extent of 5 mm or more in width x 20 mm or more in length. However, the length is to cross a region where the heat generating composition is present or to cross linearly a region where the heat generating composition is present and a region where the heat generating composition is not present.

(2) In the case of an adhesive layer-provided heat generating body, a plastic film having a bending resistance of not more than 30 mm, or a limp and soft film such as a limp film having a thickness of not more than 50 $\mu$m, and preferably not more than 25 $\mu$m and a plastic film in which wrinkles are formed by lightly crumpling is to be used as a separator of the adhesive layer and provided along the adhesive layer. Furthermore, with respect to the bending resistance of the substrate and/or the covering material, a specimen of 100 mm x 200 mm is prepared, and a bending resistance in the 200 mm direction is employed.

In the invention, the bending resistance in at least one direction is usually not more than 100 mm, preferably not more than 80 mm, more preferably not more than 50 mm, further preferably not more than 30 mm, and still further preferably not more than 20 mm.

A rate of bending resistance of the heat generating body or exothermic part in the invention is a rate of bending resistance to the full length of the heat generating body or exothermic part in one direction and is calculated according to the following expression.

$$\text{(Rate of bending resistance) = (A/B)} \times 100$$

Wherein A represents a bending resistance of the heat generating body or exothermic part in one direction; and B represents the full length of the heat generating body or exothermic part in the foregoing one direction.

In the invention, a rate of bending resistance in at least one direction is usually not more than 50, preferably not more than 40, and more preferably not more than 30.

A ratio of bending resistance in the invention is a ratio of a bending resistance in one direction to a smaller bending resistance in bending resistances in the directions orthogonal thereto in the plane orthogonal to the thickness direction of the heat generating body or exothermic part. The ratio of bending resistance is preferably 2 or more.

In the invention, in the case of a heat generating body having sectional exothermic parts provided at intervals in the striped form, a heat generating body provided with sectional exothermic parts of a parallelepiped shape at intervals in the striped form in which a maximum absolute value of a difference between bending resistances in the two directions as intersecting directions, a heat generating body further provided with an adhesive layer, and a heat generating body provided with adhesive layers at intervals in the striped form are very flexible in one direction and rigid in one direction. Thus, these heat generating bodies relieve symptoms such as stiff shoulders, lower-back pain, and muscular fatigue and especially exhibit efficacy for relieving a symptom of menstrual pain. In addition, these heat generating bodies are able to be wound in a size substantially equal to the width dimension in the width direction of the heat generating body, become compact and are convenient for accommodation. Furthermore, in the case of a separator-provided heat generating body, by using a separator having a low bending resistance, winding is possible.

Furthermore, in the case of providing a heat generating body along the body, the body includes many two-dimensional curves, and in shoulders, legs, abdomen, waist, arms, and the like, one direction is substantially linear, and the other two directions are formed of a substantially curved surface. Accordingly, since the heat generating body of the invention which is able to form a substantially linear surface in one direction and a curved surface in the other two directions is able to form a two-dimensional curved surface, it is able to well follow the body and is optimum for warming of the body and relaxation or treatment of various symptoms.

Furthermore, in the heat generating body of the invention, by adjusting the size or space of the convex sectional exothermic part, an exothermic part which is flexible and exhibits a uniform temperature distribution or an exothermic part exhibiting a pattern-like temperature distribution is obtainable. By the pattern-like temperature distribution, it is possible to improve a meridian effect of the warming part.

In the heat generating pad having sectional exothermic parts, a minimum bending resistance on the surface orthogonal to the thickness direction thereof is preferably not more than 50 mm, more preferably not more than 40 mm, further preferably not more than 30 mm, and still further preferably from 5 to 30 mm.

Such bending resistance and ratio of bending resistance are kept at least between 20 and 60 °C.

The "water retention" as referred to herein is a value as measured and calculated in the following method. That is, about 1 g of a sample fiber as prepared by cutting into a length of about 5 cm and well opening is dipped in pure water, and after elapsing 20 minutes (at 20 °C), water among the fibers is removed using a centrifuge by revolution at 2,000 rpm. A weight (W1) of the thus prepared sample is measured. Next, the sample is dried in a vacuum dryer at 80 °C until it becomes constant in weight, thereby measuring a weight (W2). A water retention is calculated according to the following expression.

$$\text{[Water retention (\%)] = [(W1 - W2)/W2]} \times 100$$

In the invention, the water retention is preferably 20 % or more.

The heat generating body of the invention is able to give various shapes, thicknesses and temperature zones and therefore, can be used for various utilities such as use for a joint, facial esthetic use, use for eyes, slimming use, use for heating or warming a dripping solution, use for a wet compress pack, use for a medical body warmer, use for a neck, use for a waist, use for a mask, use for a glove, use for hemorrhage, use for relaxation of symptoms such as shoulder pain, muscular pain, and menstrual pain, use for a cushion, use for heating or warming a human body during the operation, use for a thermal sheet, use for thermally volatilizing an aroma, use for an abdomen, insecticidal use by thermal volatilization, and use for treating cancer in addition to common warming of a human body. In addition, the heat generating body of the invention can be used for heating or warming machines, pets, etc.

For example, in the case of using for relaxation of symptoms, the heat generating body of the invention is applied directly in a necessary site of the body or indirectly via a cloth, etc. Furthermore, in the case of using for heating or warming a human body during the operation, a method for using the heat generating body of the invention includes the following methods.

(1) The heat generating body is directly applied to a body requiring heating or warming.
(2) The heat generating body is fixed on a covering, etc. and covered on the body.
(3) The heat generating body is fixed on a cushion to be placed beneath the body, etc.
(4) The heat generating body is used as a covering or a cushion which is a product having the heat generating body provided therein in advance.

Incidentally, examples of the pain of muscles or bones include acute muscle pain, acute bone pain, acute reference pain, previous muscle pain, previous bone pain, chronic reference pain, and join pain of knee, elbow, etc.

The holding time is not limited but is preferably from 20 seconds to 24 hours, more preferably from one hour to 24 hours, and further preferably from 8 hours to 24 hours.

The holding temperature is preferably from 30 to 50 °C, more preferably from 32 to 50 °C, further preferably from 32 to 43 °C, still further preferably from 32 to 41 °C, and even further preferably from 32 to 39 °C.

The invention will be specifically described below with reference to the Examples, but it should not be construed that the invention is limited thereto.

[Brief Description of the Drawings]

Fig. 1 is a plan view of an embodiment of the heat generating pad of the invention.
Fig. 2(a) is a cross-sectional view along the line Z-Z of the same; and Fig. 2(b) is a cross-sectional view of other embodiment of the heat generating pad of the invention.
Fig. 3 is an enlarged view of a part of Fig. 2.
Fig. 4 is a plan view of women's panties as seen from the waist part and is an explanatory view of the heat generating pad of one example in which the panties are worn, as stuck to the outside of the panties such that when the panties are worn, the heat generating pad transfers heat to the abdominal of a user.
Fig. 5 is a plan view of women's panties as seen from the waist part and is an explanatory view of the heat generating pad of one example in which the panties are worn, as stuck to the inside of the panties such that when the panties are worn, the heat generating pad transfers heat to the abdominal of a user.
Fig. 6 is a plan view of the waist part where women's panties are worn as seen from the waist part and is an explanatory view of the heat generating pad of one example in which the panties are worn, as stuck to the abdominal of a user such that when the panties are worn, the heat generating pad transfers heat to the abdominal of a user.

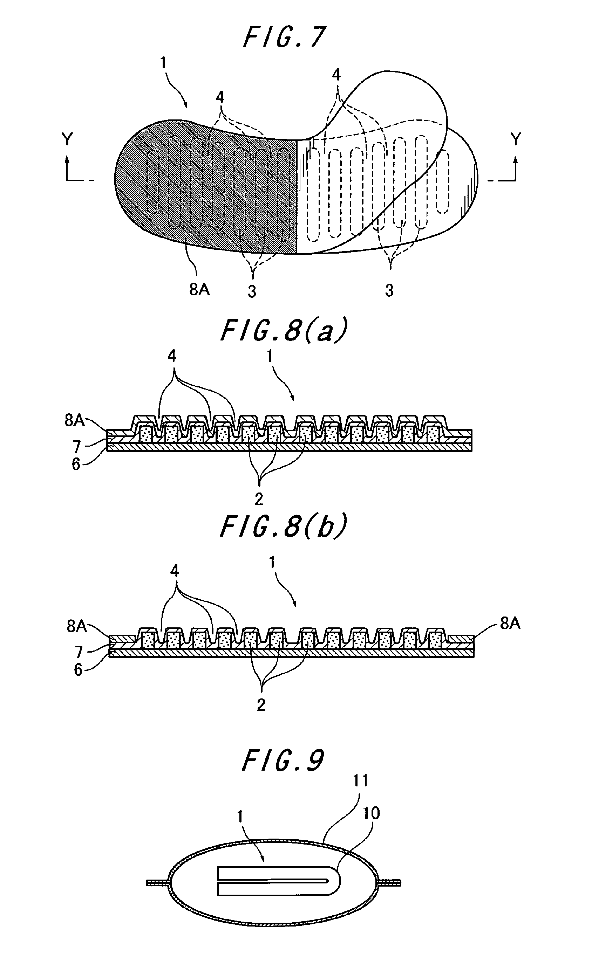

Fig. 7 is an oblique view of other embodiment of the heat generating pad of the invention.
Fig. 8(a) is a cross-sectional view along the line Y-Y of the same; and Fig. 8(b) is a cross-sectional view of other embodiment of the heat generating pad of the invention.
Fig. 9 is a cross-sectional view to show one example of the heat generating pad as folded in an outer bag which is an air-impermeable accommodating bag.
Fig. 10 is a plan view of other embodiment of the heat generating pad of the invention.
Fig. 11 (a) is a plan view of other embodiment of the heat generating pad of the invention; and Fig. 11(b) is a cross-sectional view along the line Y-Y of the same.'
Fig. 12 is an enlarged cross-sectional view of other embodiment of the heat generating pad of the invention.
Fig. 13 is a plan view of other embodiment of the heat generating pad of the invention.

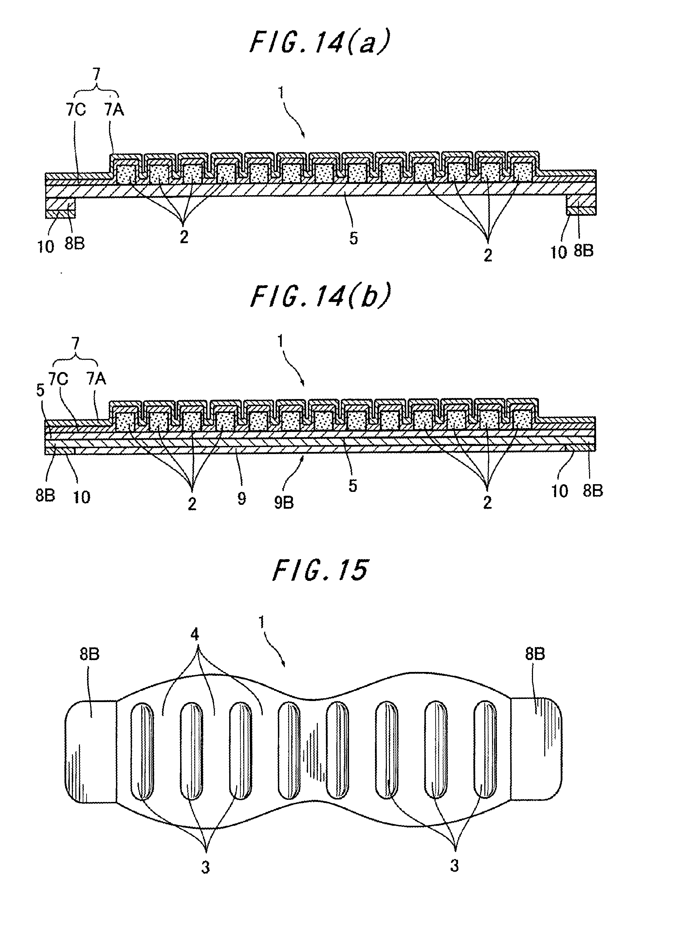

Fig. 14 (a) is a cross-sectional view along the line W-W of the same; and Fig. 14 (b) is a cross-sectional view of other embodiment.
Fig. 15 is a plan view of other embodiment of the heat generating pad of the invention.
Figs. 16(a) to 16(q) each shows an explanatory view of one example of the shape of the heat generating pad of the invention.
Fig. 17 is a plan view of a filter paper for the measurement of water mobility value in the invention.
Fig. 18 is an oblique view for explaining the measurement of water mobility value in the invention.
Fig. 19 is a cross-sectional view for explaining the measurement of water mobility value in the invention.
Fig. 20 is a cross-sectional view for explaining the measurement of water mobility value in the invention.
Fig. 21 is a plan view of a filter paper after carrying out the measurement of water mobility value in the invention.

[Description of Reference Numerals and Signs]

1:      Heat generating pad
2:      Heat generating composition molded body
3:      Sectional exothermic part
4:      Sectioned part
5:      Circumferential seal part
6:      Substrate
7:      Covering material
7A:     Non-woven fabric
7B:     Porous film
8:      Adhesive layer
9:      Air permeability adjusting material
9A:     Space
9B:     Thermal buffer sheet
10:     Separator
11:     Air-impermeable outer bag
14:     Pushing plate
15:     Flat plate
16:     Non-water absorptive film (for example, a polyethylene film)
17:     Filter paper in which eight lines are drawn radiating from the central point with an interval of 45°
18:     Die plate having a hollow cylindrical hole
19:     Hole
20:     Sample
21:     Stainless steel plate
22:     Distance to the oozed-out locus of water or aqueous solution
23:     Position corresponding to a hollow cylindrical hole on filter paper

[Examples]

(Example 1)

This Example will be described below with reference to Fig. 1.

A heat generating composition having a water mobility value of 6, which is a mixture consisting of 100 parts by weight of a reduced iron powder (particle size: not more than 300 $\mu$m), 7.0 parts by weight of active carbon (particle size: not more than 300 $\mu$m), 5.0 parts by weight of a wood meal (particle size: not more than 300 $\mu$m) 0. 8 parts by weight of a water absorptive polymer (particle size: not more than 300 $\mu$m), 0.2 parts by weight of calcium hydroxide, 0.7 parts by weight of sodium sulfite and 11 % of salt water, was used.

Next, the following molding was carried out by using a trimming die provided with fourteen cavities (5 mm in width x 80 mm in length) in total in a striped form at intervals of 5 mm. Incidentally, this trimming die is provided with a gap of 10 mm is provided in the central part between the seven cavities and the seven cavities.

By using the heat generating composition, a heat generating composition molded body 2 constituting fourteen sectional exothermic parts 3 was provided on a 30 $\mu$m-thick substrate made of a polyethylene film 6 on a separator 10 via an acrylic adhesive layer 8; next, an air-permeable covering material 7 made of a laminate of a nylon-made non-woven fabric with a basis weight of 40 g/m$^2$ on a polyethylene-made porous film was covered thereon; and a gap between the respective heat generating composition molded bodies 2 and the outer circumference as a heat generating pad 1 were then sealed. The periphery of the respective heat generating composition molded bodies 2 was heat sealed in a seal width of 3 mm. Furthermore, the outer circumference as a heat generating pad 1 was sealed in a seal width of 8 mm. There was thus obtained a heat generating pad 1 having an external dimension of 158 mm in maximum length $\times$ 98 mm in maximum width. Incidentally, the air permeability of the air-permeable covering material 7 was 400 g/m$^2$/24 hr in terms of a moisture permeability by the Lyssy method. Furthermore, the heat generating pad 1 provided with a separator having a bending resistance of 20 mm had a bending resistance of 30 mm in the long side direction (direction orthogonal to the stripe direction) and 80 mm or more in the short side direction (stripe direction), respectively. A ratio of bending resistance was 2 or more. The heat generating pad 1 was very excellent in handling properties and feeling for use because the bending resistance in one direction is very high, whereas the bending resistance in a direction substantially orthogonal thereto is very low. Furthermore, this heat generating pad 1 is able to be wound, becomes compact and is convenient for accommodation. Furthermore, as described previously, in the case of providing the separator 10, a separator having a low bending resistance may be used.

This heat generating pad 1 was sealed and accommodated in an air-impermeable accommodating bag (hereinafter referred to as "outer bag") and allowed to stand at room temperature for 24 hours. After 24 hours, the heat generating pad was taken out from the outer bag and then stuck to the outside of panties, thereby carrying out an exothermic test of the body. As a result, it was felt warm within 3 minutes, and the warmth was continued for 7 hours. At the same time, curved surface fitness, winding properties and usefulness were evaluated. As a result, the heat generating pad was superior in all of these evaluations. Incidentally, the adhesive layer can be provided only in both end parts of the heat generating pad 1 as shown in Fig. 2(b).

(Example 2)

A batchwise stirring tank composed of a mixer equipped with a rotary blade in a blade form of a ventilation fan was used as an oxidizing gas contact treatment device, and air was used as an oxidizing gas. First of all, a reaction mixture consisting of 100 parts by weight of a reduced iron powder (particle size: not more than 300 $\mu$m) and 5 parts by weight of 11 % salt water and having a water mobility value of less more than 0.01 was charged in the contact treatment device vessel. Next, the upper portion of the contact treatment device vessel was opened to air, and the reaction mixture was subjected to self heat generation with stirring in the opened state to air under circumstances at 20 °C. At a point of time when the temperature rise of the reaction mixture reached 10 °C, the reaction mixture was sealed in an air-impermeable accommodating bag and cooled to room temperature, thereby obtaining a heat generating mixture. The heat generating mixture had a wustite content of 10 % by weight. Next, the contact treated reaction mixture was mixed with 5.3 parts by weight of active carbon (particle size: not more than 300 $\mu$m), 5 parts by weight of a wood meal (particle size: not more than 300 $\mu$m), 1.2 parts by weight of a water absorptive polymer (particle size: not more than 300 $\mu$m), 0.2 parts by weight of calcium hydroxide, 0.7 parts by weight of sodium sulfite and 11 % salt water, thereby obtaining a heat generating composition having a water mobility value of 8. Next, by using the heat generating composition and using the substrate and covering material as in Example 1, a heat generating composition molded body was laminated on the substrate in the same manner as in Example 1 by force-through molding using a trimming die in which a sectioned part having a width of 10 mm was provided in the central part thereof and eight (sixteen in total) cavities having 5 mm in width × 35 mm in length in a striped form were respectively provided at intervals of 5 mm while interposing the sectioned part. Next, a non-woven fabric in which an olefin based hot melt based adhesive had been provided in a netlike form on a porous film as a covering material by a melt blow method was covered thereon such that the adhesive layer and the heat generating composition molded body were faced at each other. The periphery of the heat generating molded body was sealed by pressure bonding in a seal width of the central part of 8 mm and in a seal width of the other part of 3 mm by a temporary adhering seal plate, thereby providing a temporary adhering seal part. Next, the substantially central part of the temporary adhering seal part was heat sealed by a heat seal plate with a seal width of the central part of 5 mm and a seal width of the other part of 1 mm, and the periphery of the heat generating pad was further heat sealed in a seal width of 8 mm. Next, the surface of the heat generating pad was made even by a press roll, and a part of the heat generating composition molded body was incorporated into the temporary adhering seal part, thereby obtaining a heat generating pad. Furthermore, the heat generating pad provided with a separator having a bending resistance of 20 mm had a bending resistance of 30 mm in the long side direction (direction orthogonal to the stripe direction) and 80 mm or more in the short side direction (stripe direction), respectively. A ratio of bending resistance was 2 or more. In the case where the bending resistance in one direction is very high, whereas the bending resistance in a direction substantially orthogonal thereto is very low, the heat generating pad is very excellent in handling properties and feeling for use. Furthermore, this heat generating pad is able to be wound in a size substantially equal to the width in the width direction thereof, becomes compact and is convenient for accommodation. This heat generating pad was sealed and accommodated in an outer bag and allowed to stand at room temperature for 24 hours. After 24 hours, the heat generating pad was taken out from the outer bag and then subjected to an exothermic test of the body. As a result, it was felt warm within 3 minutes, and the warmth was continued for 7 hours. At the same time, curved surface fitness, winding properties and usefulness were evaluated. As a result, the heat generating pad was superior in all of these evaluations.

(Example 3)

As a heat generating composition, a reaction mixture consisting of 100 parts by weight of a reduced iron powder (particle size: not more than 300 $\mu$m), 5.5 parts by weight of active carbon (particle size: not more than 300 $\mu$m), 4.0 parts by weight of a wood meal (particle size: not more than 300 $\mu$m), 2.2 parts by weight of a water absorptive polymer (particle size: not more than 300 $\mu$m), 0.2 parts by weight of calcium hydroxide, 0.7 parts by weight of sodium sulfite and 6.0 % salt water and having a water mobility value of less than 0.01 was charged in a contact treatment device vessel. Next, the upper portion of the contact treatment device vessel was opened to air, and the reaction mixture was subjected to self heat generation with stirring in the opened state to air under circumstances at 25 °C. At a point of time when the temperature rise of the reaction mixture reached 25 °C, the reaction mixture was sealed in an air-impermeable accommodating bag and cooled to room temperature, thereby obtaining a heat generating mixture. 6.0 % salt water was mixed in the heat generating mixture to obtain a heat generating composition having a water mobility value of 10.

A covering material the same as in Example 2 was used, and a laminate of a napped non-woven fabric on a polyethylene film was used as a substrate. A heat generating composition molded body was laminated on the substrate by force-through molding using a trimming die provided with twelve cavities (5 mm in width $\times$ 80 mm in length) in total at intervals of 7 mm. By using a temporary adhering seal plate with a seal width of 5 mm and a heat seal plate with a seal width of 3 mm, the surroundings of the sectional exothermic parts were sealed in a seal width of 3 mm to form a sectioned part and the periphery of the heat generating pad was sealed in a seal width of 8 mm in the same manner as in Example 2, thereby obtaining a heat generating pad of 153 mm in length $\times$ 98 mm in width having twelve rectangular sectional exothermic parts in a striped form. Next, a hot melt based adhesive layer was provided in a netlike form on an air-permeable covering material by a melt blow method using an olefin based hot melt based adhesive to form an air-permeable adhesive layer-provided air-permeable covering material, and a separator was further covered thereon, followed by cutting to obtain a heat generating pad 1 as shown in Fig. 7 and Fig. 8 (a) . Furthermore, when the separator was removed, the heat generating pad had a bending resistance of 80 mm or more in the long side direction (direction orthogonal to the stripe direction) and 30 mm in the short side direction (stripe direction) with respect to the exothermic part, respectively. A bending resistance was 2 ore more. In the case where the bending resistance in one direction is very high, whereas the bending resistance in a direction substantially orthogonal thereto is very low, the heat generating pad was very excellent in handling properties and feeling for use.

Furthermore, this heat generating pad is able to be wound in a size substantially equal to the width in the width direction thereof, becomes compact and is convenient for accommodation.

Incidentally, in this Example, since a separator having a low bending resistance was used, the heat generating pad could be wound even in a state that it was provided with such a separator. This heat generating pad was sealed and accommodated in an outer bag and allowed to stand at room temperature for 24 hours. After 24 hours, the heat generating pad was taken out from the outer bag and then subjected to an exothermic test. As a result, the temperature reached 34 °C within 3 minutes, and the duration of heat generation at 34 °C or higher was long as 8 hours. Furthermore, as shown in Fig. 5, the heat generating pad 1 was stuck to the inside of panties 8 and then subjected to an exothermic test of the body. Temperature characteristics, curved surface fitness, winding properties and usefulness were evaluated. As a result, the heat generating pad was superior in all of these evaluations. Fig. 8 (b) shows a modification of Fig. 8 (a) and is a cross-sectional view of other example in which an adhesive layer 8A is provided in each end part of the heat generating pad in the side of the air-permeable surface.

(Example 4)

A heat generating pad provided with a perforation to such a degree that cutting by hand is possible in the sectioned part of the heat generating pad as prepared in Example 3 was prepared. The heat generating pad was sealed and accommodated in an air-impermeable outer bag and allowed to stand at 60 °C for 24 hours. After 24 hours, the temperature was returned to room temperature, and the heat generating pad was taken out from the outer bag. After removing the separator, the heat generating pad was stuck to the body and subjected to an exothermic test of the body. As a result, it was felt warm within 3 minutes, and the warmth was contained for 8 hours or more. Furthermore, curved surface fitness, winding properties and usefulness were evaluated. As a result, the heat generating pad was superior in all of these evaluations. Furthermore, by drawing the heat generating pad right and left from the end of the sectional exothermic part thereof by fingers along the perforation, two heat generating pads each having six sectional exothermic parts could be easily prepared. Also, the heat generating pad could be subdivided and worn separately in a necessary place depending upon the desire of a user so that it was satisfactory in usefulness.

(Example 5)

A heat generating composition having a water mobility value of 7, which is a mixture consisting of 100 parts by weight of a reduced iron powder (particle size: not more than 300 $\mu$m), 5.5 parts by weight of active carbon (particle size: not more than 300 $\mu$m), 4.0 parts by weight of a wood meal (particle size: not more than 300 $\mu$m), 2.2 parts by weight of a water absorptive polymer (particle size: not more than 300 $\mu$m), 0.2 parts by weight of calcium hydroxide, 0.7 parts by weight of sodium sulfite and 11 % of salt water, was used.

Next, by using the heat generating composition and using a trimming die provided with thirteen cavities (5 mm in width x 80 mm in length) in a striped form at intervals of 5 mm, as shown in Fig. 13 and Fig. 14 (a), a heat generating composition molded body 2 constituting thirteen sectional exothermic parts 3 was provided on the polyethylene film surface of a substrate 5 made of a polyethylene film laminating a napped nylon non-woven fabric on one surface thereof; next, an air-permeable covering material 7 having a nylon-made non-woven fabric 7A with a basis weight of 80 g/m$^2$ laminated on a polyethylene-made porous film 7C was covered thereon; and the circumference of the respective heat generating composition molded bodies 2 and the outer circumference as a heat generating pad 1 were then sealed. The outer circumference of the respective heat generating composition molded bodies 2 was heat sealed in a seal width of 3 mm.

Furthermore, the outer circumference of the heat generating pad 1 was sealed in a seal width of 8 mm. There was thus obtained a thermal body wrap 1 having an external dimension of 158 mm in maximum length $\times$ 98 mm in maximum width. Next, an adhesive double coated tape 8B provided with a separator 10 was stuck to each end part of the heat generating pad 1 in the side of the substrate 5, thereby forming a heat generating pad 1. Incidentally, the air permeability of the air-permeable covering material 7 was 400 g/m$^2$/24 hr in terms of a moisture permeability by the Lyssy method. Furthermore, when the separator 10 was removed, the heat generating pad 1 had a bending resistance of 80 mm or more in the long side direction (direction orthogonal to the stripe direction) and 30 mm in the short side direction (stripe direction) with respect to the exothermic part, respectively. A ratio of bending resistance was 2 or more. In the case where the bending resistance in one direction is very high, whereas the bending resistance in a direction substantially orthogonal thereto is very low, the heat generating pad is very excellent in handling properties and feeling for use. Furthermore, this heat generating pad becomes compact by winding up and is convenient for accommodation. Incidentally, as shown in Fig. 14(b), a thermal buffer sheet 9B may be provided on an adhesive layer 8.

This heat generating pad 1 was sealed and accommodated in an air-impermeable accommodating bag (hereinafter referred to as "outer bag") and allowed to stand at room temperature for 24 hours. After 24 hours, the heat generating pad was taken out from the outer bag and then subjected to an exothermic test by hanging it on a shoulder, sticking it to the shoulder and bringing the side of the napped non-woven fabric into contact with the skin. As a result, it was felt warm within 3 minutes, and the warmth was continued for 7 hours. At the same time, curved surface fitness, winding properties, feeling for use and usefulness were evaluated. As a result, the heat generating pad was superior in all of these evaluations. A heat generating pad as shown in Fig. 15 is a modification of the heat generating pad 1 as shown in Fig. 13 and was formed such that the central part thereof is constricted.

(Example 6)

A batchwise stirring tank composed of a mixer equipped with a rotary blade in a blade form of a ventilation fan was used as an oxidizing gas contact treatment device, and air was used as an oxidizing gas. First of all, a reaction mixture consisting of 100 parts by weight of a reduced iron powder (particle size: not more than 300 $\mu$m) and 5 parts by weight of 11 % salt water and having a water mobility value of less than 0.01 was charged in the contact treatment device vessel. Next, the upper portion of the contact treatment device vessel was opened to air, and the reaction mixture was subjected to self heat generation with stirring in the opened state to air under circumstances at 20 °C. At a point of time when the temperature rise of the reaction mixture reached 15 °C, the reaction mixture was sealed in an air-impermeable accommodating bag and cooled to room temperature, thereby obtaining a heat generating mixture. The heat generating mixture had a wustite content of 12 %. Next, the heat generating mixture was mixed with 5. 3 parts by weight of active carbon (particle size: not more than 300 $\mu$m), 5 parts by weight of a wood meal (particle size: not more than 300 $\mu$m), 1.2 parts by weight of a water absorptive polymer (particle size: not more than 300 $\mu$m), 0.2 parts by weight of calcium hydroxide, 0.7 parts by weight of sodium sulfite and 11 % salt water, thereby obtaining a heat generating composition having a water mobility value of 15. Next, by using the heat generating composition and using the substrate and covering material as in Example 1, a heat generating composition molded body was laminated on the substrate in the same manner as in Example 1 by force-through molding using a trimming die in which a sectioned part having a width of 10 mm was provided in the central part thereof and eight (sixteen in total) cavities having 5 mm in width $\times$ 35 mm in length in a striped form were respectively provided at intervals of 5 mm while interposing the sectioned part. Next, a non-woven fabric in which an olefin based hot melt based adhesive had been provided in a netlike form on a porous film as a covering material by a melt blow method was covered thereon such that the adhesive layer and the heat generating composition molded body were faced at each other. The periphery of the heat generating molded body was sealed by pressure bonding in a seal width of the central part of 8 mm and in a seal width of the other part of 3 mm by a temporary adhering seal plate, thereby providing a temporary adhering seal part. Next, the substantially central part of the temporary adhering seal part was heat sealed by a heat seal plate with a seal width of the central part of 5 mm and a seal width of the other part of 1 mm, and the periphery of the heat generating pad was further heat sealed in a seal width of 8 mm. Next, the surface of the heat generating pad was made even by a press roll, and a part of the heat generating composition molded body was incorporated into the temporary adhering seal part, thereby obtaining a heat generating pad.

This heat generating pad was sealed and accommodated in an outer bag and allowed to stand at room temperature for 24 hours. After 24 hours, the heat generating pad was taken out from the outer bag and then subjected to an exothermic test of the body. As a result, it was felt warm within 3 minutes, and the warmth was continued for 7 hours. At the same time, curved surface fitness, winding properties and usefulness were evaluated. As a result, the heat generating pad was superior in all of these evaluations.

(Example 7)

As a heat generating composition, a reaction mixture consisting of 100 parts by weight of a reduced iron powder (particle size: not more than 300 $\mu$m), 5.5 parts by weight of active carbon (particle size: not more than 300 $\mu$m), 20 parts by weight of a wood meal (particle size: not more than 300 $\mu$m), 2.2 parts by weight of a water absorptive polymer (particle size: not more than 300 $\mu$m), 0.2 parts by weight of calcium hydroxide, 0.7 parts by weight of sodium sulfite and 3. 5 % salt water and having a water mobility value of less than 0.01 was charged in a contact treatment device vessel. Next, the upper portion of the contact treatment device vessel was opened to air, and the reaction mixture was subjected to self heat generation with stirring in the opened state to air under circumstances at 20 °C. At a point of time when the temperature rise of the reaction mixture reached 30 °C, the reaction mixture was sealed in an air-impermeable accommodating bag and cooled to room temperature, thereby obtaining a heat generating mixture. 3.5 % salt water was mixed in the heat generating mixture to obtain a heat generating composition having a water mobility value of 7. Next, by using a covering material the same as in Example 6 and a substrate the same as in Example 1 except for not providing an adhesive layer, a heat generating composition molded body was laminated on the substrate using a trimming die provided with twelve cavities (5 mm in width $\times$ 80 mm in length) at intervals of 7 mm. By using a temporary adhering seal plate with a seal width of 5 mm and a heat seal plate with a seal width of 3 mm, the surroundings of the sectional exothermic parts were sealed in a seal width of 3 mm and the outer circumference as a heat generating pad 1 was sealed in a seal width of 8 mm in the same manner as in Example 2, thereby obtaining a heat generating pad of 153 mm in length $\times$ 98 mm in width having twelve rectangular sectional exothermic parts in a striped form. Next, a hot melt based adhesive layer was provided in a netlike form by a melt blow method using an olefin based hot melt based adhesive, and a separator was further covered thereon, followed by cutting to obtain a heat generating pad 1. Furthermore, when the separator was removed, the heat generating pad had a bending resistance of 80 mm or more in the long side direction (direction orthogonal to the stripe direction) and 30 mm in the short side direction (stripe direction) with respect to the exothermic part, respectively. A ratio of bending resistance was 2 or more. In the case where the bending resistance in one direction is very high, whereas the bending resistance in a direction substantially orthogonal thereto is very low, the heat generating pad is very excellent in handling properties and feeling for use. Furthermore, this heat generating pad is able to be wound in the width direction thereof, becomes compact and is convenient for accommodation. Incidentally, in this Example, since a separator having a low bending resistance was used, the heat generating pad could be wound even in a state that it was provided with such a separator.

The heat generating pad 1 was sealed and accommodated in an air-impermeable outer bag and allowed to stand at room temperature for 24 hours. After 24 hours, the heat generating pad was taken out from the outer bag and then subjected to an exothermic test of the body. As a result, the temperature reached 34 °C within 3 minutes, and the duration of heat generation of 34 °C or higher was long as 8 hours. Furthermore, by drawing the heat generating pad right and left from the end of the sectional exothermic part thereof by fingers along the perforation, two heat generating pads each having six sectional exothermic parts could be easily prepared. The results of the exothermic test were satisfactory. Furthermore, the heat generating pad was evaluated by subjecting to an exothermic test of the body with respect to curved surface fitness, winding properties and usefulness were evaluated. As a result, the heat generating pad was superior in all of these evaluations.

(Example 8)

A heat generating pad provided with a hydrophilic adhesive layer was prepared by using, as the substrate, a heat seal layer-provided laminate (moisture permeability: 0.3 g/m$^2$/day) which is a laminate of biaxially stretched polypropylene and a silicon oxide-deposited polyester film and changing the adhesive layer to a hydrophilic adhesive layer. The heat generating pad was sealed and accommodated in an air-impermeable outer bag and allowed to stand at 60 °C for 24 hours. After 24 hours, the temperature was returned to room temperature, and the heat generating pad was taken out from the outer bag. After removing the separator, the heat generating pad was stuck to the body and subjected to an exothermic test of the body. As a result, it was felt warm within 3 minutes, and the favorable warmth was continued for 8 hours or more. Furthermore, the heat generating pad was evaluated with respect to curved surface fitness, winding properties, feeling for use and usefulness were evaluated. As a result, the heat generating pad was superior in all of these evaluations. Incidentally, the hydrophilic adhesive layer was prepared in the following manner. A component made of 4.5% by weight of polyacrylic acid, 1.5 % by weight of poly(sodium acrylate), 4.0 % by weight of carboxymethyl cellulose, 15.0 % by weight of glycerin, 5.0 % by weight of propylene glycol, 0.1 % by weight of aluminum hydroxide, 0.05 % by weight of synthetic hydrotalcite, 1.0 % by weight of polyoxyethylene glycol, 6.0 % by weight of kaolin and 62. 85 % by weight of water was charged in a mixing machine and thoroughly stirred until the mixture became pasty, thereby preparing a hydrophilic adhesive. This hydrophilic adhesive was uniformly coated on a separator resulting from a silicone treatment of polyethylene terephthalate (PET) having a thickness of 40 $\mu$m. The surface of this hydrophilic adhesive layer was stuck onto the exposed surface of the substrate of the heat generating pad. Furthermore, the heat generating pad having been sealed and accommodated in an outer bag was kept in a thermostatic chamber at 50 °C for 10 days. As a result, the exothermic characteristics of the heat generating pad after keeping were the same as those before keeping. Furthermore, the heat generating pad having been sealed and accommodated in an outer bag was kept in a thermostatic chamber at 50 °C for 10 days. As a result, the exothermic characteristics of the heat generating pad after keeping were the same as those before keeping.

The time when the reaction mixtures in the present Examples came into contact with air as the oxidizing gas was all within 5 minutes.

**Claims**

1. A heat generating pad in which a heat generating composition molded body resulting from molding a moldable heat generating composition containing surplus water as a connecting substance is interposed between packaging materials and the periphery of the heat generating composition molded body is heat sealed, **characterized in that**:

    1) the packaging materials are constituted of a substrate and a covering material,
    2) the substrate is substantially planar and does not contain an accommodating pocket,
    3) the heat sealing forms a sectioned part as formed in a striped form, and plural sectional exothermic parts as sectioned by the heat sealing are provided via the sectioned part,
    4) the moldable heat generating composition has a water content of from 1 to 60 % by weight, contains, as essential components, an iron powder, a carbon component, a reaction accelerator and water, does not contain a flocculant aid, a flocculant, an agglomeration aid, a dry binder, a dry binding agent, a dry binding material, a sticky raw material, a thickener and an excipient, contains the surplus water so as to have a water mobility value of from 0.01 to 20, with the water in the moldable heat generating composition not functioning as a barrier layer, and is capable of causing an exothermic reaction upon contact with air,
    5) the heat generating pad contains a region having a ratio of bending resistance in the orthogonal directions of 2 or more on the surface thereof orthogonal to the thickness direction of the heat generating pad,
    6) at least a part of the heat generating pad has air permeability, and
    7) the heat generating pad has a fixing measure on at least a part of the exposed surface thereof.

2. The heat generating pad according to claim 1, **characterized in that** a minimum bending resistance in one direction on the surface orthogonal to the thickness direction of the heat generating pad is not more than 100 mm.

3. The heat generating pad according to claim 1, **characterized in that** a ratio of the capacity of the sectional exothermic parts to a volume of the heat generating composition molded body is from 0.6 to 1.0.

4. The heat generating pad according to claim 1, **characterized in that** the heat generating composition contains a component resulting from a contact treatment of a mixture containing at least an iron powder, a carbon component, a reaction accelerator and water as essential components with an oxidizing gas.

5. The heat generating pad according to claim 1, **characterized in that** the iron powder comprising particles, a surface of each of which is at least partially coverd with an iron oxide film, the iron oxide film has a thickness of 3 nm or more, and the iron powder at least contains from 20 to 100 % by weight of an active iron powder, which comprises particles having a region of an oxygen-free iron component in at least one region selected from a central part region of the iron powder particle and a region beneath the iron oxide film.

6. The heat generating pad according to claim 1, **characterized in that** the iron powder particle is covered on at least a part of the surface thereof by a wustite film and contains from 20 to 100 % by weight of an active iron powder having an amount of wustite of from 2 to 50 % by weight in terms of an X-ray peak intensity ratio to iron.

7. The heat generating pad according to claim 1, **characterized in that** the heat generating composition molded body is compressed.

8. The heat generating pad according to claim 1, **characterized in that** the heat seal part is heat sealed after temporary adhesion by an adhesive layer, and an adhesive component which constitutes the adhesive layer and a component of a heat seal material which constitutes the heat seal layer are copresent in the heat seal part.

9. The heat generating pad according to claim 8, **characterized in that** after heat sealing, a part of the heat generating composition molded body is moved to a temporary adhering part which is not heat sealed, thereby deadhering the temporary adhering part.

10. The heat generating pad according to claim 1, **characterized in that** the substrate and the covering material have stretchability.

11. The heat generating pad according to claim 1, **characterized in that** at least a part of the sectioned part has an irregular pattern.

12. The heat generating pad according to claim 1, **characterized in that** the sectional exothermic parts and the sectioned part have a difference of altitude, the sectional exothermic parts and the sectioned part are covered with an air permeability adjusting material, an airway is formed under the air permeability adjusting material, and air is taken in from an end part of the heat generating pad.

13. The heat generating pad according to claim 12, **characterized in that** the air permeability adjusting material is constituted of an air-impermeable raw material.

14. The heat generating pad according to claim 12, **characterized in that** the fixing measure is provided outside the air permeability adjusting material.

15. The heat generating pad according to claim 1, **characterized in that** the fixing measure is an adhesive layer.

16. The heat generating pad according to claim 15, **characterized in that** the adhesive layer is provided on at least a part of the air-permeable surface.

17. The heat generating pad according to claim 15, **characterized in that** a thermal buffer sheet is provided in the central part of the adhesive layer.

18. The heat generating pad according to claim 15, **characterized in that** an adhesive which constitutes the adhesive layer is a non-aromatic hot melt based adhesive.

19. The heat generating pad according to claim 15, **characterized in that** the adhesive layer is a hydrophilic adhesive layer, and the packaging material between the hydrophilic adhesive layer and the heat generating composition molded body has a moisture permeability of not more than 2 $g/m^2/24$ hr.

20. The heat generating pad according to claim 15, **characterized in that** the adhesive layer contains a drug.

21. The heat generating pad according to claim 1, **characterized in that** the moldable heat generating composition contains at least one member selected from additional components consisting of a water retaining agent, a water absorptive polymer, a pH adjusting agent, a hydrogen formation inhibitor, an aggregate, a fibrous material, a functional substance, a surfactant, an organosilicon compound, a pyroelectric substance, a moisturizer, a fertilizer component, a hydrophobic polymer compound, a heat generating aid, a metal other than iron, a metal oxide other than iron oxide, an acidic substance, and a mixture thereof.

22. The heat generating pad according to claim 1, **characterized in that** the heat generating pad is accommodated in an air-impermeable accommodating bag.

23. A method of use of a heat generating pad, **characterized by** bringing the heat generating pad according to claim 1 into contact with a necessary part of the body.

FIG.1

FIG.2(a)

FIG.2(b)

FIG.3

*FIG. 4*

8        1

*FIG.5*

8        1

*FIG.6*

8        1

*FIG.7*

*FIG.8(a)*

*FIG.8(b)*

*FIG.9*

# FIG.10

# FIG.11(a)

# FIG.11(b)

*FIG.12*

*FIG.13*

## FIG.14(a)

## FIG.14(b)

## FIG.15

FIG.16(a)

FIG.16(b)

FIG.16(c)

FIG.16(d)

FIG.16(e)

FIG.16(f)

FIG.16(g)

FIG.16(h)

FIG.16(i)

FIG.16(j)

FIG.16(k)

FIG.16(l)

FIG.16(m)

FIG.16(n)

FIG.16(o)

FIG.16(p)

FIG.16(q)

## FIG.17

## FIG.18

## FIG.19

## FIG.20

## FIG.21

<div align="center">

**INTERNATIONAL SEARCH REPORT**

</div>

| | International application No. |
|---|---|
| | PCT/JP2005/013008 |

**A. CLASSIFICATION OF SUBJECT MATTER**
Int.Cl⁷ A61F7/08, C09K5/16

According to International Patent Classification (IPC) or to both national classification and IPC

**B. FIELDS SEARCHED**

Minimum documentation searched (classification system followed by classification symbols)
Int.Cl⁷ A61F7/08, C09K5/16

Documentation searched other than minimum documentation to the extent that such documents are included in the fields searched
Jitsuyo Shinan Koho          1922-1996    Jitsuyo Shinan Toroku Koho    1996-2005
Kokai Jitsuyo Shinan Koho    1971-2005    Toroku Jitsuyo Shinan Koho    1994-2005

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y<br><br>A | JP 2003-334212 A (Maikoru Kabushiki Kaisha),<br>25 November, 2003 (25.11.03),<br>Full text; Figs. 1 to 23<br>(Family: none) | 1-4,7,8,10,<br>11,15,16,<br>18-22<br>5,6,9,12-14,<br>17 |
| A | JP 2002-155273 A (Kaoru USUI),<br>28 May, 2002 (28.05.02),<br>Full text; Figs. 1 to 14<br>(Family: none) | 1-22 |

☒ Further documents are listed in the continuation of Box C.          ☐ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search<br>20 September, 2005 (20.09.05) | Date of mailing of the international search report<br>11 October, 2005 (11.10.05) |
|---|---|
| Name and mailing address of the ISA/<br>Japanese Patent Office | Authorized officer |
| Facsimile No. | Telephone No. |

Form PCT/ISA/210 (second sheet) (January 2004)

INTERNATIONAL SEARCH REPORT

| International application No. |
| --- |
| PCT/JP2005/013008 |

C (Continuation).   DOCUMENTS CONSIDERED TO BE RELEVANT

| Category* | Citation of document, with indication, where appropriate, of the relevant passages | Relevant to claim No. |
| --- | --- | --- |
| Y | CD-ROM of the specification and drawings annexed to the request of Japanese Utility Model Application No. 5759/1992(Laid-open No. 58123/1993) (Shozo NAKAJIMA), 03 August, 1993 (03.08.93), Full text; Fig. 1 (Family: none) | 1 |
| Y | JP 2003-204983 A  (Asahi Kasei Corp.), 22 July, 2003 (22.07.03), Column 4, line 32 to 35; column 6, lines 6 to 17 (Family: none) | 1,2 |
| A | JP 10-17907 A  (Dowa Iron Powder Co., Ltd.), 20 January, 1998 (20.01.98), Full text; Fig. 1 (Family: none) | 6 |
| Y | JP 2003-509120 A  (The Procter & Gamble Co.), 11 March, 2003 (11.03.03), Full text; Figs. 1, 2 & US 6336935 B1        & EP 1212020 A & WO 2001/019302 A1     & AU 7116900 A & BR 14044 A            & CN 1373649 A & CA 2381812 A          & AU 768363 B | 1,11 |
| A | JP 11-512954 A  (The Procter & Gamble Co.), 09 November, 1999 (09.11.99), Full text; Figs. 1 to 3 & JP 3545769 B           & WO 1997/049361 A1 & AU 735088 B | 9,12-14 |

Form PCT/ISA/210 (continuation of second sheet) (January 2004)

## INTERNATIONAL SEARCH REPORT

| International application No. |
| --- |
| PCT/JP2005/013008 |

---

**Box No. II        Observations where certain claims were found unsearchable (Continuation of item 2 of first sheet)**

This international search report has not been established in respect of certain claims under Article 17(2)(a) for the following reasons:

1.  [×] Claims Nos.: 23
    because they relate to subject matter not required to be searched by this Authority, namely:
    Claim 23 relates to a method of using a heating pad. Therefore, this claim pertains to methods for treatment of a human body by surgery or therapy as well as relevant diagnostic methods and hence relates to a subject matter (continued to extra sheet)

2.  [ ] Claims Nos.:
    because they relate to parts of the international application that do not comply with the prescribed requirements to such an extent that no meaningful international search can be carried out, specifically:

3.  [ ] Claims Nos.:
    because they are dependent claims and are not drafted in accordance with the second and third sentences of Rule 6.4(a).

---

**Box No. III        Observations where unity of invention is lacking (Continuation of item 3 of first sheet)**

This International Searching Authority found multiple inventions in this international application, as follows:

1.  [ ] As all required additional search fees were timely paid by the applicant, this international search report covers all searchable claims.

2.  [ ] As all searchable claims could be searched without effort justifying an additional fee, this Authority did not invite payment of any additional fee.

3.  [ ] As only some of the required additional search fees were timely paid by the applicant, this international search report covers only those claims for which fees were paid, specifically claims Nos.:

4.  [ ] No required additional search fees were timely paid by the applicant.   Consequently, this international search report is restricted to the invention first mentioned in the claims; it is covered by claims Nos.:

**Remark on Protest**        [ ] The additional search fees were accompanied by the applicant's protest.

[ ] No protest accompanied the payment of additional search fees.

---

Form PCT/ISA/210 (continuation of first sheet (2)) (January 2004)

**INTERNATIONAL SEARCH REPORT**

International application No.

PCT/JP2005/013008

Continuation of Box No.II-1 of continuation of first sheet(2)

which this International Searching Authority is not required to search under the provisions of PCT Article 17(2)(a)(i) and Rule 39.1(iv).

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 53091114 A
- JP 3184915 A
- JP 4293989 A
- JP 6343658 A
- JP 59189183 A
- WO 0013626 A
- JP 9075388 A
- JP 60101448 A
- JP 9276317 A
- JP 11299817 A
- JP 1110718 A
- JP 6026829 A
- JP 2000288008 A
- JP 11507593 T
- JP 11508314 T
- JP 11508786 T
- JP 11512954 T
- JP 2002514104 T
- JP 2002200108 A
- JP 10265373 A
- JP 9087173 A
- JP 6145050 A
- JP 6199660 A
- JP 10279466 A
- JP 10182408 A
- JP 3161605 B
- JP 2001507593 T
- JP 7194641 A